(19) Europäisches Patentamt
European Patent Office
Office européen des brevets

(11) Veröffentlichungsnummer: **0 298 332 B1**

(12) # EUROPÄISCHE PATENTSCHRIFT

(45) Veröffentlichungstag der Patentschrift: **28.11.90**

(21) Anmeldenummer: **88110197.6**

(22) Anmeldetag: **27.06.88**

(51) Int. Cl.⁵: **C07D 249/08**, C07D 233/60, C07D 303/08, C07D 405/06, C07D 407/06, C07D 319/20, C07C 49/163, C07C 49/237, C07C 49/225, C07C 33/50

(54) **Hydroxyalkyl-azolyl-Derivate.**

(30) Priorität: **10.07.87 DE 3722794**
**25.04.88 DE 3813874**

(43) Veröffentlichungstag der Anmeldung: **11.01.89 Patentblatt 89/2**

(45) Bekanntmachung des Hinweises auf die Patenterteilung: **28.11.90 Patentblatt 90/48**

(84) Benannte Vertragsstaaten: **AT BE CH DE ES FR GB GR IT LI NL SE**

(56) Entgegenhaltungen:
**EP-A- 0 052 424**
**EP-A- 0 143 379**
**EP-A- 0 143 384**
**EP-A- 0 180 136**
**EP-A- 0 313 783**

**CHEMICAL ABSTRACTS, Band 77, Nr. 18, 30. Oktober 1972, Columbus, Ohio, USA; SHONO, T. et al. "Small ring compounds. XXVII. Migratory aptitude of the cyclopropyl group in the Beta-cyclopropylethyl cation systems", Seite 324, Spalte 1, Zusammenfassung Nr. 125 650holms, P Duprat, P Gautheron, K L Shepard, R L Smith, M F Sugrue)B. 1985), 480-4 000**
**CHEMICAL ABSTRACTS, Band 86, Nr. 7, 14.**

(73) Patentinhaber: **BAYER AG, D-5090 Leverkusen 1 Bayerwerk(DE)**

(72) Erfinder: **Stroech, Klaus, Dr., Rolsberger Strasse 22, D-5650 Solingen 19(DE)**
Erfinder: **Frie, Monika, Dr., Im alten Driesch 1, D-5068 Odenthal(DE)**
Erfinder: **Himmler, Thomas, Dr., Bonhoefferstrasse 20, D-5000 Köln 80(DE)**
Erfinder: **Brandes, Wilhelm, Dr., Eichendorffstrasse 3, D-5653 Leichlingen(DE)**
Erfinder: **Dutzmann, Stefan, Dr., Leinenweberweg 33, D-4000 Düsseldorf 13(DE)**
Erfinder: **Hänssler, Gerd, Dr., Am Arenzberg 58a, D-5090 Leverkusen 3(DE)**

(56) Entgegenhaltungen: (Fortsetzung)
**Februar 1977, Columbus, Ohio, USA; OLAH, GEORGE A. et al.: "The unusual Favorskii-Nazarov reaction of dicyclopropyl ketone." Seite 494, Spalte 1, Zusammenfassung-Nr. 43 227x**
**CHEMICAL ABSTRACTS, Band 92, Nr. 3, 21. Januar 1980, Columbus, Ohio, USA; TIMBERLAKE, JACK W.; JUN, YOUNG M.: "Tetracyclopropylethylene glycol." Seite 648, Spalte 2, Zusammenfassung-Nr. 22 100j**

**Die Akte enthält technische Angaben, die nach dem Eingang der Anmeldung eingereicht wurden und die nicht in dieser Patentschrift enthalten sind.**

EP 0 298 332 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Hydroxyalkylazolyl-Derivate, mehrere Verfahren zu deren Herstellung und deren Verwendung als Fungizide und Pflanzenwachstumsregulatoren.

Es ist bereits bekannt, daß zahlreiche Hydroxyalkylazolyl-Derivate fungizide und pflanzenwuchsregulierende Eigenschaften besitzen (vgl. EP-A 0 040 345 und EP-A 0 052 424). Die Wirksamkeit dieser Stoffe ist sehr gut; bei niedrigen Aufwandmengen läßt die Wirksamkeit allerdings in manchen Fällen zu wünschen übrig.

Ferner sind aus der EP-A 0 180 136 schon fungizid wirksame Hydroxyethyl-Derivate bekannt, die am Carbinol-Kohlenstoffatom sowohl einen substituierten Cyclopropyl-Rest als auch einen Aryl-Substituenten enthalten. Es werden aber keine Verbindungen beschrieben, in denen der Aryl-Rest über eine gesättigte oder ungesättigte Kohlenstoffkette mit dem zentralen Kohlenstoffatom verbunden ist.

Schließlich sind aus der EP-A 0 313 783 Hydroxyethyl-azolyl-Derivate mit antimykotischen Eigenschaften bekannt. In diesen Verbindungen kann am zentralen Kohlenstoffatom ein substituierter Cyclopropyl-Rest und ein über gesättigtes oder ungesättigtes Alkyl gebundener Rest der Formel

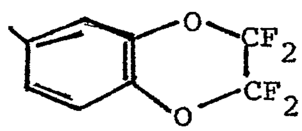

vorhanden sein. Bei dieser Druckschrift handelt es sich um ein Dokument gemäß Artikel 54 (3) EPÜ.

Es wurden nun neue Hydroxyalkyl-azolyl-Derivate der Formel

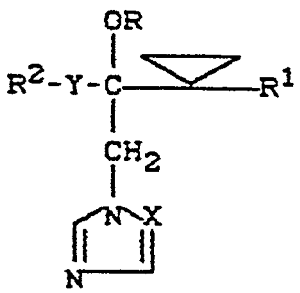

(I)

in welcher

R für Wasserstoff, Alkyl mit 1 bis 6 Kohlenstoffatomen oder Alkylcarbonyl mit 1 bis 6 Kohlenstoffatomen im Alkylteil steht,

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin

Z für Sauerstoff, Schwefel, SO oder $SO_2$ steht und

$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,

$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

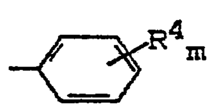

steht, worin

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes

Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und
m für die Zahlen 0, 1, 2 oder 3 steht,
X für Stickstoff oder eine CH-Gruppe steht und
Y für die Gruppierungen $-CH_2-CH_2-$, $-CH=CH-$, $-C\equiv C-$,

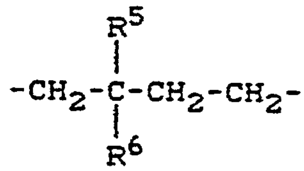 oder 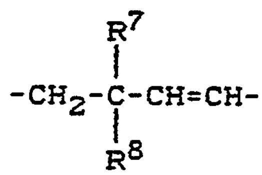 steht,

worin $R^5$, $R^6$, $R^7$ und $R^8$ unabhängig voneinander für Wasserstoff, Methyl oder Ethyl stehen, sowie deren Säureadditionssalze und Metallsalzkomplexe gefunden.

Die erfindungsgemäßen Stoffe enthalten ein asymmetrisch substituiertes Kohlenstoffatom. Sie können daher in optischen Isomerenformen anfallen. Darüber hinaus können diejenigen Stoffe der Formel (I), in denen Y für eine -CH=CH-Gruppe oder für eine

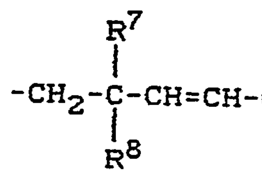Gruppe

steht, auch in Form von cis- bzw. trans-Isomeren vorliegen. Die Erfindung betrifft sowohl die einzelnen Isomeren als auch deren Gemische.

Weiterhin wurde gefunden, daß man Hydroxyalkyl-azolyl-Derivate der Formel (I) sowie deren Säureadditions-Salze und Metallsalz-Komplexe erhält, wenn man

a) Oxirane der Formel

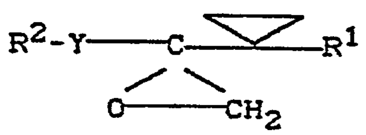

(II)

in welcher
$R^1$, $R^2$ und Y die oben angegebene Bedeutung haben,
mit Azolen der Formel

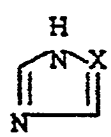

(III)

in welcher
X die oben angegebene Bedeutung hat, in Gegenwart eines Säurebindemittels und in Gegenwart eines Verdünnungsmittels umsetzt, oder

b) Hydroxyalkyl-azolyl-Derivate der Formel

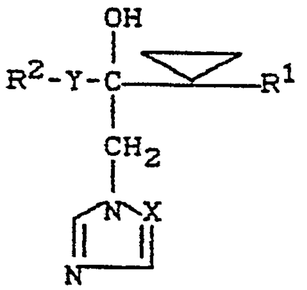

(Ia)

in welcher
$R^1$, $R^2$, X und Y die oben angegebene Bedeutung haben, mit starken Basen in Gegenwart eines Verdünnungsmittels umsetzt und die dabei entstehenden Alkoholate der Formel

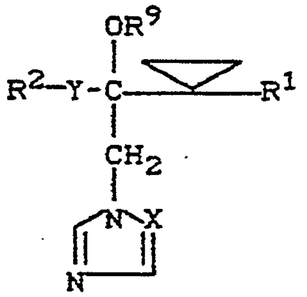

(Ib)

in welcher
$R^1$, $R^2$, X und Y die oben angegebene Bedeutung haben und
$R^9$ für ein Alkalimetallkation oder für ein quarternäres Ammonium- oder Phosphoniumkation steht, mit Halogenverbindungen der Formel

$R^{10}$-Hal (IV)

in welcher
$R^{10}$ für Alkyl mit 1 bis 6 Kohlenstoffatomen oder Alkylcarbonyl mit 1 bis 6 Kohlenstoffatomen im Alkylteil steht und
Hal für Chlor oder Brom steht, in Gegenwart eines Verdünnungsmittels umsetzt, und gegebenenfalls anschließend an die so erhaltenen Verbindungen der Formel (I) eine Säure oder ein Metallsalz addiert.

Schließlich wurde gefunden, daß die neuen Hydroxyalkyl-azolyl-Derivate der Formel (I) sowie deren Säureadditionssalze und Metallsalzkomplexe starke fungizide und pflanzenwuchsregulierende Eigenschaften besitzen.

Überraschenderweise zeigen die erfindungsgemäßen Stoffe eine bessere fungizide und pflanzenwuchsregulierende Wirksamkeit als die konstitutionell ähnlichsten vorbekannten Verbindungen gleicher Wirkungsrichtung.

Die erfindungsgemäßen Hydroxyalkyl-azolyl-Derivate sind durch die Formel (I) allgemein definiert.

Besonders bevorzugt sind diejenigen Verbindungen der Formel (I), in denen

R für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl und Isobutylcarbonyl steht,

$R^1$ für Fluor, Chlor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung der Formel -Z-$R^3$ steht, worin
Z für Sauerstoff, Schwefel, SO oder $SO_2$ steht und

$R^3$ für Methyl, Ethyl, n-Propyl, Isopropyl, n-Butyl, Isobutyl oder tert.-Butyl steht, wobei jeder dieser Reste einfach bis dreifach, gleichartig oder verschieden substituiert sein kann durch Methoxy, Ethoxy, Methylthio, Phenyl, Fluor, Chlor und/oder Brom, oder

$R^3$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder

$R^3$ für Benzyl steht, das im Phenylteil gegebenenfalls einfach bis dreifach, gleichartig oder verschieden durch Fluor, Chlor, Brom, Trifluormethyl, Methyl, Ethyl, Methoxy und/oder Ethoxy substituiert sein kann,

$R^2$ für gegebenenfalls einfach bis dreifach, gleichartig oder verschieden durch Methoxy, Ethoxy, Methylthio, Ethylthio, Phenyl, Fluor, Chlor und/oder Brom substituiertes Alkyl mit 1 bis 4 Kohlenstoffatomen steht, ferner für gegebenenfalls einfach bis dreifach, gleichartig oder verschieden durch Methyl, Ethyl, Fluor und/oder Chlor substituiertes Cycloalkyl mit 3 bis 7 Kohlenstoffatomen steht, außerdem für gegebenenfalls einfach bis dreifach, gleichartig oder verschieden durch Methyl, Fluor und/oder Chlor substituiertes Naphthyl steht oder für den Rest der Formel

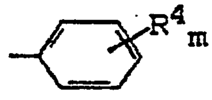

steht, worin

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht, oder für Nitro, Amino, Alkylamino mit 1 oder 2 Kohlenstoffatomen in der Alkylgruppe, Dialkylamino mit 1 oder 2 Kohlenstoffatomen in jeder Alkylgruppe, Phenylamino oder für Alkylcarbonylamino mit 1 oder 2 Kohlenstoffatomen in der Alkylgruppe steht,

m für die Zahlen 0, 1, 2 oder 3 steht,

X für Stickstoff oder eine CH-Gruppe steht und

Y für die Gruppierungen $-CH_2-CH_2-$, $-CH=CH-$, $-C\equiv C-$,

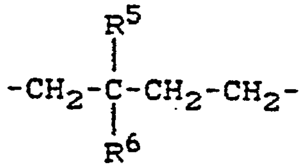 oder 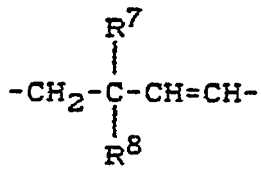 steht,

worin

$R^5$, $R^6$, $R^7$ und $R^8$ unabhängig voneinander für Wasserstoff, Methyl oder Ethyl stehen.

Bevorzugte erfindungsgemäße Verbindungen sind auch Additionsprodukte aus Säuren und denjenigen Hydroxyalkyl-azolyl-Derivaten der Formel (I), in denen R, $R^1$, $R^2$, X und r die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II, sowie IV. bis VIII. Nebengruppe des Periodensystems der Elemente und denjenigen Hydroxyalkyl-azolyl-Derivaten der Formel (I), in denen R, $R^1$, $R^2$, X und Y die Bedeutungen haben, die bereits vorzugsweise für diese Reste genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Hydroxyalkyl-azolyl-Derivate der Formel (I) seien die in den folgenden Tabellen aufgeführten Stoffe genannt:

Tabelle 1

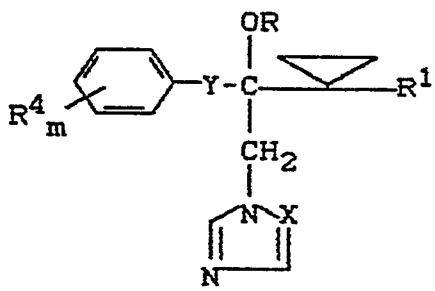

(Ic)

| $R^4_m$ | R | X | $R^1$ | Y |
|---|---|---|---|---|
| 2,4-$Cl_2$ | H | N | Cl | $-CH_2-CH_2-$ |
| 2,4-$F_2$ | H | N | Cl | " |
| 4-$CH_3$ | H | N | Cl | " |
| 4-$CF_3$ | H | N | Cl | " |
| 4-$OCF_3$ | H | N | Cl | " |
| 4-$OCH_3$ | H | N | Cl | " |
| 4-$SCH_3$ | H | N | Cl | " |
| 3-Cl | H | N | $-S-CH_3$ | " |
| 4-Cl | H | N | $-S-C_2H_5$ | " |

Tabelle 1 (Fortsetzung)

| $R^4{}_m$ | R | X | $R^1$ | Y |
|---|---|---|---|---|
| 2,4,6-$Cl_3$ | " | " | Cl | $-CH_2-CH_2-$ |
| 4-Cl | " | " | F | " |
| 4-Cl | " | CH | Cl | " |
| 4-Cl | $CH_3$ | N | Cl | " |
| 4-Cl | H | " | 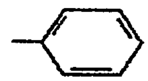 | " |
| 4-Cl | " | " | 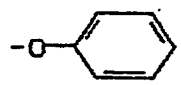 | " |
| 4-Cl | " | " | 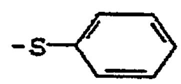 | " |
| 4-Cl | " | " | 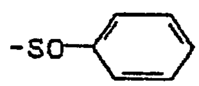 | " |
| 4-Cl | " | " | 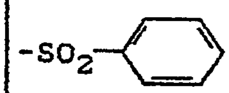 | " |
| 4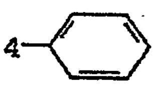 | " | " | Cl | " |
| 4-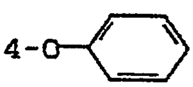 | " | " | Cl | " |
| 4-$C_4H_9$-t. | " | " | Cl | " |
| 2-Cl, 4-$CH_3$ | " | " | Cl | " |
| — | " | " | Cl | " |
| 4-Cl | $-CO-CH_3$ | " | Cl | " |
| 4-Cl | $-C_2H_5$ | " | Cl | " |
| 4-F | $CH_3$ | " | F | " |

Tabelle 1 (Fortsetzung)

| $R^4_m$ | R | X | $R^1$ | Y |
|---|---|---|---|---|
| 2,4-$Cl_2$ | H | N | Cl | -CH=CH |
| 2,4-$F_2$ | H | N | Cl | " |
| 4-$CH_3$ | H | N | Cl | " |
| 4-$CF_3$ | H | N | Cl | " |
| 4-$OCF_3$ | H | N | Cl | " |
| 4-$OCH_3$ | H | N | Cl | " |
| 4-$SCH_3$ | H | N | Cl | " |
| 3-Cl | H | N | -S-$CH_3$ | " |
| 4-Cl | H | N | -S-$C_2H_5$ | " |
| 2,4,6-$Cl_3$ | " | " | Cl | -CH=CH- |
| 4-Cl | " | " | F | " |
| 4-Cl | " | CH | Cl | " |
| 4-Cl | $CH_3$ | N | Cl | " |
| 4-Cl | H | " | 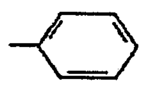 | " |
| 4-Cl | " | " | -O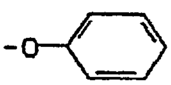 | " |
| 4-Cl | " | " | -S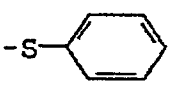 | " |
| 4-Cl | " | " | -SO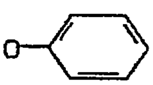 | " |
| 4-Cl | " | " | -$SO_2$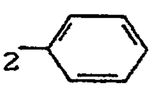 | " |
| 4-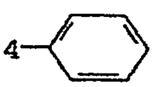 | " | " | Cl | " |

Tabelle 1 (Fortsetzung)

| $R^4_m$ | R | X | $R^1$ | Y |
|---|---|---|---|---|
| 4-O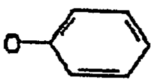 | H | N | Cl | -CH=CH- |
| 4-$C_4H_9$-t. | " | " | Cl | " |
| 2-Cl, 4-$CH_3$ | " | " | Cl | " |
| - | " | " | Cl | " |
| 4-Cl | -CO-$CH_3$ | " | Cl | " |
| 4-Cl | -$C_2H_5$ | " | Cl | " |
| 4-F | $CH_3$ | " | F | " |
| 2,4-$Cl_2$ | H | N | Cl | -C≡C- |
| 2,4-$F_2$ | H | N | Cl | " |
| 4-$CH_3$ | H | N | Cl | " |
| 4-$CF_3$ | H | N | Cl | " |
| 4-$OCF_3$ | H | N | Cl | " |
| 4-$OCH_3$ | H | N | Cl | " |
| 4-$SCH_3$ | H | N | Cl | " |
| 3-Cl | H | N | -S-$CH_3$ | " |
| 4-Cl | H | N | -S-$C_2H_5$ | " |

Tabelle 1 (Fortsetzung)

| $R^4_m$ | R | X | $R^1$ | Y |
|---|---|---|---|---|
| 2,4,6-$Cl_3$ | " | " | Cl | -C≡C- |
| 4-Cl | " | " | F | " |
| 4-Cl | " | CH | Cl | " |
| 4-Cl | $CH_3$ | N | Cl | " |
| 4-Cl | H | " | 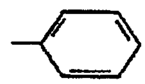 | " |
| 4-Cl | " | " | 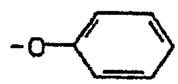 | " |
| 4-Cl | " | " | 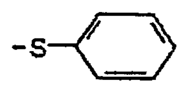 | " |
| 4-Cl | " | " | 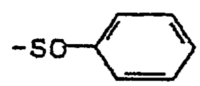 | " |
| 4-Cl | " | " | 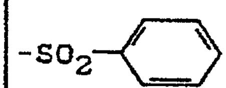 | " |
| 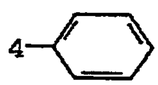 | " | " | Cl | " |
| 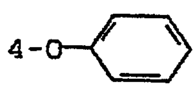 | " | " | Cl | " |
| 4-$C_4H_9$-t. | " | " | Cl | " |
| 2-Cl, 4-$CH_3$ | " | " | Cl | " |
| - | " | " | Cl | " |
| 4-Cl | -CO-$CH_3$ | " | Cl | " |
| 4-Cl | -$C_2H_5$ | " | Cl | " |
| 4-F | $CH_3$ | " | F | " |

Tabelle 2

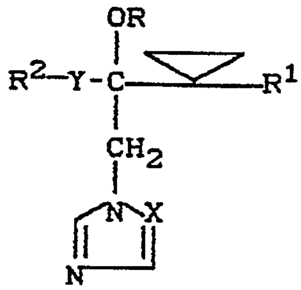

(I)

| $R^2$ | R | X | $R^1$ | Y |
|---|---|---|---|---|
| 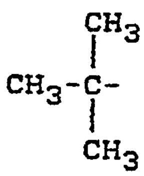 | H | N | Cl | $-CH_2-CH_2-$ |
| 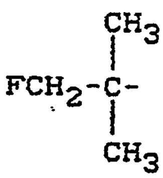 | H | N | Cl | $-CH_2-CH_2-$ |
| 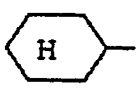 | H | N | Cl | $-CH_2-CH_2-$ |
| 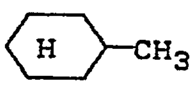 | H | N | Cl | $-CH_2-CH_2-$ |
| 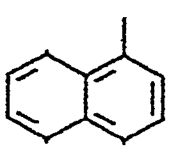 | H | N | Cl | $-CH_2-CH_2-$ |

Tabelle 2 (Fortsetzung)

| $R^2$ | R | X | $R^1$ | Y |
|---|---|---|---|---|
| 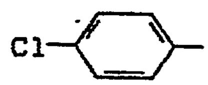 | H | N | Cl | 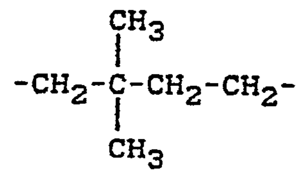 |
| 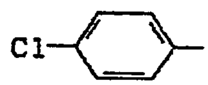 | H | N | Cl | 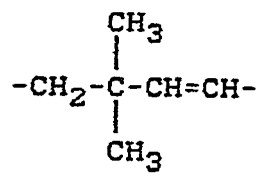 |
| 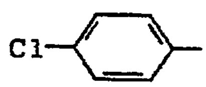 | H | N | Cl | $-CH_2-CH_2-CH=CH-$ |

Verwendet man 2-(1-Chlorcyclopropyl)-2-(4-chlorphenylethyl)-oxiran und 1,2,4-Triazol als Ausgangsstoffe, so kann der Verlauf des erfindungsgemäßen Verfahrens (a) durch das folgende Formelschema veranschaulicht werden:

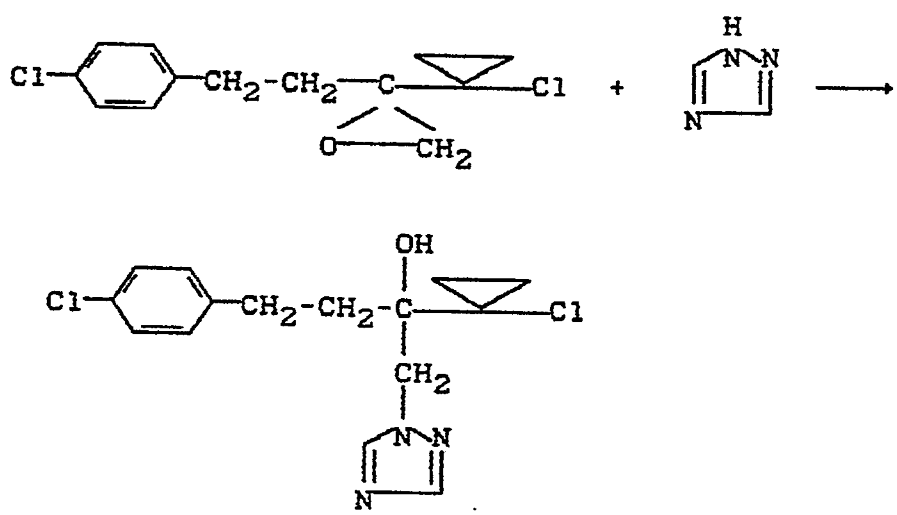

Verwendet man 2-(1-Chlorcyclopropyl)-4-(4-chlorphenyl)1-(1,2,4-triazol-1-yl)-butan-2-ol und Natriumhydrid als Ausgangsstoffe und Iodmethan als Reaktionskomponente, so kann der Verlauf des erfindungsgemäßen Verfahrens (b) duch das folgende Formelschema veranschaulicht werden:

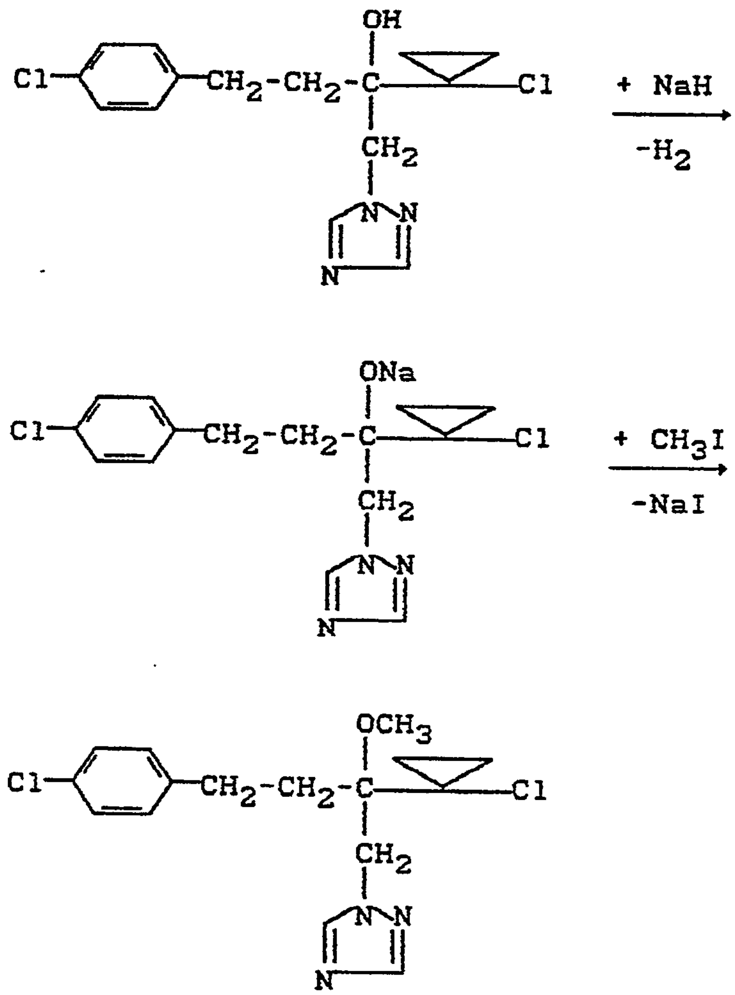


Die bei dem erfindungsgemäßen Verfahren (a) als Ausgangsstoffe benötigten Oxirane sind durch die Formel (II) allgemein definiert. In dieser Formel haben $R^1$, $R^2$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Oxirane der Formel (II) sind bisher noch nicht bekannt. Sie lassen sich herstellen, indem man

c) Cyclopropylketone der Formel

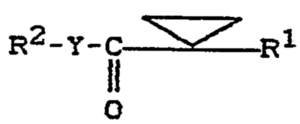
(V)

in welcher
$R^1$, $R^2$ und Y die oben angegebene Bedeutung haben, entweder

α) mit Dimethyloxosulfonium-methylid der Formel

$$\overset{\delta^{\oplus}\,\delta^{\ominus}}{(CH_3)_2SOCH_2} \quad (VI)$$

oder

β) mit Dimethylsulfonium-methylid der Formel

$$\overset{\delta^{\oplus}}{(CH_3)_2S}\ \overset{\delta^{\ominus}}{CH_2} \qquad (VII)$$

in Gegenwart eines Verdünnungsmittels umsetzt, oder indem man

d) Carbinole der Formel

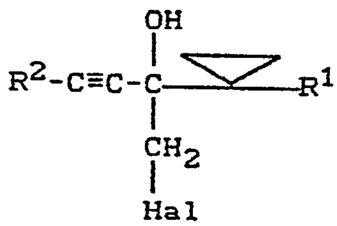

(VIII)

in welcher

$R^1$ und $R^2$ die oben angegebene Bedeutung haben und Hal' für Chlor oder Brom steht, mit Basen in Gegenwart eines Verdünnungsmittels umsetzt.

Die bei der Durchführung des Verfahrens (c) als Ausgangsstoffe benötigten Cyclopropylketone der Formel (V) sind bisher noch nicht bekannt. Sie lassen sich herstellen, indem man

e) Aldehyde der Formeln

$R^2$-CHO (IX-a)

oder

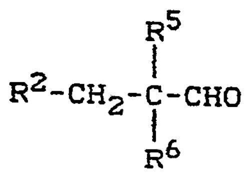

(IX-b)

in welcher

$R^2$, $R^5$ und $R^6$ die oben angegebene Bedeutung haben,

mit Methyl-cyclopropyl-ketonen der Formel

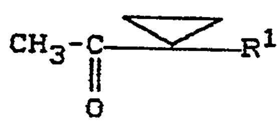

(X)

in welcher

$R^1$ die oben angegebene Bedeutung hat,

in Gegenwart eines Katalysators und in Gegenwart eines Verdünnungsmittels umsetzt und gegebenenfalls die dabei entstehenden Cyclopropylketone der Formeln

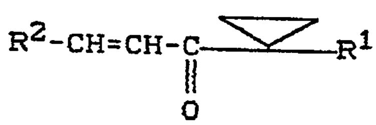

(V-a)

oder

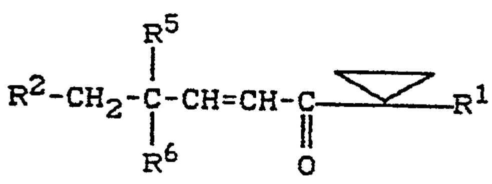

(V-b)

in welchen
$R^1$, $R^2$, $R^5$ und $R^6$ die oben angegebene Bedeutung haben,
in Gegenwart eines Katalysators und in Gegenwart eines Verdünnungsmittels hydriert, oder indem man
f) Acetylene der Formel

$R^2$-C≡CH (XI)

in welcher
$R^2$ die oben angegebene Bedeutung hat, mit Säurehalogeniden der Formel

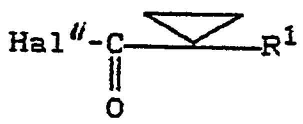

(XII)

in welcher
$R^1$ die oben angegebene Bedeutung hat und
Hal″ für Chlor oder Brom steht, in Gegenwart eines Katalysators und in Gegenwart eines Verdünnungsmittels umsetzt.

Die bei dem Verfahren (e) als Ausgangsstoffe benötigten Aldehyde der Formeln (IX-a) und (IX-b) sind allgemein bekannte Verbindungen der organischen Chemie.

Die außerdem bei der Durchführung des Verfahrens (e) als Reaktionskomponenten benötigten Methyl-cyclopropylketone der Formel (X) sind bekannt oder lassen sich nach prinzipiell bekannten Verfahren herstellen (vgl. Synthesis 1977, 189).

Als Katalysatoren kommen bei der Durchführung der ersten Stufe des Verfahrens (e) alle für derartige Kondensationen üblichen Reaktionsbeschleuniger in Frage. Vorzugsweise verwendbar sind basische Substanzen, z.B. Alkalimetallhydroxide, wie Natriumhydroxid oder Kaliumhydroxid.

Als Verdünnungsmittel kommen bei der Durchführung der ersten Stufe des Verfahrens (e) alle für derartige Umsetzungen üblichen inerten organischen Solventien in Betracht. Vorzugsweise verwendbar sind Alkohole, wie Methanol, Ethanol, Isopropanol, n-Butanol und tert.Butanol.

Die Reaktionstemperaturen können bei der Durchführung der ersten Stufe des Verfahrens (e) innerhalb eines größeren Bereiches variiert werden. Im allgemeinen arbeitet man bei Temperaturen zwischen 0°C und 100°C, vorzugsweise zwischen 10°C und 80°C.

Die erste Stufe des Verfahrens (e) wird im allgemeinen unter Normaldruck durchgeführt. Es ist aber auch möglich, unter erhöhtem oder vermindertem Druck zu arbeiten.

Bei der Durchführung der ersten Stufe des Verfahrens (e) setzt man auf 1 Mol an Methyl-cyclopropylketon der Formel (X) 1 Mol an Aldehyd der Formel (IX) sowie eine katalytische Menge an Reaktionsbeschleuniger ein. Es ist jedoch auch möglich, die eine oder andere Komponente in einem Überschuß zu verwenden. Die Aufarbeitung erfolgt nach üblichen Methoden. Im allgemeinen geht man so vor, daß man die in festem Zustand anfallenden Reaktionsprodukte absaugt und nach gegebenenfalls vorheriger Reinigung für die weiteren Umsetzungen verwendet.

In der zweiten Stufe des Verfahrens (e) werden die Cyclopropylketone der Formeln (V-a) oder (V-b) in Gegenwart eines Katalysators und eines Verdünnungsmittels mit Wasserstoff hydriert. Man arbeitet dabei in flüssiger Phase unter Verwendung eines suspendierten, pulverförmigen Hydrierkatalysators (heterogen) oder unter Verwendung eines im Verdünnungsmittel löslichen Katalysatorkomplexes (homogen). Die Durchführung der Hydrierung kann diskontinuierlich (chargenweise) oder kontinuierlich als Sumpf- oder Rieselphasenhydrierung in bekannten Hydrierreaktoren, wie Autoklaven, Autoklavenkaskaden, Rohrreaktoren oder Umlaufreaktoren erfolgen. Die bevorzugte Arbeitsweise ist die diskontinuierliche Sumpfphasenhydrierung im Autoklaven bei erhöhtem Druck.

Als Verdünnungsmittel kommen bei der Durchführung der zweiten Stufe des Verfahrens (e) inerte organische Lösungsmittel in Frage. Hierzu gehören vorzugsweise Alkohole, wie Methanol, Ethanol, Isopropanol oder Ethylenglykol; Ether, wie Diethylether, Diisopropylether, Ethylenglykolmonomethylether, Ethylenglykoldimethylether, Dioxan oder Tetrahydrofuran; gesättigte Kohlenwasserstoffe, wie n-Heptan oder Cyclohexan; aromatische Kohlenwasserstoffe, wie Toluol; sowie Ester, wie Essigsäureethylester.

Für die zweite Stufe des Verfahrens (e) geeignete Hydrierkatalysatoren sind beispielsweise solche, die aus Metallen und/oder Verbindungen von Elementen der achten Nebengruppe des periodischen Systems der Elemente nach Mendelejew bestehen oder diese enthalten. Dabei sind die Metalle Ruthenium, Rhodium, Palladium, Platin, Kobalt und Nickel und deren Verbindungen bevorzugt. Bei den Metallverbindungen kann es sich beispielsweise um Chloride, Oxide, Hydroxide und/oder Oxihydrate handeln.

Zusätzlich können die Metalle Kupfer, Vanadium, Molybdan, Chrom und/oder Mangan, sowie Verbindungen dieser Metalle zugegen sein.

Die Hydrierkatalysatoren können ausschließlich oder überwiegend aus Wasserstoff-übertragenden Substanzen bestehen, diese können aber auch auf Trägermaterialien aufgebracht sein.

Als Trägermaterialien für die Wasserstoff-übertragenden Substanzen kommen beispielsweise in Frage: anorganische Materialien, wie Kieselgur, Kieselsäure, Aluminiumoxid, Alkali- und Erdalkalisilikate, Aluminiumsilikate, Montmorillonit, Zeolithe, Spinelle, Dolomit, Kaolin, Magnesiumsilikate, Zirkonoxid, Zinkoxid, Calciumcarbonat, Siliciumcarbid, Aluminiumphosphat, Borphosphat, Asbest, Aktivkohle oder Bariumsulfat, aber auch organische Materialien, beispielsweise natürlich vorkommende oder synthetische Verbindungen mit hohen Molekulargewichten wie Seide, Polyamide, Polystyrole, Zellstoff oder Polyurethane. Bevorzugt sind anorganische Tragermaterialien in Pulverform.

Derartige Trägerkatalysatoren können im allgemeinen 0,5 bis 50 Gew-%, vorzugsweise 1 bis 10 Gew.-% der Wasserstoff-übertragenden Substanz, bezogen auf die Gesamtmasse des Trägerkatalysators, enthalten. Die Wasserstoff-übertagende Substanz kann dabei homogen im Trägermaterial verteilt sein, bevorzugt sind jedoch Katalysatoren, in deren äußerer Schicht oder auf deren Oberfläche die Wasserstoff-übertragende Substanz abgelagert ist. Die Herstellung und die Formgebung der Katalysatoren, die im Verfahren (e) Verwendung finden können, kann in bekannter Weise erfolgen (siehe beispielsweise Houben-Weyl, Methoden der organischen Chemie, Band IV, Ic, Teil I, S. 16 bis 26, Georg Thieme Verlag, Stuttgart, 1980).

Bevorzugte Trägerkatalysatoren sind Ruthenium auf Kohle, Ruthenium auf Aluminiumoxid, Rhodium auf Kohle, Rhodium auf Aluminiumoxid, Palladium auf Kohle, Palladium auf Aluminiumoxid, Palladium auf Calciumcarbonat, Palladium auf Bariumsulfat, Palladium auf Kieselsäure, Platin auf Kohle und Platin auf Aluminiumoxid, Nickel auf Kieselgur, Nickel auf Aluminiumoxid sowie Nickel und Palladium auf Aluminiumoxid.

Bei Hydrierung in heterogenem System bevorzugte Hydrierkatalysatoren, die ausschließlich oder überwiegend aus Wasserstoff-übertragender Substanz bestehen, sind beispielsweise oxidische Katalysatoren, wie Palladiumoxid, Platinoxid, Rutheniumoxid und/oder Rhodiumoxid/Platinoxid nach Nishimura, ferner durch Reduktion von entsprechenden Metallsalzen oder Metallsalzgemischen mit Alkalihydriden, Alkaliboranaten, Metallalkylen, Hydrazin, Formaldehyd, Wasserstoff oder elektropositiven Metallen herstellbare Schwarzkatalysatoren, wie Palladium/Schwarz, Platin/Schwarz und Rhodium/Schwarz; sowie Skelettkatalysatoren vom Raney-Typ, wie Raney-Nickel, Raney-Kobalt, Raney-Nickel-Kobalt, Raney-Nickel-Eisen, Raney-Nickel-Kupfer, Raney-Nickel-Eisen-Chrom, Raney-Nickel-Palladium und Raney-Nickel-Eisen-Vanadium.

Bei Hydrierung im heterogenen System werden die Hydrierkatalysatoren bei der zweiten Stufe des Verfahrens (e) in einer solchen Menge eingesetzt, daß 0,05 bis 2,5, vorzugsweise 0,1 bis 1 Gew.-% Wasserstoff-übertragende Substanz bezogen auf das Gesamtgewicht des Reaktionsgemisches vorliegen.

Zur Durchführung der zweiten Stufe des Verfahrens (e) können auch Gemische aus zwei oder mehreren der genannten Hydrierkatalysatoren verwendet werden.

Die katalytische Aktivität der Hydrierkatalysatoren bleibt bei der Durchführung der zweiten Stufe des Verfahrens (e) im allgemeinen weitgehend erhalten, so daß diese bei diskontinuierlicher Arbeitsweise wiederholt eingesetzt werden können und bei kontinuierlicher Arbeitsweise längere Zeit in Gebrauch bleiben können.

Die Reaktionstemperaturen können in einem größeren Bereich variiert werden. Im allgemeinen arbeitet man im Bereich zwischen 0°C und 15°C, vorzugsweise zwischen 2°C und 12°C.

Die heterogen katalysierten Hydrierungen in der zweiten Stufe des Verfahrens (e) werden vorzugsweise bei erhöhtem Druck durchgeführt. Im allgemeinen arbeitet man zwischen 1 und 150 bar, vorzugsweise zwischen 10 und 60 bar.

Außer den genannten Hydrierkatalysatoren heterogener Natur können auch homogen gelöste Hydrierkatalysatoren bei der Durchführung der zweiten Stufe des Verfahrens (e) eingesetzt werden. Die im Vergleich zu heterogenen Katalysatoren oft höhere Selektivität homogener Hydrierkatalysatoren erlaubt die selektive Hydrierung von Cyclopropylketonen der Formeln (V-a) oder (V-b), die zusätzliche hydrierbare oder hydrogenolyse-empfindliche Substituenten, wie z.B. Halogen am Phenylrest, enthalten. Solche homogenen Hydrierkatalysatoren sind beispielsweise Komplexe, die Metalle der achten Nebengruppe des Periodensystems der Elemente nach Mendelejew als Zentralatom enthalten. Bevorzugt sind dabei die Metalle Ruthenium, Rhodium, Palladium, Iridium, Kobalt und Nickel. Besonders bevorzugt sind Ruthenium, Rhodium und Indium. Als Beispiele für derartige Metallkomplexe seien Tris-(triphenylphosphin)-rhodium(I)-chlorid, Tris-(triphenylphosphin)-ruthenium(II)-chlorid und Bis-(triphenylphorphin)-carbonyl-iridium(I)-chlorid genannt.

Bei Verwendung homogen gelöster Hydrierkatalysatoren kommen bei der Durchführung der zweiten Stufe des Verfahrens (e) als Verdünnungsmittel inerte organische Solventien in Frage. Vorzugsweise verwendbar sind Alkohole, wie Methanol, Ethanol, Isopropanol oder Ethylenglykol, ferner Kohlenwasserstoffe, wie Toluol, außerdem Ketone, wie Aceton und Butanon, und auch Ester, wie Essigsäureethylester.

Bei der Hydrierung im homogenen System werden die Hydrierkatalysatoren bei der Durchführung der zweiten Stufe des Verfahrens (e) im allgemeinen in einer solchen Menge eingesetzt, daß 0,01 bis 2,5 Mol-%, vorzugsweise 0,05 bis 1,0 Mol-% an Hydrierkatalysator-Komplex bezogen auf eingesetztes Cyclopropylketon der Formel (V-a) oder (V-b) vorhanden sind.

Die Reaktionstemperaturen können auch bei der Hydrierung im homogenen System bei der Durchführung der zweiten Stufe des Verfahrens (e) innerhalb eines größeren Bereiches variiert werden. Im allgemeinen arbeitet man bei Temperaturen zwischen 0°C und 150°C, vorzugsweise zwischen 20°C und 120°C.

Die im homogenen System durchgeführten Hydrierungen in der zweiten Stufe des Verfahrens (e) werden vorzugsweise unter erhöhtem Druck vorgenommen. Im allgemeinen arbeitet man unter Drucken zwischen 1 und 150 bar, vorzugsweise zwischen 10 und 100 bar.

In einer Variante kann die Hydrierung im homogenen System in der zweiten Stufe des Verfahrens (e) auch so durchgeführt werden, daß nicht mit molekularem Wasserstoff hydriert wird, sondern daß Reduktionsmittel eingesetzt werden, die in der Lage sind, in Gegenwart eines geeigneten Katalysators ein oder mehrere Wasserstoffatome an das Cyclopropylketon der Formel (V-a) oder (V-b) im Sinne einer Transferhydrierung zu übertragen und daher als Wasserstoffdonatoren wirken. Als Katalysatoren für eine solche Transferhydrierung kommen prinzipiell die bereits für die homogen katalysierte Hydrierung mit molekularem Wasserstoff in der zweiten Stufe des Verfahrens (e) beschriebenen Komplexe von Metallen der achten Nebengruppe des Periodensystems der Elemente nach Mendelejew in Frage.

Als Wasserstoffdonatoren kommen hierbei primäre und sekundäre, ein- oder mehrwertige Alkohole in Betracht. Vorzugsweise verwendbar sind Methanol, Ethanol, n-Propanol, i-Propanol, n-Butanol, 2-Butanol, Benzylalkohol, Ethylenglykol, 1,3-Propandiol, 1,4-Butandiol und 1,5-Pentandiol. Diese Alkohole können sowohl als Wasserstoffdonatoren als auch als Lösungsmittel dienen.

Weitere Wasserstoffdonatoren, die in der zweiten Stufe des Verfahrens (e) eingesetzt werden können, sind Alkalimetall- und Erdalkalimetall-Salze der Ameisensäure, wie Natriumformiat und Kaliumformiat, und auch die Ameisensäure selbst. – Bei Verwendung eines Salzes der Ameisensäure kann die zweite Stufe des Verfahrens (e) in Form einer Phasen-transfer-Katalyse durchgeführt werden, wobei das Cyclopropylketon der Formel (V-a) oder (V-b) und der Hydrierkatalysator in einem geeigneten inerten Solvens gelöst sind und das Formiat als wäßrige Lösung in einer zweiten Phase vorliegt. Geeignete Solventien sind deshalb in diesem Zusammenhang solche Lösungsmittel, die einerseits das Cyclopropylketon der Formel (V-a) oder (V-b) und den Hydrierkatalysator lösen, andererseits aber nicht mit Wasser mischbar sind. Derartige Lösungsmittel sind z.B. Benzol, Toluol, Chlorbenzol, Dichlorbenzole und Methylenchlorid. – Als Phasen-transfer-Katalysatoren kommen alle zu diesem Zweck in der organischen Chemie einsetzbaren Reaktionsbeschleuniger in Betracht. Vorzugsweise verwendbar sind Tetrabutyl-ammonium-bromid und Methyl-tridecyl-ammoniumchlorid (Aliquat®336).

Die für die zweite Stufe des Verfahrens (e) erforderliche Reaktionszeit ist abhängig von der Reaktionstemperatur, dem Wasserstoffpartialdruck, der Intensität der Durchmischung des Reaktionsgemisches und von der Aktivität und Konzentration des Hydrierkatalysators. Im allgemeinen liegt die erforderliche Reaktionszeit im Bereich von 15 Minuten bis zu mehreren Stunden. — Die Aufarbeitung erfolgt jeweils nach üblichen Methoden.

Die bei der Durchführung des Verfahrens (f) als Ausgangsstoffe benötigten Acetylene der Formel (XI) sind bekannt oder lassen sich nach prinzipiell bekannten Verfahren in einfacher Weise herstellen.

Die bei der Durchführung des Verfahrens (f) als Reaktionskomponenten benötigten Säurehalogenide der Formel (XII) sind ebenfalls bekannt oder lassen sich nach prinzipiell bekannten Verfahren herstellen.

Als Katalysatoren kommen bei der Durchführung des Verfahrens (f) alle für derartige Umsetzungen üblichen Reaktionsbeschleuniger in Frage. Vorzugsweise verwendbar sind Kupfersalze, wie z.B. Kupferiodid.

Als Verdünnungsmittel kommen bei der Durchführung des Verfahrens (f) alle für derartige Umsetzungen üblichen inerten organischen Solventien in Betracht. Vorzugsweise verwendbar sind Ether, wie Tetrahydrofuran und Diethylether.

Die Reaktionstemperaturen können bei der Durchführung des Verfahrens (f) innerhalb eines bestimmten Bereiches variiert werden. Im allgemeinen arbeitet man bei Temperaturen zwischen –78°C und +50°C, vorzugsweise zwischen –78°C und +40°C.

Bei der Durchführung des Verfahrens (f) setzt man auf 1 Mol an Acetylen der Formel (XI) im allgemeinen 1 bis 1,2 Mol an Säurehalogenid der Formel (XII) sowie Katalysator ein. Die Aufarbeitung erfolgt nach üblichen Methoden.

Das bei dem Verfahren (c) als Reaktionskomponente benötigte Dimethyl-oxo-sulfonium-methylid der Formel (VI) ist bekannt (vgl. J. Am, Chem. Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kaliumtert.-butylat oder Natriummethylat, in Gegenwart eines Verdünnungsmittels erzeugt.

Das bei dem Verfahren (c) außerdem als Reaktionskomponente in Betracht kommende Dimethylsulfonium-methylid der Formel (VII) ist ebenfalls bekannt (vgl. Heterocycles 8, 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (c) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol,

sowie stark polare Lösungsmittel, wie Dimethylsulfoxid.

Die Reaktionstemperaturen können bei der Durchführung des Verfahrens (c) in einem größeren Bereich variiert werden. Im allgemeinen arbeitet man zwischen 0°C und 100°C, vorzugsweise zwischen 10°C und 60°C.

Bei der Durchführung des Verfahrens (c) setzt man auf 1 Mol an Cyclopropyl-keton der Formel (V) im allgemeinen 1 bis 3 Mol an Dimethyloxosulfonium-methylid der Formel (VI) bzw. an Dimethylsulfonium-methylid der Formel (VII) ein. Die Isolierung der Oxirane der Formel (II) erfolgt nach üblichen Methoden.

Die bei der Durchführung des Verfahrens (d) als Ausgangsstoffe benötigten Carbinole der Formel (VIII) sind bisher noch nicht bekannt. Sie lassen sich herstellen, indem man

g) Halogenketone der Formel

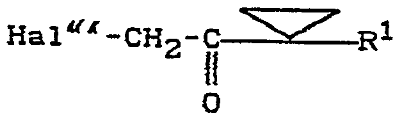

(XIII)

in welcher

$R^1$ die oben angegebene Bedeutung hat und

Hal‴ für Chlor oder Brom steht, mit Acetylensalzen der Formel

$R^2$-C≡C Me (XIV)

in welcher

$R^2$ die oben angegebene Bedeutung hat und

Me für ein Lithiumkation

oder für ein Äquivalent eines Cer(III)-Kations steht

gegebenenfalls in Gegenwart eines Säurebindemittels und in Gegenwart eines Verdünnungsmittels umsetzt.

Die bei dem Verfahren (g) als Ausgangsstoffe benötigten Halogenketone der Formel (XIII) sind teilweise bekannt (BE-A 879 785 und DE-A 2 944 342). Sie lassen sich herstellen, indem man

h) Methyl-cyclopropyl-ketone der Formel

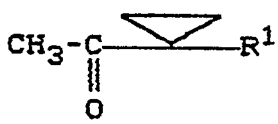

(X)

in welcher

$R^1$ die oben angegebene Bedeutung hat,

mit Chlorierungsmitteln oder Bromierungsmitteln in Gegenwart eines Verdünnungsmittels umsetzt.

Als Chlorierungsmittel und Bromierungsmittel kommen bei dem Verfahren (h) alle für derartige Umsetzungen üblichen Chlorierungs- und Bromierungsreagenzien in Betracht. Vorzugsweise verwendbar sind Sulfurylchlorid, Sulfurylbromid und Brom.

Als Verdünnungsmittel kommen bei dem Verfahren (h) alle für derartige Umsetzungen üblichen inerten organischen Solventien in Frage. Vorzugsweise verwendbar sind halogenierte aliphatische Kohlenwasserstoffe, wie Methylenchlorid, Chloroform und Tetrachlorkohlenstoff.

Die Reaktionstemperaturen können bei dem Verfahren (h) innerhalb eines bestimmten Bereiches variiert werden. Im allgemeinen arbeitet man bei Temperaturen zwischen −10°C und +60°C, vorzugsweise zwischen 0°C und +40°C.

Bei der Durchführung des Verfahrens (h) arbeitet man unter Normaldruck. Es ist jedoch jeweils auch möglich, unter erhöhtem oder vermindertem Druck zu arbeiten.

Bei der Durchführung des Verfahrens (h) setzt man auf 1 Mol an Keton der Formel (X) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß man das Reaktionsgemisch nacheinander mit verdünnter, wäßriger Natriumhydrogencarbonat-Lösung und Wasser wäscht, dann trocknet und einengt.

Die bei dem Verfahren (g) als Reaktionskomponenten benötigten Acetylen-Salze sind durch die Formel (XIV) allgemein definiert. In dieser Formel hat $R^2$ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Acetylen-Salze der Formel (XIV) sind bekannt oder lassen sich nach prinzipiell bekannten Verfahren herstellen.

Als Säurebindemittel kommen bei der Durchführung des Verfahrens (g) alle üblichen Säureakzeptoren in Frage.

Als Verdünnungsmittel kommen bei der Durchführung des Verfahrens (g) alle üblichen inerten organischen Solventien in Betracht. Vorzugsweise verwendbar sind aromatische Kohlenwasserstoffe, wie Toluol, und außerdem Ether, wie Diethylether, Tetrahydrofuran, tert.-Butylmethyl-ether und Gemische dieser Ether.

Die Reaktionstemperaturen können bei der Durchführung des Verfahrens (g) innerhalb eines größeren Bereiches variiert werden. Im allgemeinen arbeitet man bei Temperaturen zwischen –100°C und +100°C, vorzugsweise zwischen –80°C und +50°C.

Das Verfahren (g) wird im allgemeinen unter Normaldruck durchgeführt. Es ist aber auch möglich, unter erhöhtem oder vermindertem Druck zu arbeiten.

Bei der Durchführung des Verfahrens (g) setzt man auf 1 Mol an Halogenketon der Formel (XIII) im allgemeinen 1 bis 3 Mol an Acetylensalz der Formel (XIV) ein. Die Aufarbeitung erfolgt nach üblichen Methoden.

Als Basen kommen bei der Durchführung des Verfahrens (d) alle üblicherweise für derartige Umsetzungen geeigneten organischen und anorganischen Säurebindemittel in Betracht. Vorzugsweise verwendbar sind Alkalicarbonate, wie Natrium- und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kalium-tert, -butylat, und weiterhin niedere tertiäre Alkylamine, Cycloalkylamine und Aralkylamine, wie insbesondere Triethylamin.

Als Verdünnungsmittel kommen bei der Durchführung des Verfahrens (d) alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des Verfahrens (d) innerhalb eines bestimmten Bereiches variiert werden. Im allgemeinen arbeitet man bei Temperaturen zwischen –20°C und +100°C, vorzugsweise zwischen 0°C und 60°C.

Bei der Durchführung des Verfahrens (d) arbeitet man im allgemeinen unter Normaldruck. Es ist jedoch auch möglich, unter erhöhtem oder vermindertem Druck zu arbeiten.

Bei der Durchführung des Verfahrens (d) setzt man auf 1 Mol an Carbinol der Formel (VIII) im allgemeinen 1 bis 3 Mol an Base ein. Die Aufarbeitung erfolgt nach üblichen Methoden.

Die bei der Durchführung des erfindungsgemäßen Verfahrens (a) als Reaktionskomponenten benötigten Azole der Formel (III) sind allgemein bekannte Verbindungen der organischen Chemie.

Als Säurebindemittel kommen bei der Durchführung des erfindungsgemäßen Verfahrens (a) alle üblichen Säureakzeptoren in Frage. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kaliumtert.-butylat, außerdem tertiäre aliphatische oder aromatische Amine, wie Triethylamin, N,N-Dimethyl-cyclohexylamin, N,N-Dimethyl-benzylamin und Pyridin, und außerdem cyclische Amine, wie 1,5-Diaza-bicyclo[4.3.0]-non-5-en (DBN), 1,8-Diaza-bicyclo[5.4.0]undec-7-en (DBU) und 1,4-Diaza-bicyclo[2.2.2]octan (DABCO).

Als Verdünnungsmittel kommen bei der Durchführung des erfindungsgemäßen Verfahrens (a) alle üblichen inerten organischen Solventien in Betracht. Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsauretriamid.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 20°C, vorzugsweise zwischen 50° und 150°C.

Bei der Durchführung des erfindungsgemäßen Verfahrens (a) setzt man vorzugsweise auf 1 Mol an Oxiran der Formel (II) 1 bis 4 Mol an Azol der Formel (III) und 1 bis 2 Mol an Base ein. Die Isolierung der Endprodukte erfolgt in üblicher Weise.

Die für das erfindungsgemäße Verfahren (b) als Ausgangsstoffe benötigten Hydroxyalkylazolyl-Derivate der Formel (Ia) sind erfindungsgemäße Verbindungen. Ihre Überführung in die entsprechenden Alkoholate erfolgt in allgemein bekannter Weise, indem man sie mit geeigneten starken Basen, wie Alkalimetall-amiden oder -hydriden, quarternären Ammonium-hydroxiden oder Phosphonium-hydroxiden in einem inerten Verdünnungsmittel, wie zum Beispiel Dioxan, bei Raumtemperatur umsetzt.

Die bei dem erfindungsgemäßen Verfahren (b) außerdem als Ausgangsstoffe benötigten Halogenverbindungen sind durch die Formel (IV) allgemein definiert. In dieser Formel steht $R^{10}$ vorzugsweise für die Bedeutungen, die bereits im Zusammenhang mit der Beschreibung der erfindungsgemäßen Stoffe der Formel (I) für den Substituenten R genannt wurden, mit Ausnahme der Bedeutung von Wasserstoff.

Die Halogenverbindungen der Formel (IV) sind bekannt oder lassen sich nach im Prinzip bekannten Methoden herstellen.

Als Verdünnungsmittel kommen bei der Durchführung des erfindungsgemäßen Verfahrens (b) inerte organische Lösungsmittel in Frage. Hierzu gehören vorzugsweise Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchführung des erfindungsgemäßen Verfahrens (b) in einem größeren Bereich variiert werden. Im allgemeinen arbeitet man zwischen 0°C und 120°C, vorzugsweise zwischen 20°C und 100°C.

Bei der Durchführung des erfindungsgemäßen Verfahrens (b) setzt man zunächst Hydroxy-Verbindungen der Formel (Ia) mit starken Basen zu den entsprechenden Alkoholaten der Formel (Ib) um. In der danach folgenden Stufe setzt man auf 1 Mol eines Alkoholates der Formel (Ib) vorzugsweise 1 bis 2 Mol Halogenverbindung der Formel (IV) ein.

Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmäßigerweise so verfahren, daß man von einer Hydroxy-Verbindung der Formel (Ia) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (IV) umsetzt, wobei unter Austritt von Alkalimetallhalogenid die erfindungsgemäßen Verbindungen der Formel (I) in einem Arbeitsgang erhalten werden.

Nach einer weiteren bevorzugten Ausführungsform werden zweckmäßigerweise die Herstellung der Alkoholate sowie die Umsetzung mit einer Halogenverbindung der Formel (IV) in einem Zweiphasensystem, wie beispielsweise wäßrige Natron- oder Kalilauge/Toluol oder Methylenchlorid, unter Zusatz von 0,01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die nach den erfindungsgemäßen Verfahren erhältlichen Hydroxyalkyl-azolyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis; Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries; Ustilago-Arten, wie Ustilago nuda oder Ustilago avenae;
Pellicularia-Arten, wie Pellicularia sasakii; Pyricularia-Arten, wie Pyricularia oryzae;
Fusarium-Arten, wie Fusarium culmorum; Botrytis-Arten, wie Botrytis cinerea;

Septoria-Arten, wie Septoria nodorum; Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten. So lassen sich Mehltau- und Rost-Krankheiten, Puccinia recondita, Leptosphaeria nodorum, Pyrenophora teres, Cochliobolus sativus und Pseudocercosporella herpotrichoides an Getreide sowie Pyricularia und Pellicularia an Reis besonders gut bekämpfen. Die Stoffe besitzen außerdem eine sehr gute in-vitro-Wirkung.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergarten, Park- und Sportanlagen, an Straßenrändern, auf Flughafen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung der Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefordert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefordert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Vorzugsweise lassen sich die erfindungsgemäßen Stoffe zur Wuchshemmung bei Getreide und Gras verwenden.

Die Wirkstoffe konnen in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel konnen z.B. auch organische Losungsmittel als Hilfslosungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser: mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, kornige oder latexformige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001%. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50kg, bevorzugt 0,05 bis 10kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

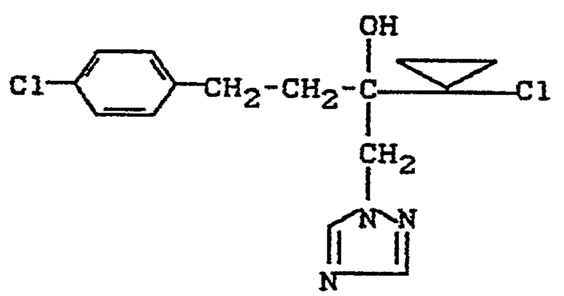

(I-1)

In ein Gemisch aus 10 g Kaliumcarbonat, 15 g (0,22 Mol) 1,2,4-Triazol und 50ml Acetonitril wird unter Stickstoffatmosphäre eine Lösung von 13,4 g (0,05 Mol) 2-(1-Chlorcyclopropyl)-2-(4-chlorphenylethyl)-oxiran in 20 ml Acetonitril eingetropft, wobei das Reaktionsgemisch unter Rückfluß erhitzt wird. Nach beendeter Zugabe rührt man das Reaktionsgemisch noch 8 Stunden unter Rückfluß, saugt dann vom Rückstand ab und engt das Filtrat durch Abziehen des Lösungsmittels unter vermindertem Druck ein. Man nimmt den verbleibenden Rückstand in Essigester auf, wäscht die organische Phase mit Wasser, trocknet über Natriumsulfat und engt durch Abziehen des Lösungsmittels unter vermindertem Druck ein. Der anfallende Rückstand wird mit Chloroform/Ethanol = 98:2 als Laufmittel über eine Kieselgel-Säule chromatographiert. Nach Umkristallisation des dabei verbleibenden Produktes aus Cyclohexan erhält man 5,1 g (30% der Theorie) an 2-(1-Chlorcyclopropyl)-4-(4-chlorphenyl)-1-(1,2,4-triazol-1-yl)-butan-2-ol in Form einer Festsubstanz vom Schmelzpunkt 137°C.

Herstellung von Ausgangssubstanzen:

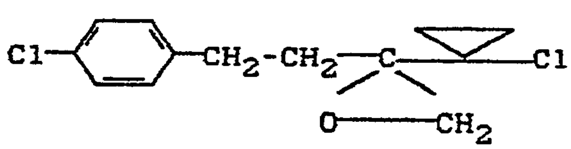

(II-1)

16 ml (0,22 Mol) Dimethylsulfid und 24,2 g (0,19 Mol) Dimethylsulfat werden in 30 ml tert.-Butanol gegeben und 14 Stunden bei 20°C stehengelassen. Unter Rühren tropft man in das Reaktionsgemisch zunächst eine Lösung von 17 g (0,07 Mol) 1-Chlorcyclopropyl-4-chlorphenylethylketon in 70 ml tert.-Butanol und trägt dann 22 g Kaliumhydroxid-Pulver ein, wobei die Temperatur des Reaktionsgemisches auf 20 bis 30°C gehalten wird. Man rührt noch 3 Stunden bei 30°C, zieht dann das Dimethylsulfid unter vermindertem Druck ab und gießt das Reaktionsgemisch danach auf 50 ml einer 1%igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter vermindertem Druck eingeengt. Man erhält auf diese Weise 13,4 g (75% der Theorie) an 2-(1-Chlorcyclopropyl)-2-(4-chlorphenylethyl)-oxiran in Form eines öligen Produktes, das ohne zusätzliche Reinigung weiter umgesetzt wird.

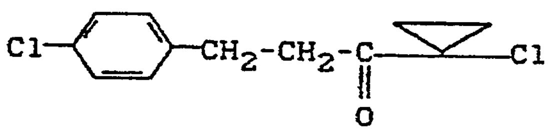

(V-1)

In einen 100 ml Autoklaven werden 460 mg (0,5mmol) Tris-triphenylphosphin-rhodiumchlorid (= 1 Mol-%, bezogen auf die Reaktionskomponenten) gegeben. Nach dem Spülen mit Stickstoff wird eine luftfreie Lösung von 12 g (0,05 Mol) 1-Chlorcyclopropyl-4-chlorphenylethenyl-keton in 40 ml Toluol hinzugefügt und unter einem Wasserstoffdruck von 30 bar auf 50°C erhitzt. Der Wasserstoffdruck wird zwischen 40 und 50 bar gehalten bis die Gasaufnahme beendet ist (nach etwa 1 Stunde). Anschließend läßt man 1 Stunde nachreagieren. Zur Aufarbeitung wird das Lösungsmittel unter vermindertem Druck abgezogen und der verbleibende Rückstand mit Dichlormethan als Laufmittel an Kieselgel gereinigt. Man erhalt 11g (90% der Theorie) an 1-Chlorcyclopropyl-4chlorphenylethyl-keton in Form eines öligen Produktes.

$^1$H-NMR (200 MHz, $CDCl_3$):
$\delta$ = 1,28–1,38 (m, 2H), 1,57–1,67 (m, 2H), 2,84 (t, 2H), 3,15 (t, 2H), 7,08–7,29 (m, 4H).

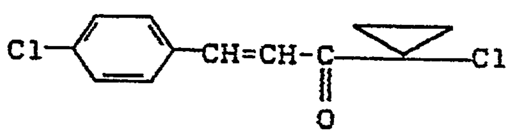

(V-2)

In ein Gemisch von 60 g (0,5 Mol) 1-Chlorcyclopropylmethyl-keton, 70 g (0,5 Mol) 4-Chlorbenzaldehyd und 250 ml Ethanol werden bei Raumtemperatur 50 ml Wasser und 10 Plätzchen festes Natriumhydroxid gegeben. Es wird 16 Stunden bei Raumtemperatur gerührt. Danach wird der ausgefallene Feststoff angesaugt. Man erhält auf diese Weise 108,5 g (89% der Theorie) an 1-Chlorcyclopropyl-4-chlorphenylethenyl-keton in Form einer Festsubstanz vom Schmelzpunkt 92°C.

Beispiel 2

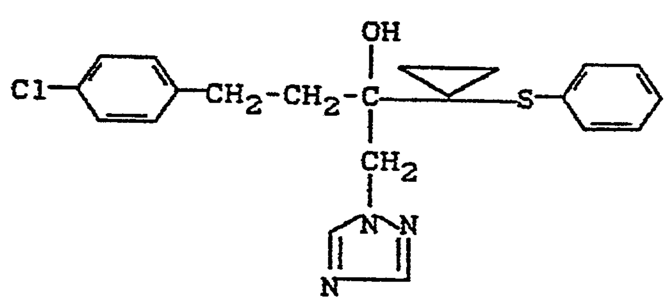

(I-2)

In ein Gemisch aus 14 g Kaliumcarbonat, 21 g (0,3 Mol) 1,2,4-Triazol und 70 ml Acetonitril wird unter Stickstoffatmosphäre eine Lösung von 25,6 g (0,1 Mol) 2-(4-Chlorphenylethyl)-2-(1-phenylmercapto-cyclopropyl)-oxiran in 30 ml Acetonitril eingetropft, wobei das Reaktionsgemisch unter Rückfluß erhitzt wird. Nach beendeter Zugabe rührt man noch 8 Stunden unter Rückfluß, saugt dann vom Rückstand ab und engt das Filtrat durch Abziehen des Lösungsmittels unter vermindertem Druck ein. Man nimmt den verbleibenden Rückstand in Essigester/Toluol auf, wäscht die organische Phase mit Wasser, trocknet

über Natriumsulfat und engt durch Abziehen des Lösungsmittels unter vermindertem Druck ein. Der anfallende Rückstand wird mit Chloroform/Ethanol = 99:1 als Laufmittel über eine Kieselgel-Säule chromatographiert. Nach Umkristallisation des verbleibenden Rückstandes aus Cyclohexan erhält man 11,1 g (37% der Theorie) an 4-(4-Chlorphenyl)-2-(1-phenylmercapto-cyclopropyl)-1-(1,2,4-triazol-1-yl)-butan-2-ol in Form eines Feststoffes vom Schmelzpunkt 137°C.

Herstellung von Ausgangssubstanzen:

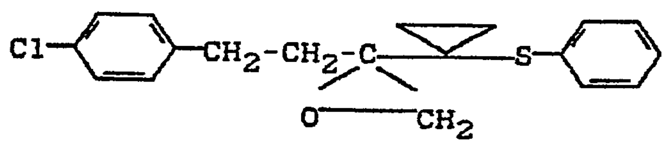

(II-2)

21 ml (0,29 Mol) Dimethylsulfid und 32,5 g (0,26 Mol) Dimethylsulfat werden in 40 ml tert.-Butanol gegeben und 14 Stunden bei Raumtemperatur stehengelassen. unter Rühren tropft man in das Reaktionsgemisch zunächst eine Lösung von 30 g (0,095 Mol) 4-Chlorphenylethyl-1-phenylmercapto-cyclopropylketon in 90ml tert.-Butanol und trägt dann 29,2 g Kaliumhydroxid-Pulver ein, wobei die Temperatur des Reaktionsgemisches auf 20 bis 30°C gehalten wird. Man rührt noch 3 Stunden bei 30°C, zieht dann das Dimethylsulfid unter vermindertem Druck ab und gießt das Reaktionsgemisch danach auf 70 ml einer 1%igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter vermindertem Druck eingeengt. Man erhält auf diese Weise 25,6 g (82% der Theorie) an 2-(4-Chlorphenylethyl)-2-(1-phenylmercapto-cyclopropyl)-oxiran in Form eines öligen Produktes, das ohne zusätzliche Reinigung weiter umgesetzt wird.

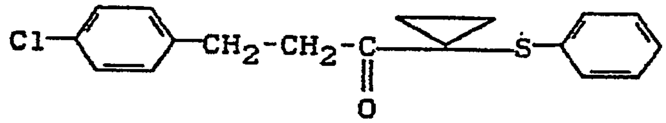

(V-3)

In einen 100 ml Autoklaven werden 460 mg (0,5 mmol) Tris-triphenylphosphin-rhodiumchlorid (= 1 Mol-%, bezogen auf die Reaktionskomponente) gegeben. Nach dem Spülen mit Stickstoff wird eine luftfreie Lösung von 15,7 g (0,05 Mol) 4-Chlorphenylethenyl-1-phenylmercaptocyclopropyl-keton in 40 ml Toluol hinzugefügt und unter einem Wasserstoffdruck von 30 bar auf 50°C erhitzt. Der Wasserstoffdruck wird zwischen 40 und 50 bar gehalten bis die Gasaufnahme beendet ist. Anschließend läßt man 1 Stunde nachreagieren. Zur Aufarbeitung wird das Lösungsmittel unter vermindertem Druck abgezogen und der verbleibende Rückstand mit Dichlormethan als Laufmittel an Kieselgel gereinigt. Man erhält 14,2 g (90% der Theorie) an 4-Chlorphenylethyl-1-phenylmercaptocyclopropyl-keton in Form eines öligen Produktes,
$^1$H-NMR (200 MHz, $CDCl_3$):
δ = 1,25–1,35 (m, 2H), 1,75–1,90 (m, 2H), 2,79 (t, 2H), 3,18 (t, 2H), 6,95–7,45 (m, 9H).

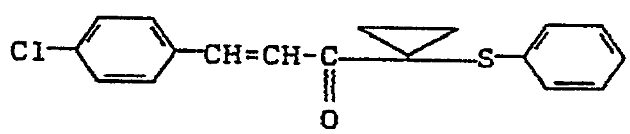

(V-4)

In ein Gemisch von 75 g (0,39 Mol) 1-Phenylmercaptocyclopropyl-methyl-keton, 56 g (0,39 Mol) 4-Chlorbenzaldehyd und 200 ml Ethanol werden bei Raumtemperatur 50 ml Wasser und 8 Plätzchen festes Natriumhydroxid gegeben. Es wird 14 Stunden bei Raumtemperatur gerührt. Danach wird der ausgefallene Feststoff angesaugt. Man erhält auf diese Weise 120,3 g (98% der Theorie) an 4-Chlorphenylethenyl-1-phenylmercapto-cyclopropyl-keton in Form einer Festsubstanz.
$^1$H-NMR (200 MHz, $CDCl_3$):
δ = 1,32–1,41 (m, 2H), 1,87–1,95 (m, 2H), 7,05–7,84 (m, 11H).

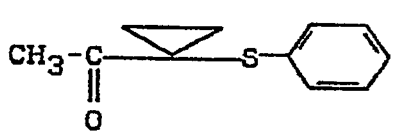

(X-1)

In eine Lösung von 100 g (0,83 Mol) 5-Chlorpentan-2-on in 400 ml Methylenchlorid wird bei 10°C unter Rühren eine Lösung von 134 g (0,83 Mol) Brom in 130 ml Methylenchlorid eingetropft. Das Reaktionsgemisch wird 1 Stunde bei Raumtemperatur nachgerührt, dann mit Wasser und verdünnter, wäßriger Natriumcarbonat-Lösung gewaschen und über Natriumsulfat getrocknet. Man engt das Gemisch durch Abziehen des Lösungsmittels unter vermindertem Druck ein, nimmt den Rückstand in 200 ml Methanol auf und gibt bei 5°C unter Rühren 91,5 g (0,83 Mol) Thiophenol hinzu. Danach wird ein Gemisch aus 93 g Kaliumhydroxid-Pulver in 500 ml Methanol zugetropft. Das Reaktionsgemisch wird zunachst 2 Stunden bei Raumtemperatur und dann 4 Stunden bei 40°C gerührt. Anschließend wird durch Abziehen des Lösungsmittels unter vermindertem Druck eingeengt und der verbleibende Rückstand in Methylenchlorid aufgenommen. Die organische Lösung wird nacheinander mit Wasser, verdünnter wäßriger Natronlauge und wieder mit Wasser gewaschen, danach unter vermindertem Druck eingeengt und dann einer Vakuumdestillation unterworfen. Man erhält auf diese Weise 76,8 g (48% der Theorie) an Methyl-1-phenylmercaptocyclopropyl-keton vom Siedepunkt 155°C/20 mbar.

Beispiel 3 und 4

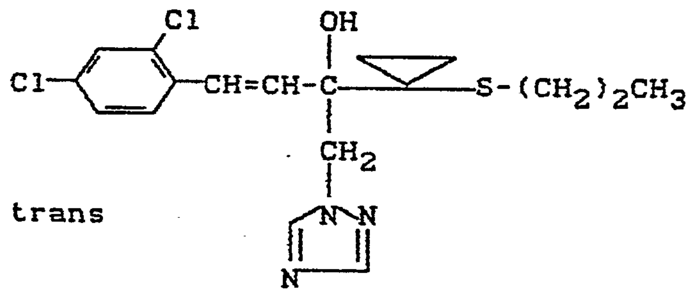

(I-3)

In ein Gemisch aus 24 g (0,35 Mol) 1,2,4-Triazol, 2,7 g (0,02 Mol) Kalium-tert.-butylat und 50ml Dimethylformamid wird bei 80°C unter Stickstoffatmosphäre und unter Rühren eine Lösung von 38,2 g (0,12 Mol) 2-(2,4-Dichlorphenylethenyl)-2-(1-propylmercaptocyclopropyl)-oxiran in 30 ml Dimethylformamid eingetropft. Man rührt 6 Stunden bei 80°C, zieht dann das Lösungsmittel unter vermindertem Druck ab, nimmt den Rückstand in Essigester/Toluol auf, wäscht mit Wasser, trocknet über Natriumsulfat und engt unter vermindertem Druck ein. Der verbleibende Rückstand wird mit Chloroform als Laufmittel an Kieselgel chromatographiert. Man erhält auf diese Weise 12,1 g (26% der Theorie) an trans-4-(2,4-Dichlorphenyl)-2-(1-propylmercaptocyclopropyl)-1-(1,2,4-triazol-1-yl)-but-3-en-2-ol in Form eines öligen Produktes.

$^1$H-NMR (200 MHz, $CDCl_3$):
$\delta$ = 0,75–1,65 (m, 9H), 2,63 (t, 2H), 4,62 (d, 1H), 4,73 (d, 1H), 6,30 (d, 1H), 6,90 (d, 1H), 7,12–7,42 (m, 3H), 7,90 (s, 1H), 8,17 (s, 1H).

Außerdem werden 1,8 g (4% der Theorie) an cis-4-(2,4-Dichlorphenyl)-2-(1-propylmercaptocyclopropyl)-1-(1,2,4-triazol-1-yl)-but-3-en-2-ol isoliert (Verbindung I-4).

$^1$H-NMR (200 MHz, $CDCl_3$):
$\delta$ = 0,75–1,10 (m, 7H), 1,50 (sex, 2H), 2,40 (t, 2H), 4,35–4,53 (m, 3H), 5,97 (d, 1H), 7,00 (d, 1H), 7,20–7,44 (m, 3H), 7,92 (s, 1H), 8,13 (s, 1H).

Herstellung von Ausgangssubstanzen:

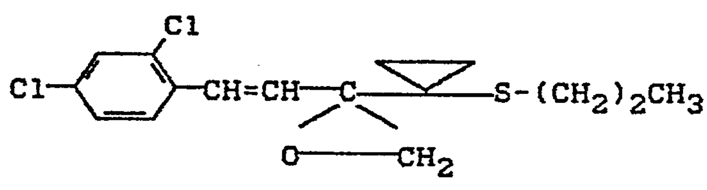

(II-3)

30 ml (0,41 Mol) Dimethylsulfid und 43,5 g (0,35 Mol) Dimethylsulfat werden in 60 ml tert.-Butanol gegeben und 14 Stunden bei Raumtemperatur stehengelassen. Unter Rühren tropft man in das Reaktionsgemisch zunächst eine Lösung von 40 g (0,13 Mol) 2,4-Dichlorphenylethenyl-1-propylmercaptocyclopropyl-keton in 120 ml tert.-Butanol und trägt dann 39,1 g Kaliumhydroxid-Pulver ein, wobei die Temperatur des Reaktionsgemisches auf 20 bis 30°C gehalten wird. Man rührt noch 3 Stunden bei 30°C, zieht dann das Dimethylsulfid unter vermindertem Druck ab und gießt das Reaktionsgemisch danach auf 70 ml

einer 1%igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter vermindertem Druck eingeengt. Man erhält auf diese Weise 38,2 g (91% der Theorie) an 2-(2,4-Dichlorphenylethenyl)-2-(1-propylmercaptocyclopropyl)-oxiran in Form eines öligen Produktes, das ohne zusätzliche Reinigung weiter umgesetzt wird.

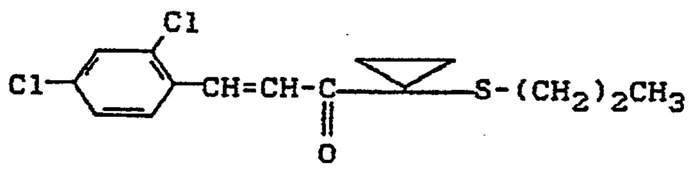
(V-5)

In ein Gemisch von 41,7 g (0,26 Mol) 1-Acetyl-1-propylmercapto-cyclopropan, 46 g (0,26 Mol) 2,4-Dichlorbenzaldehyd, 130 ml Ethanol und 30 ml Dichlormethan werden bei Raumtemperatur 5 Plätzchen festes Natriumhydroxid gegeben. Es wird 14 Stunden bei Raumtemperatur nachgerührt. Man versetzt mit 50 ml Wasser, trennt die Ölphase ab und nimmt in Dichlormethan auf. Die organische Phase wird mit Wasser gewaschen, über Natriumsulfat getrocknet und durch Abziehen des Lösungsmittels unter vermindertem Druck eingeengt. Man erhält auf diese Weise 80 g (97% der Theorie) an 2,4-Dichlorphenyl-ethenyl-1-propylmercaptocyclopropyl-keton (cis-trans-Isomere) in Form eines öligen Produktes.
$^{1}$H-NMR (200 MHz, $CDCl_3$):
δ = 0,95 (t, 3H), 1,19–1,29 (m, 2H), 1,50–1,70 (m, 4H), 2,57 (t, 2H), 7,20–7,95 (m, 5H).

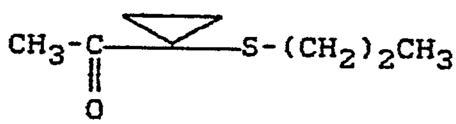
(X-2)

In eine Lösung von 100 g (0,83 Mol) 5-Chlorpentan-2-on in 400 ml Methylenchlorid wird bei 10°C unter Rühren eine Lösung von 134 g (0,83 Mol) Brom in 130 ml Methylenchlorid eingetropft. Das Reaktionsgemisch wird 1 Stunde bei Raumtemperatur nachgerührt, dann mit Wasser und verdünnter, wäßriger Natriumcarbonat-Lösung gewaschen und über Natriumsulfat getrocknet. Man engt das Gemisch durch Abziehen des Lösungsmittels unter vermindertem Druck ein, nimmt den Rückstand in 200 ml Methanol auf und gibt bei 5°C unter Rühren 63 g (0,83 Mol) n-Propylmercaptan hinzu. Danach wird ein Gemisch aus 93 g Kaliumhydroxid-Pulver in 500 ml Methanol zugetropft. Das Reaktionsgemisch wird zunächst 2 Stunden bei Raumtemperatur und dann 4 Stunden bei 40°C gerührt. Anschließend wird durch Abziehen des Lösungsmittels unter vermindertem Druck eingeengt und der verbleibende Rückstand in Methylenchlorid aufgenommen. Die organische Lösung wird nacheinander mit Wasser, verdünnter wäßriger Natronlauge und wieder mit Wasser gewaschen, danach unter vermindertem Druck eingeengt und dann einer Vakuumdestillation unterworfen. Man erhält auf diese Weise 42 g (32% der Theorie) an 1-Acetyl-1-propylmercapto-cyclopropan vom Siedepunkt 107°C/20 mbar.

Beispiel 5

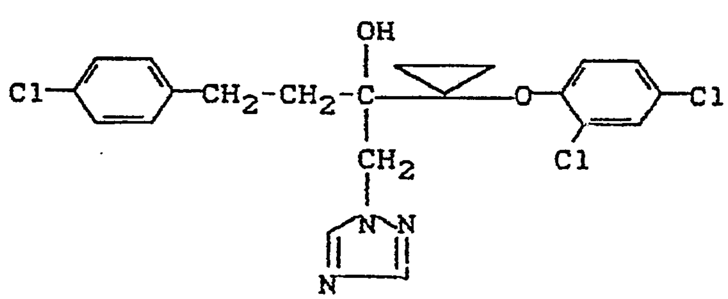
(I-5)

Nach den zuvor angegebenen Methoden wird auch 4-(4-Chlorphenyl)-2-[1-(2,4-dichlorphenoxy)-cyclopropyl]-1(1,2,4-triazol-1-yl)-butan-2-ol in Form eines öligen Produktes erhalten.
$^{1}$H-NMR (200 MHz, $CDCl_3$):
δ = 0,25–0,45 (m, 2H), 0,67-0,83 (m, 1H), 0,83–1,05 (m, 1H), 1,80-2,15 (m, 2H), 2,70–2,90 (m, 1H), 2,90–

3,13 (m, 1H), 4,16 (s, 1H), 4,38 (d, 1H), 4,70 (d, 1H), 7,00–7,40 (m, 7H), 8,02 (s, 1H), 8,38 (s, 1H).

Nach den Methoden, die in den zuvor angegebenen Beispielen beschrieben wurden, wurden auch die in der folgenden Tabelle 3 aufgeführten Stoffe erhalten.

Tabelle 3

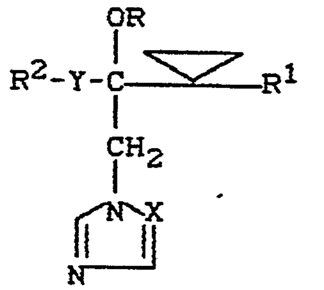

(I)

| Verbindung Nr. | $R^2$ | Y | $R^1$ | R | X | Charakterisierung durch | |
|---|---|---|---|---|---|---|---|
| I-6 | 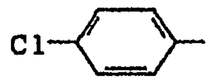 | $-CH_2-CH_2-$ | $-SCH_3$ | H | N | Fp = 115° C | |
| I-7 | 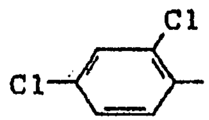 | $-CH_2-CH_2-$ | $-S(CH_2)_2CH_3$ | H | N | NMR-Spektrum | Abb. 1 |
| I-8 | 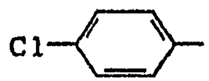 | $-CH_2-CH_2-$ | $-SCH(CH_3)_2$ | H | N | NMR-Spektrum | Abb. 2 |
| I-9 | 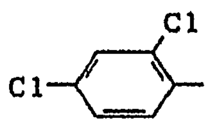 | $-CH_2-CH_2-$ | $-SC_2H_5$ | H | N | NMR-Spektrum | Abb. 3 |
| I-10 | 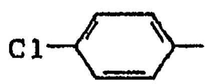 | $-CH_2-CH_2-$ | $-S-CH_2-$ 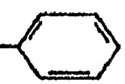 | H | N | NMR-Spektrum | Abb. 4 |
| I-11 | 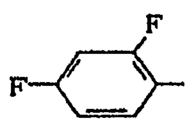 | $-CH_2-CH_2-$ | Cl | H | N | NMR-Spektrum | Abb. 5 |

| Verbindung Nr. | $R^2$ | Y | $R^1$ | R | X | Charakterisierung durch | |
|---|---|---|---|---|---|---|---|
| I-12 | 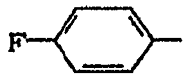 | $-CH_2-CH_2-$ | Cl | H | N | NMR-Spektrum | Abb. 6 |
| I-13 | 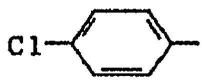 | -CH=CH- | $-SCH_3$ | H | N | NMR-Spektrum | Abb. 7 |
| I-14 | 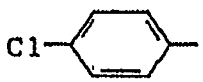 | -CH=CH- (trans) | 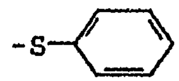 | H | N | NMR-Spektrum | Abb. 8 |
| I-15 | 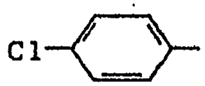 | -CH=CH- (cis) | 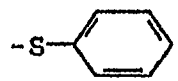 | H | N | NMR-Spektrum | Abb. 9 |
| I-16 | 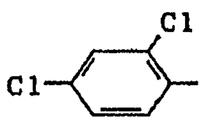 | -CH=CH- | $-SC_2H_5$ | H | N | NMR-Spektrum | Abb. 10 |
| I-17 | 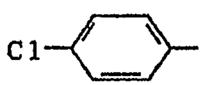 | 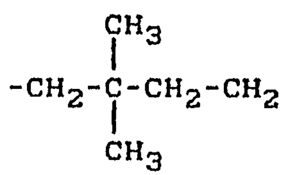 | Cl | H | N | NMR-Spektrum | Abb. 11 |

| Verbindung Nr. | $R^2$ | Y | $R^1$ | R | X | Charakterisierung durch | |
|---|---|---|---|---|---|---|---|
| I-18 | 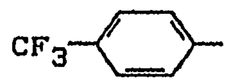 | $-CH_2-CH_2-$ | Cl | H | N | NMR-Spektrum | Abb. 12 |
| I-19 | 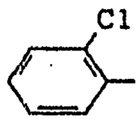 | $-CH_2-CH_2-$ | Cl | H | N | Fp: 100° C | |
| I-20 | 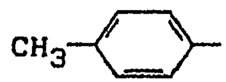 | $-CH_2-CH_2-$ | Cl | H | N | Fp: 118° C | |
| I-21 | 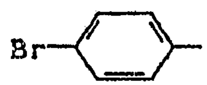 | $-CH_2-CH_2-$ | Cl | H | N | Fp: 132° C | |
| I-22 | 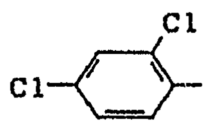 | $-CH_2-CH_2-$ | Cl | H | N | Fp: 108° C | |
| I-23 | 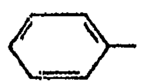 | $-CH_2-CH_2-$ | Cl | H | N | Fp: 94° C | |

| Verbindung Nr. | $R^2$ | Y | $R^1$ | R | X | Charakterisierung durch | |
|---|---|---|---|---|---|---|---|
| I-24 | 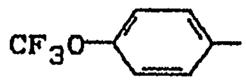 | $-CH_2-CH_2-$ | Cl | H | N | NMR-Spektrum | Abb. 13 |
| I-25 | 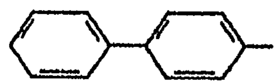 | $-CH_2-CH_2-$ | Cl | H | N | Fp: 128° C | |
| I-26 | 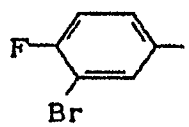 | $-CH_2-CH_2-$ | Cl | H | N | NMR-Spektrum | Abb. 14 |
| I-27 | 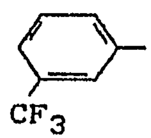 | $-CH_2-CH_2-$ | Cl | H | N | NMR-Spektrum | Abb. 15 |
| I-28 | 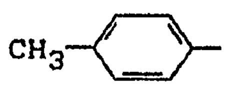 | -CH=CH- | Cl | H | N | Fp: 106° C | |
| I-29 | 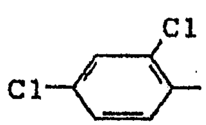 | -CH=CH- | Cl | H | N | Fp: 112° C | |

| Verbindung Nr. | $R^2$ | Y | $R^1$ | R | X | Charakterisierung durch |
|---|---|---|---|---|---|---|
| I-30 | 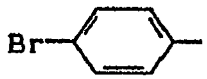 | -CH=CH- | Cl | H | N | Fp: 130° C |
| I-31 | 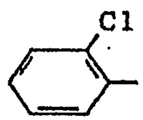 | -CH=CH- | Cl | H | N | Fp: 133° C |
| I-32 | 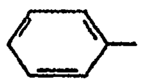 | -CH=CH- | Cl | H | N | Fp: 90° C |
| I-33 | 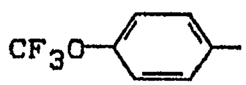 | -CH=CH- | Cl | H | N | Fp: 67° C |
| I-34 | 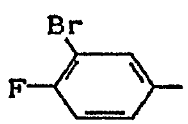 | -CH=CH- | Cl | H | N | Fp: 126° C |
| I-35 | 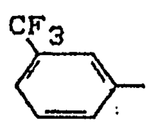 | -CH=CH- | Cl | H | N | NMR-Spektrum Abb. 16 |

| Verbindung Nr. | $R^2$ | Y | $R^1$ | R | X | Charakterisierung durch |
|---|---|---|---|---|---|---|
| I-36 | 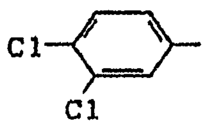 | -CH=CH- | Cl | H | N | NMR-Spektrum Abb. 17 |
| I-37 | 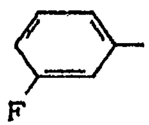 | -CH=CH- | Cl | H | N | NMR-Spektrum Abb. 18 |
| I-38 | $(CH_3)_3C-$ | -CH=CH- | Cl | H | N | Fp: 74° C |
| I-39 | 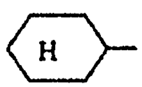 | -CH=CH- | Cl | H | N | Fp: 95° C |
| I-40 | 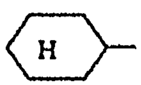 | $-CH_2-CH_2-$ | Cl | H | N | Fp: 67° C |

| Verbindung Nr. | $R^2$ | Y | $R^1$ | R | X | Charakterisierung durch |
|---|---|---|---|---|---|---|
| I-41 | 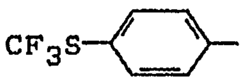 | $-CH=CH-$ | Cl | H | N | $R_f$ = 0,32 ($CH_2Cl_2$/Essigester = 4:1) |
| I-42 | 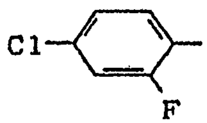 | $-CH=CH-$ | Cl | H | N | $F_p$: 92° C |
| I-43 | 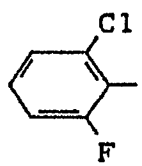 | $-CH=CH-$ | Cl | H | N | $F_p$: 112° C |
| I-44 | 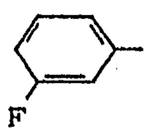 | $-CH_2-CH_2-$ | Cl | H | N | $R_f$ = 0,34 ($CH_2Cl_2$ / Essigester = 4:1) |
| I-45 | 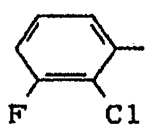 | $-CH=CH-$ | Cl | H | N | $F_p$: 112° C |

| Verbindung Nr. | $R^2$ | Y | $R^1$ | R | X | Charakterisierung durch |
|---|---|---|---|---|---|---|
| I-46 | 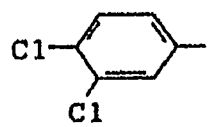 | $-CH_2CH_2-$ | Cl | H | N | Fp: 137°C |
| I-47 | 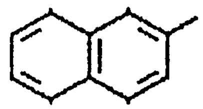 | -CH=CH- | Cl | H | N | Fp: 114-116°C |
| I-48 | 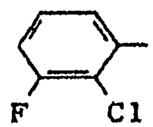 | $-CH_2-CH_2-$ | Cl | H | N | Fp: 84°C |
| I-49 | 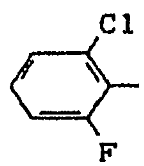 | $-CH_2-CH_2-$ | Cl | H | N | Fp: 72°C |
| I-50 | 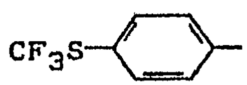 | $-CH_2-CH_2-$ | Cl | H | N | Fp: 88°C |

| Verbin-dung Nr. | $R^2$ | Y | $R^1$ | R | X | Charakterisierung durch |
|---|---|---|---|---|---|---|
| I-51 | 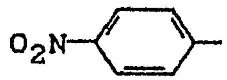 | $-CH_2-CH_2-$ | Cl | H | N | Fp: 105° C |
| I-52 | 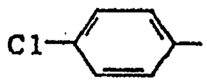 | -CH=CH- | Cl | H | N | Fp: 122° C |
| I-53 | 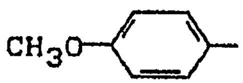 | $-CH_2-CH_2-$ | Cl | H | N | Fp: 90° C |
| I-54 | 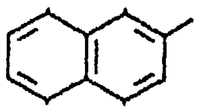 | $-CH_2-CH_2-$ | Cl | H | N | Fp: 134° C |
| I-55 | 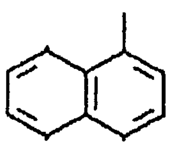 | $-CH_2-CH_2-$ | Cl | H | N | Fp: 102° C |

| Verbindung Nr. | $R^2$ | Y | $R^1$ | R | X | Charakterisierung durch |
|---|---|---|---|---|---|---|
| I-56 | 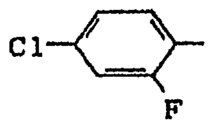 | $-CH_2-CH_2-$ | Cl | H | N | Fp: 112° C |
| I-57 | 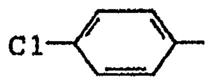 | $-C{\equiv}C-$ | Cl | H | N | Fp: 112° C |
| I-58 | 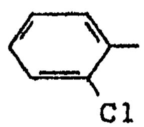 | $-C{\equiv}C-$ | Cl | H | N | Öl |
| I-59 | 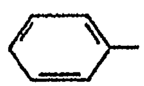 | $-C{\equiv}C-$ | Cl | H | N | Fp: 116-118° C |
| I-60 | 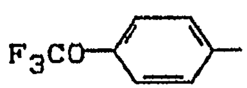 | $-CH_2-CH_2-$ | 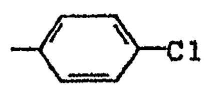 | H | N | Fp: 132° C |

| Verbindung Nr. | $R^2$ | Y | $R^1$ | R | X | Charakterisierung durch |
|---|---|---|---|---|---|---|
| I-61 | 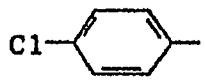 | $-CH_2-CH_2-$ | 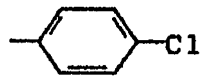 | H | N | Fp: 123° C |
| I-62 | 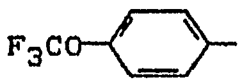 | -CH=CH- | 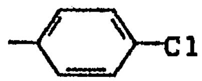 | H | N | Fp: 82° C |
| I-63 | 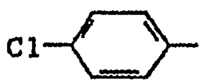 | -CH=CH- | 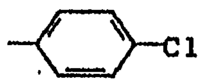 | H | N | Fp: 146° C |

In den folgenden Verwendungsbeispielen wurden die Verbindungen der nachstehend angegebenen Formeln als Vergleichssubstanzen eingesetzt:

(A) =

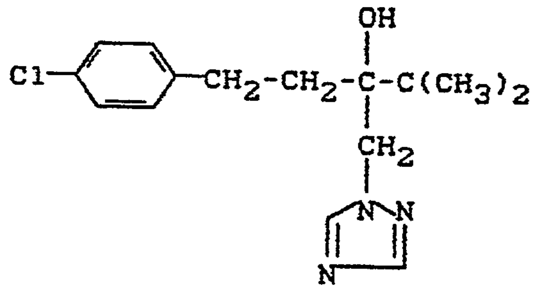

(Bekannt aus EP-A 0 040 345)

(B) =

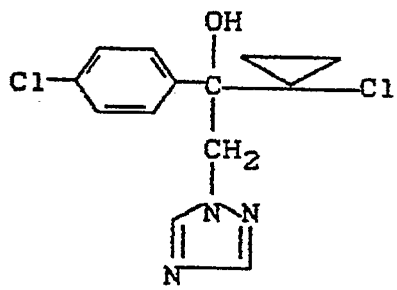

(bekannt aus EP-A 0 180 136)

(C) =

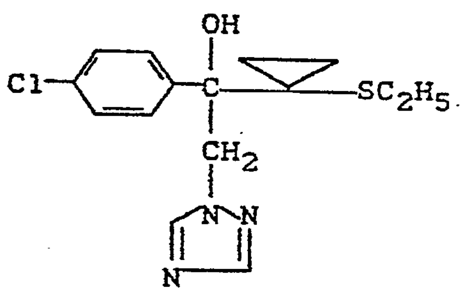

(bekannt aus EP-A 0 180 136)

(D) =

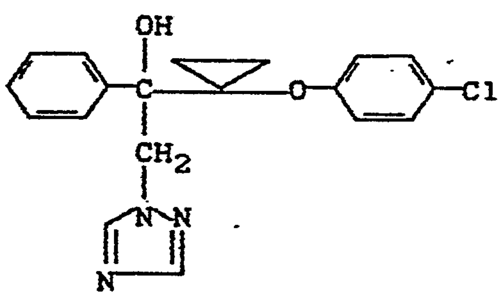

(bekannt aus EP-A 0 180 136).

(E) = 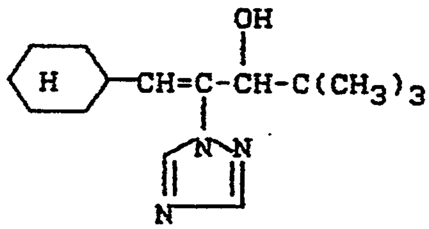

(bekannt aus EP-A 0 015 387)

(F) = 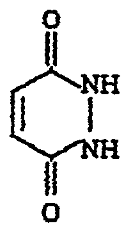

(bekannt)

$$(G) : Cl-CH_2-CH_2-\overset{\ominus}{N}(CH_3)_3 \quad Cl^{\ominus}$$

(bekannt)

Beispiel A

Venturia-Test (Apfel)/protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

In diesem Test zeigt die erfindungsgemäße Verbindung (I-1) eine bessere Wirksamkeit als die Vergleichssubstanzen (A) und (B).

Beispiel B

Cochliobolus sativus-Test (Gerste)/protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt.

7 Tage nach der Inokulation erfolgt die Auswertung.

In diesem Test zeigen die erfindungsgemäßen Verbindungen (I-1) und (I-5) eine bessere Wirksamkeit als die Vergleichssubstanzen (C) und (D).

Beispiel C

Wachstum bei Weizen

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Weizenpflanzen werden im Gewächshaus bis zum Zweiblatt-Stadium angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100% Wachstum ein Wachstum entsprechend dem der Kontrollpflanzen und 0% den Stillstand des Wachstums, Werte über 100% kennzeichnen eine Wuchsförderung.

In diesem Test zeigt die erfindungsgemäße Verbindung (I-38) eine wesentlich bessere Wirksamkeit als die Vergleichssubstanz (E).

Beispiel D

Wachstum bei Gerste

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration.

Gerstepflanzen werden im Gewächshaus bis zum Zweiblatt-Stadium angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100% Wachstum ein Wachstum entsprechend dem der Kontrollpflanzen und 0% den Stillstand des Wachstums. Werte über 100% kennzeichnen eine Wuchsförderung.

In diesem Test zeigt die erfindungsgemäße Verbindung (I-38) eine sehr gute wuchshemmende Wirkung.

Beispiel E

Wachstum bei Gras (Festuca pratensis)

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100% Wachstum ein Wachstum entsprechend dem der Kontrollpflanzen und 0% den Stillstand des Wachstums, Werte über 100% kennzeichnen eine Wuchsförderung.

In diesem Test zeigt die erfindungsgemäße Verbindung (I-38) eine wesentlich bessere wuchshemmende Wirkung als die Vergleichssubbtanzen (F) und (E).

Beispiel F

Wachstum bei Roggen

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Roggenpflanzen werden im Gewächshaus bis zum Zweiblatt-Stadium angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeutet 100% Wachstum ein Wachstum entsprechend dem der Kontrollpflanzen und 0% den Stillstand des Wachstums, Werte über 100% kennzeichnen eine Wuchsförderung.

In diesem Test zeigt die erfindungsgemäße Verbindung (I-38) eine wesentlich bessere wuchshemmende Wirkung als die Vergleichssubstanz (G).

**Patentansprüche**

1. Hydroxyalkyl-azolyl-Derivate der Formel

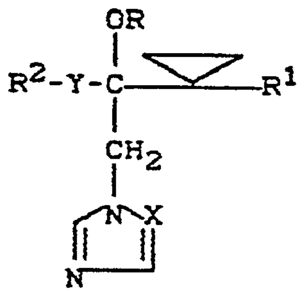

(I)

in welcher

R für Wasserstoff, Alkyl mit 1 bis 6 Kohlenstoffatomen oder Alkylcarbonyl mit 1 bis 6 Kohlenstoffatomen im Alkylteil steht,

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin

Z für Sauerstoff, Schwefel, SO oder $SO_2$ steht und

$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,

$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

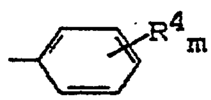

steht, worin

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und

m für die Zahlen 0, 1, 2 oder 3 steht,

X für Stickstoff oder eine CH-Gruppe steht und

Y für die Gruppierungen $-CH_2-CH_2-$, $-CH=CH-$, $-C{\equiv}C-$,

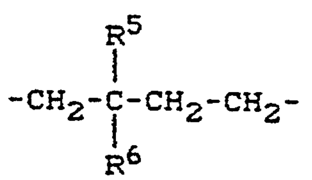 oder 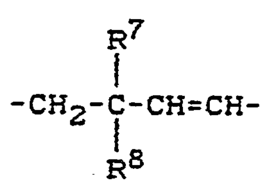 steht,

steht, worin

$R^5$, $R^6$, $R^7$ und $R^8$ unabhängig voneinander für Wasserstoff, Methyl oder Ethyl stehen,

sowie deren Säureadditionssalze und Metallsalzkomplexe.

2. Verfahren zur Herstellung von Hydroxyalkyl-azolyl-Derivaten der Formel

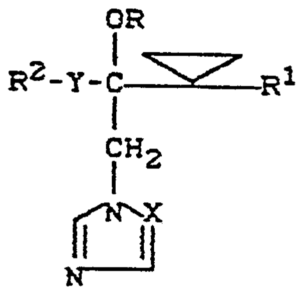

(I)

in welcher

R für Wasserstoff, Alkyl mit 1 bis 6 Kohlenstoffatomen oder Alkylcarbonyl mit 1 bis 6 Kohlenstoffatomen im Alkylteil steht,

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin

Z für Sauerstoff, Schwefel, SO oder $SO_2$ steht und

$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,

$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

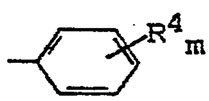

steht, worin

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und

m für die Zahlen 0, 1, 2 oder 3 steht,

X für Stickstoff oder eine CH-Gruppe steht und

Y für die Gruppierungen $-CH_2-CH_2-$, $-CH=CH-$, $-C\equiv C-$,

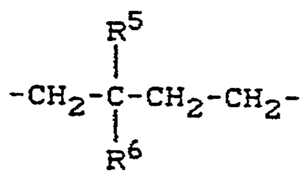 oder 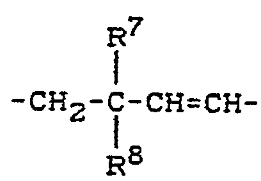 steht,

steht, worin

$R^5$, $R^6$, $R^7$ und $R^8$ unabhängig voneinander für Wasserstoff, Methyl oder Ethyl stehen,

sowie von deren Säureadditionssalzen und Metallsalzkomplexen, dadurch gekennzeichnet, daß man

a) Oxirane der Formel

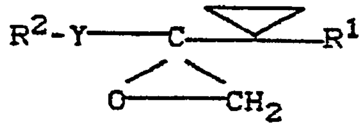

(II)

in welcher
$R^1$, $R^2$ und Y die oben angegebene Bedeutung haben,
mit Azolen der Formel

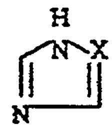

(III)

in welcher
X die oben angegebene Bedeutung hat, in Gegenwart eines Säurebindemittels und in Gegenwart eines Verdünnungsmittels umsetzt, oder
b) Hydroxyalkyl-azolyl-Derivate der Formel

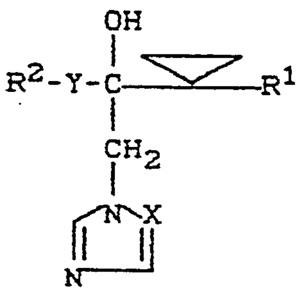

(Ia)

in welcher
$R^1$, $R^2$, X und Y die oben angegebene Bedeutung haben,
mit starken Basen in Gegenwart eines Verdünnungsmittels umsetzt und die dabei entstehenden Alkoholate der Formel

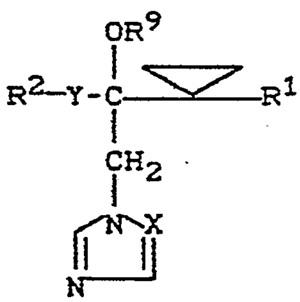

(Ib)

in welcher
$R^1$, $R^2$, X und Y die oben angegebene Bedeutung haben und
$R^9$ für ein Alkalimetallkation oder für ein quarternäres Ammonium- oder Phosphoniumkation steht, mit Halogenverbindungen der Formel $R^{10}$–Hal (IV) in welcher
$R^{10}$ für Alkyl mit 1 bis 6 Kohlenstoffatomen oder Alkylcarbonyl mit 1 bis 6 Kohlenstoffatomen im Alkylteil steht und Hal für Chlor oder Brom steht, in Gegenwart eines Verdünnungsmittels umsetzt,
und gegebenenfalls anschließend an die so erhaltenen Verbindungen der Formel (I) eine Säure oder ein Metallsalz addiert.

3. Fungizide und pflanzenwuchsregulierende Mittel, gekennzeichnet durch einen Gehalt an mindestens einem Hydroxyalkyl-azolyl-Derivat der Formel (I) gemäß Anspruch 1 bzw. an einem Säureadditionssalz oder Metallsalzkomplex eines Hydroxyalkyl-azolyl-Derivates der Formel (I).

4. Verwendung von Hydroxyalkyl-azolyl-Derivaten der Formel (I) gemäß Anspruch 1 bzw. von deren Säureadditionssalzen und Metallsalzkomplexen zur Bekämpfung von Pilzen sowie zur Regulierung des Pflanzenwachstums.

5. Oxirane der Formel

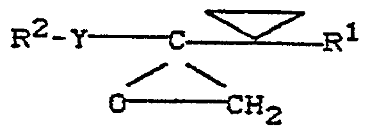

(II)

in welcher

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin

Z für Sauerstoff, Schwefel, SO oder $SO_2$ steht und

$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,

$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

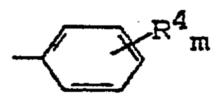

steht, worin

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und

m für die Zahlen 0, 1, 2 oder 3 steht

Y für die Gruppierungen $-CH_2-CH_2-$, $-CH=CH-$, $-C\equiv C-$,

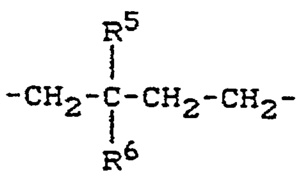

oder

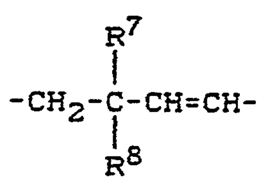

steht,

steht, worin

$R^5$, $R^6$, $R^7$ und $R^8$ unabhängig voneinander für Wasserstoff, Methyl oder Ethyl stehen.

6. Verfahren zur Herstellung von Oxiranen der Formel

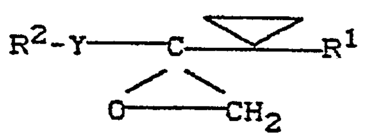

(II)

in welcher

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin

Z für Sauerstoff, Schwefel, SO oder $SO_2$ steht und

$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,
$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

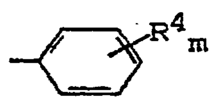

steht,
worin
$R^4$ fur Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und
m für die Zahlen 0, 1, 2 oder 3 steht, und
Y für die Gruppierungen $-CH_2-CH_2-$, $-CH=CH-$, $-C\equiv C-$,

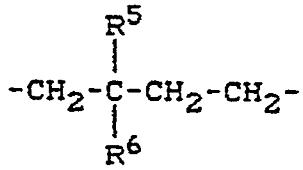 oder 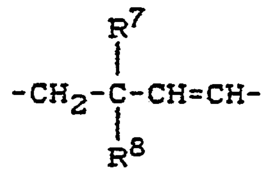 steht,

steht, worin
$R^5$, $R^6$, $R^7$ und $R^8$ unabhängig voneinander für Wasserstoff, Methyl oder Ethyl stehen, dadurch gekennzeichnet, daß man
c) Cyclopropylketone der Formel

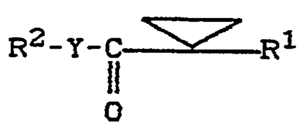

(V)

in welcher
$R^1$, $R^2$ und Y die oben angegebene Bedeutung haben,
entweder
α) mit Dimethyloxosulfonium-methylid der Formel

$$\overset{\delta\oplus\,\delta\ominus}{(CH_3)_2SOCH_2} \qquad (VI)$$

oder
β) mit Dimethylsulfonium-methylid der Formel

$$\overset{\delta\oplus\,\delta\ominus}{(CH_3)_2S\ CH_2} \qquad (VII)$$

in Gegenwart eines Verdünnungsmittels umsetzt, oder indem man
d) Carbinole der Formel

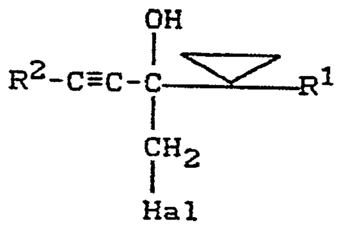

(VIII)

in welcher
$R^1$ und $R^2$ die oben angegebene Bedeutung haben und Hal' für Chlor oder Brom steht,
mit Basen in Gegenwart eines Verdünnungsmittels umsetzt.

7. Cyclopropylketone der Formel

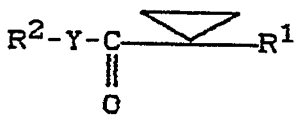

(V)

in welcher
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin
Z für Sauerstoff, Schwefel, SO oder $SO_2$ steht und
$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,
$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

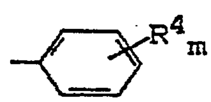

steht, worin
$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und
m für die Zahlen 0, 1, 2 oder 3 steht
Y für die Gruppierungen $-CH_2-CH_2-$, $-CH=CH-$, $-C \equiv C-$,

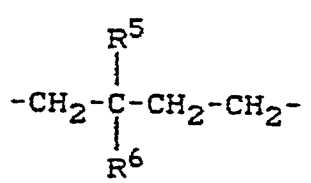 oder 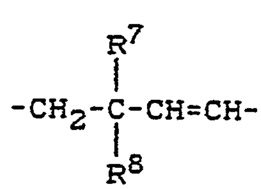 steht,

steht worin

$R^5$, $R^6$, $R^7$ und $R^8$ unabhängig voneinander für Wasserstoff, Methyl oder Ethyl stehen

8. Verfahren zur Herstellung von Cyclopropylketonen der Formel

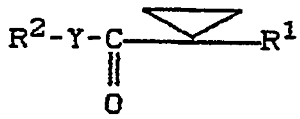 (V)

in welcher

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin

Z für Sauerstoff, Schwefel, SO oder $SO_2$ steht und

$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,

$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

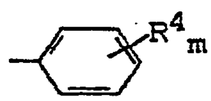

steht,

worin

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und

m für die Zahlen 0, 1, 2 oder 3 steht

Y für die Gruppierungen $-CH_2-CH_2-$, $-CH=CH-$, $-C\equiv C-$,

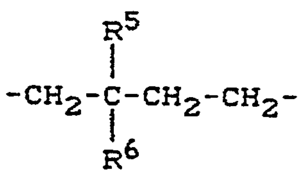 oder 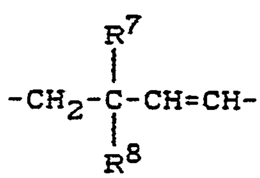

steht, worin

$R^5$, $R^6$, $R^7$ und $R^8$ unabhängig voneinander für Wasserstoff, Methyl oder Ethyl stehen, dadurch gekennzeichnet, daß man

e) Aldehyde der Formel

$R^2$–CHO (IX-a)

oder

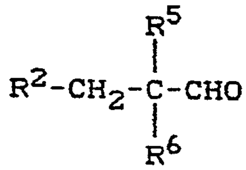

(IX-b)

in welcher $R^2$, $R^5$ und $R^6$ die oben angegebene Bedeutung haben, mit Methyl-cyclopropyl-ketonen der Formel

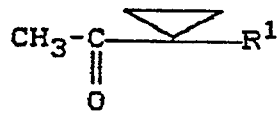

(X)

in welcher $R^1$ die oben angegebene Bedeutung hat, in Gegenwart eines Katalysators und in Gegenwart eines Verdünnungsmittels umsetzt und gegebenenfalls die dabei entstehenden Cyclopropylketone der Formeln

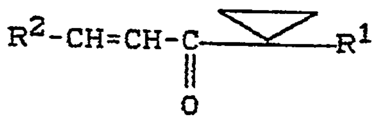

(V-a)

oder

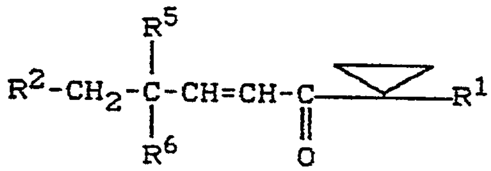

(V-b)

in welchen $R^1$, $R^2$, $R^5$ und $R^6$ die oben angegebene Bedeutung haben, in Gegenwart eines Katalysators und in Gegenwart eines Verdünnungsmittels hydriert, oder

f) Acetylene der Formel

R2–C≡CH (XI)

in welcher $R^2$ die oben angegebene Bedeutung hat, mit Säurehalogeniden der Formel

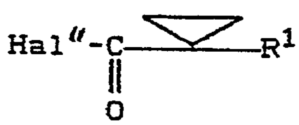

(XII)

in welcher $R^1$ die oben angegebene Bedeutung hat und Hal" für Chlor oder Brom steht, in Gegenwart eines Katalysators und in Gegenwart eines Verdünnungsmittels umsetzt.

9. Carbinole der Formel

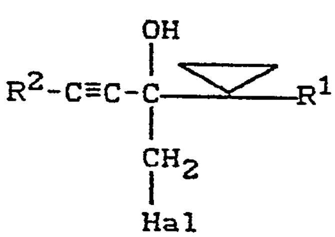

(VIII)

in welcher

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung –Z–$R^3$ steht, worin
Z für Sauerstoff, Schwefel, SO oder $SO_2$ steht und
$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,
$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

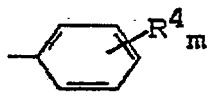

steht, worin
$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und
m für die Zahlen 0, 1, 2 oder 3 steht
und Hal' für Chlor oder Brom steht.

10. Verfahren zur Herstellung von Carbinolen der Formel

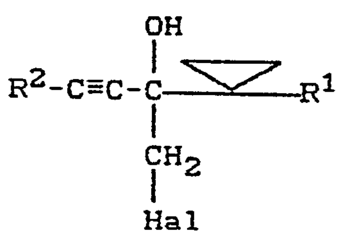

(VIII)

in welcher
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung –Z–$R^3$ steht, worin
Z für Sauerstoff, Schwefel, SO oder $SO_2$ steht und
$R^3$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht oder für Benzyl steht, das gegebenenfalls im Phenylteil durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiert ist,
$R^2$ für gegebenenfalls durch Alkoxy mit 1 bis 4 Kohlenstoffatomen, Alkylthio mit 1 bis 4 Kohlenstoffatomen, Phenyl und/oder Halogen substituiertes Alkyl mit 1 bis 6 Kohlenstoffatomen, für gegebenenfalls durch Alkyl mit 1 bis 4 Kohlenstoffatomen und/oder Halogen substituiertes Cycloalkyl mit 3 bis 8 Kohlenstoffatomen, für gegebenenfalls durch Alkyl mit 1 bis 4 Kohlenstoffatomen und/oder Halogen substituiertes Naphthyl oder für den Rest der Formel

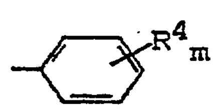

steht, worin

$R^4$ für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl, für gegebenenfalls durch Fluor, Chlor und/oder Methyl substituiertes Phenoxy steht, oder für Nitro, Amino, Alkylamino mit 1 bis 4 Kohlenstoffatomen in der Alkylgruppe, Dialkylamino mit 1 bis 4 Kohlenstoffatomen in jeder Alkylgruppe, Phenylamino oder für Alkylcarbonylamino mit 1 bis 4 Kohlenstoffatomen in der Alkylgruppe steht und
m für die Zahlen 0, 1, 2 oder 3 steht und
Hal′ für Chlor oder Brom steht, dadurch gekennzeichnet, daß man
g) Halogenketone der Formel

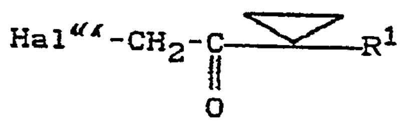

(XIII)

in welcher $R^1$ die oben angegebene Bedeutung hat und Hal‴ für Chlor oder Brom steht, mit Acetylensalzen der Formel

$R^2$–C=C Me (XIV)

in welcher
$R^2$ die oben angegebene Bedeutung hat und Me für ein Lithiumkation oder für ein Äquivalent eines Cer(III)-Kations steht,
gegebenenfalls in Gegenwart eines Säurebindemittels und in Gegenwart eines Verdünnungsmittels umsetzt.

**Claims**

1. Hydroxyalkyl-azolyl derivatives of the formula

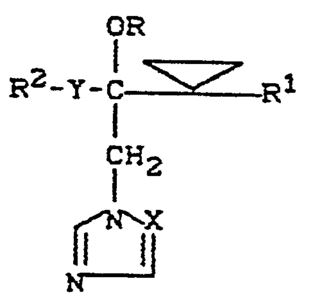

(I)

in which
R represents hydrogen, alkyl having 1 to 6 carbon atoms or alkylcarbonyl having 1 to 6 carbon atoms in the alkyl part,
$R^1$ represents fluorine, chlorine, bromine, phenyl which is optionally substituted by halogen, halogenoalkyl having 1 to 2 carbon atoms and 1 to 5 halogen atoms, alkyl having 1 to 4 carbon atoms and/or alkoxy having 1 to 4 carbon atoms, or the grouping –Z–$R^3$, wherein
Z represents oxygen, sulphur, SO or $SO_2$ and
$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or benzyl which is optionally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,
$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of the formula

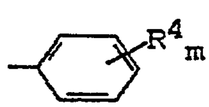

wherein
$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon atoms in each alkyl group, phenylamino or alkylcarbonylamino having 1 to 4 carbon atoms in the alkyl group and
m represents the numbers 0, 1, 2 or 3,
X represents nitrogen or a CH group and
Y represents the groupings $-CH_2-CH_2-$, $-CH=CH-$, $-C\equiv C-$,

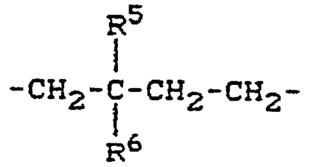
or
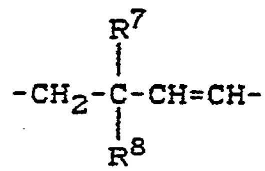

wherein
$R^5$, $R^6$, $R^7$ and $R^8$ independently of one another represent hydrogen, methyl or ethyl, and their acid addition salts and metal salt complexes.
2. Process for the preparation of hydroxyalkyl-azolyl derivatives of the formula

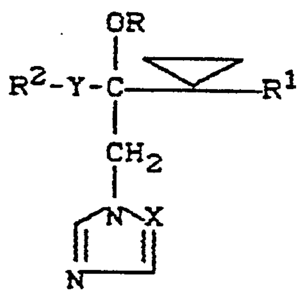
(I)

in which
R represents hydrogen, alkyl having 1 to 6 carbon atoms or alkylcarbonyl having 1 to 6 carbon atoms in the alkyl part,
$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or the grouping $-Z-R^3$, wherein
Z represents oxygen, sulphur, SO or $SO_2$ and
$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 or 4 carbon atoms and/or alkoxy having 1 to 4 carbon atoms, or benzyl which is optionally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,
$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of the formula

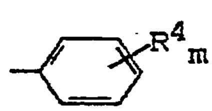

wherein
$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon

atoms in each alkyl group, phenylamino or alkylcarbonylamino having 1 to 4 carbon atoms in the alkyl group and
m represents the numbers 0, 1, 2 or 3,
X represents nitrogen or a CH group and
Y represents the groupings $-CH_2-CH_2-$, $-CH=CH-$, $-C\equiv C-$,

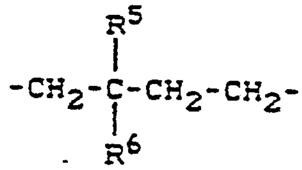 or 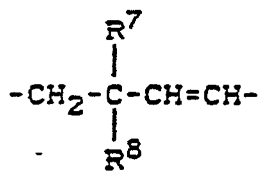

wherein $R^5$, $R^6$, $R^7$ and $R^8$ independently of one another represent hydrogen, methyl or ethyl, and for their acid addition salts and metal salt complexes, characterized in that
a) oxiranes of the formula

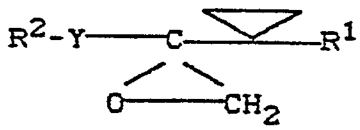 (II)

in which $R^1$, $R^2$ and Y have the abovementioned meaning, are reacted with azoles of the formula

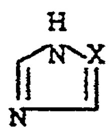 (III)

in which X has the abovementioned meaning, in the presence of an acid-binding agent and in the presence of a diluent, or
b) hydroxyalkyl-azolyl derivatives of the formula

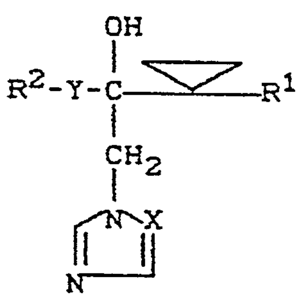 (Ia)

in which $R^1$, $R^2$, X and Y have the abovementioned meaning, are reacted with strong bases in the presence of a diluent and the alcoholates of the formula

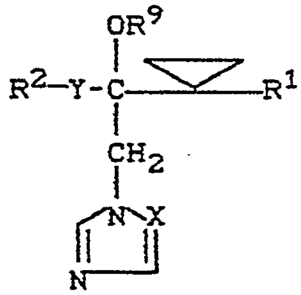 (Ib)

in which
$R^1$, $R^2$, X and Y have the abovementioned meaning and $R^9$ represents an alkali metal cation, or represents a quaternary ammonium or phosphonium cation produced in this reaction are reacted with halogen compounds of the formula

$R^{10}$-Hal (IV)

in which
$R^{10}$ represents alkyl having 1 to 6 carbon atoms or alkylcarbonyl having 1 to 6 carbon atoms in the alkyl part, and
Hal represents chlorine or bromine in the presence of a diluent,
and subsequently if desired, an acid or a metal salt is added to the compounds of the formula (I) thus obtained.

3. Fungicides and plant growth-regulating agents, characterized in that they contain at least one hydroxyalkyl-azolyl derivative of the formula (I) according to Claim 1 or one acid addition salt or metal salt complex of a hydroxyalkyl-azolyl derivative of the formula (I).

4. Use of hydroxyalkyl-azolyl derivatives of the formula (I) according to Claim 1 or their acid addition salts and metal salt complexes for combating fungi and for regulating plant growth.

5. Oxiranes of the formula

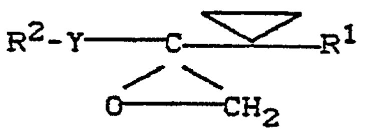 (II)

in which
$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or the grouping $-Z-R^3$, wherein
Z represents oxygen, sulphur, SO or $SO_2$ and
$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or benzyl which is optionally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,
$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of the formula

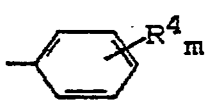

wherein
$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon atoms in each alkyl group, phenylamino or alkylcarbonylamino having 1 to 4 carbon atoms in the alkyl group and
m represents the numbers 0, 1, 2 or 3, and
Y represents the groupings $-CH_2-CH_2-$, $-CH=CH-$, $-C\equiv C-$,

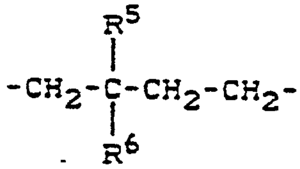 or 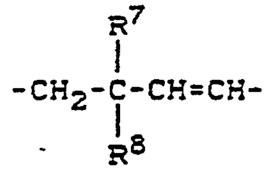

wherein
$R^5$, $R^6$, $R^7$ and $R^8$ independently of one another represent hydrogen, methyl or ethyl.

6. Process for the preparation of oxiranes of the formula

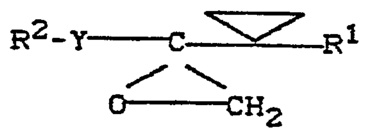

(II)

in which

$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or the grouping $-Z-R^3$, wherein

Z represents oxygen, sulphur, SO or $SO_2$ and

$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or benzyl which is optionally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,

$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of the formula

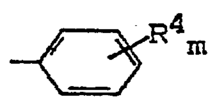

wherein

$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon atoms in each alkyl group, phenylamino or alkylcarbonylamino having 1 to 4 carbon atoms in the alkyl group and

m represents the numbers 0, 1, 2 or 3, and

Y represents the groupings $-CH_2-CH_2-$, $-CH=CH-$, $-C\equiv C-$,

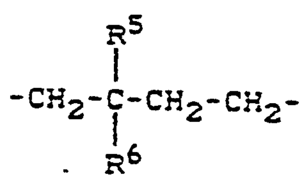

or

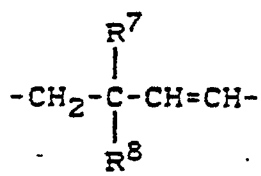

wherein $R^5$, $R^6$, $R^7$ and $R^8$ independently of one another represent hydrogen, methyl or ethyl, characterized in that

c) cyclopropyl ketones of the formula

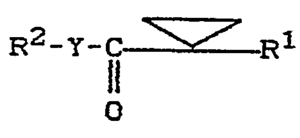

(V)

in which $R^1$, $R^2$ and Y have the abovementioned meaning are reacted with either

α) dimethyloxosulphonium methylide of the formula

$$\overset{\delta\oplus\,\delta\ominus}{(CH_3)_2SOCH_2} \qquad (VI)$$

or
β) dimethylsulphonium methylide of the formula

$$\overset{\delta\oplus\,\delta\ominus}{(CH_3)_2S\ CH_2} \qquad (VII)$$

in the presence of a diluent or where
d) carbinols of the formula

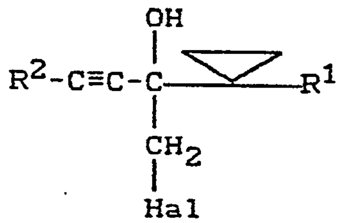

(VIII)

in which $R^1$ and $R^2$ have the abovementioned meaning and Hal' represents chlorine or bromine, are reacted with bases in the presence of a diluent.

7. Cyclopropyl ketones of the formula

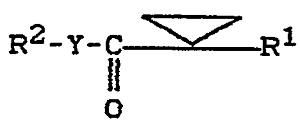

(V)

in which

$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or the grouping $-Z-R^3$, wherein

Z represents oxygen, sulphur, SO or $SO_2$ and

$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or benzyl which is optionally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,

$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of the formula

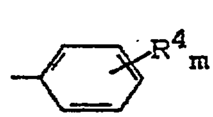

wherein

$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon

atoms in each alkyl group, phenylamino or alkylcarbonylamino having 1 to 4 carbon atoms in the alkyl group and
m represents the numbers 0, 1, 2 or 3, and
Y represents the groupings $-CH_2-CH_2-$, $-CH=CH-$, $-C\equiv C-$,

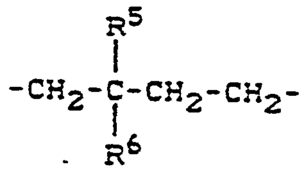

or

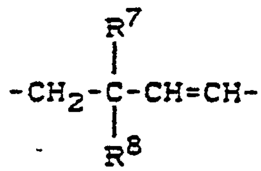

wherein $R^5$, $R^6$, $R^7$ and $R^8$ independently of one another represent hydrogen, methyl or ethyl.

8. Process for the preparation of cyclopropyl ketones of the formula

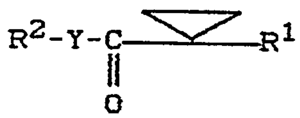

(V)

in which

$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or the grouping -Z-$R^3$, wherein
Z represents oxygen, sulphur, SO or $SO_2$ and
$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or benzyl which is opticnally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,
$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of the formula

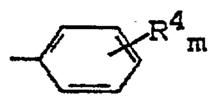

wherein
$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon atoms in each alkyl group, phenylamino or alkylcarbonylamino having 1 to 4 carbon atoms in the alkyl group and
m represents the numbers 0, 1, 2 or 3, and
Y represents the groupings $-CH_2-CH_2-$, $-CH=CH-$, $-C\equiv C-$,

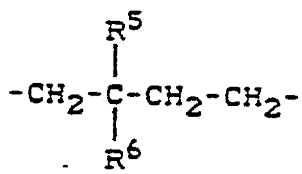

or

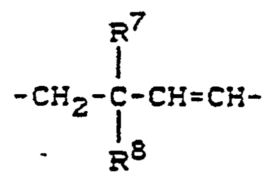

wherein $R^5$, $R^6$, $R^7$ and $R^8$ independently of one another represent hydrogen, methyl or ethyl, characterized in that
e) aldehydes of the formula

$R^2$–CHO (IX-a)

or

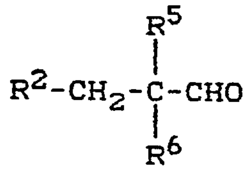

(IX-b)

in which
$R^2$, $R^5$ and $R^6$ have the abovementioned meaning are reacted with methyl cyclopropyl ketones of the formula

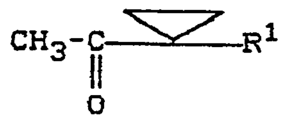

(X)

in which $R^1$ has the abovementioned meaning, in the presence of a catalyst and in the presence of a diluent, and if appropriate the resulting cyclopropyl ketones of the formulae

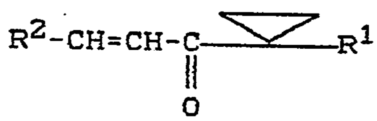

(V-a)

or

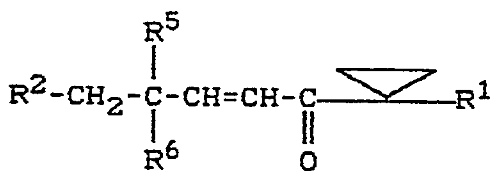

(V-b)

in which $R^1$, $R^2$, $R^5$ and $R^6$ have the abovementioned meaning, are hydrogenated in the presence of a catalyst and in the presence of a diluent, or
f) acetylenes of the formula

$R^2$–C≡CH (XI)

in which
$R^2$ has the abovementioned meaning, are reacted with acid halides of the formula

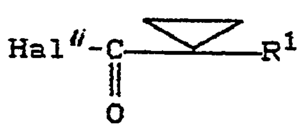

(XII)

in which
$R^1$ has the abovementioned meaning and Hal" represents chlorine or bromine, in the presence of a catalyst and in the presence of a diluent.
9. Carbinols of the formula

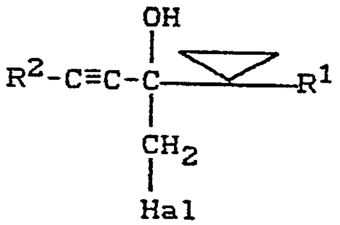

(VIII)

in which

$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or the grouping $-Z-R^3$, wherein

Z represents oxygen, sulphur, SO or $SO_2$ and

$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or benzyl which is optionally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,

$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of the formula

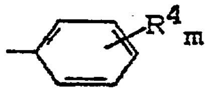

wherein

$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon atoms in each alkyl group, phenylamino or alkylcarbonylamino having 1 to 4 carbon atoms in the alkyl group and

m represents the numbers 0, 1, 2 or 3, and Hal' represents chlorine or bromine.

10. Process for the preparation of carbinols of the formula

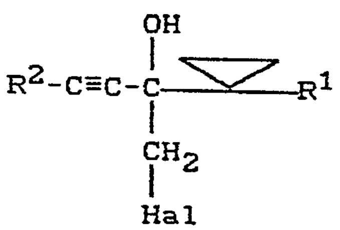

(VIII)

in which

$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or the grouping $-Z-R^3$, wherein

Z represents oxygen, sulphur, SO or $SO_2$ and

$R^3$ represents alkyl having 1 to 6 carbon atoms, which is optionally substituted by alkoxy having 1 to 4 carbon atoms, alkylthio having 1 to 4 carbon atoms, phenyl and/or halogen, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or benzyl which is optionally substituted in the phenyl part by halogen, halogenoalkyl having 1 or 2 carbon atoms and 1 to 5 halogen atoms, alkyl having 1 to 4 carbon atoms and/or alkoxy having 1 to 4 carbon atoms,

$R^2$ represents alkyl having 1 to 6 carbon atoms, which is optionally substituted by alkoxy having 1 to 4 carbon atoms, alkylthio having 1 to 4 carbon atoms, phenyl and/or halogen, cycloalkyl having 3 to 8 carbon atoms, which is optionally substituted by alkyl having 1 to 4 carbon atoms and/or halogen, naphthyl which is optionally substituted by alkyl having 1 to 4 carbon atoms and/or halogen, or the radical of

the formula

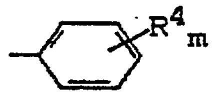

wherein
$R^4$ represents fluorine, chlorine, bromine, methyl, ethyl, isopropyl, tert.-butyl, methoxy, methylthio, trifluoromethyl, trifluoromethoxy, trifluoromethylthio, phenyl which is optionally substituted by fluorine, chlorine and/or methyl, phenoxy which is optionally substituted by fluorine, chlorine and/or methyl, or nitro, amino, alkylamino having 1 to 4 carbon atoms in the alkyl group, dialkylamino having 1 to 4 carbon atoms in each alkyl group, phenylamino or alkylcarbonylamino having 1 to 4 carbon atoms in the alkyl group and
m represents the numbers 0, 1, 2 or 3, and Hal′ represents chlorine or bromine, characterized in that
g) halogenoketones of the formula

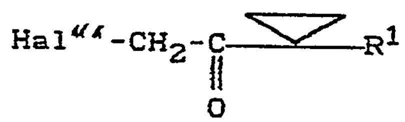 (XIII)

in which
$R^1$ has the abovementioned meaning and Hal‴ represents chlorine or bromine, are reacted with acetylene salts of the formula

$R^2$–C=C Me (XIV)

in which $R^2$ has the abovementioned meaning and
Me represents a lithium cation, or represents an equivalent of a cerium(III) cation, if appropriate in the presence of an acid-binding agent and in the presence of a diluent.

**Revendications**

1. Dérivés hydroxyalkyl-azolyliques de formule

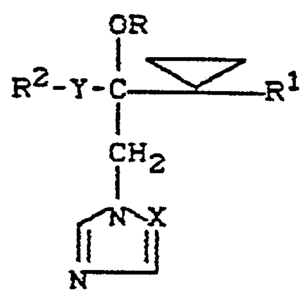 (I)

dans laquelle
R représente l'hydrogène, un groupe alkyle en $C_1$–$C_6$ ou alkylcarbonyle contenant 1 à 6 atomes de carbone dans la partie alkyle,
$R^1$ représente le fluor, le chlore, le brome, un groupe phényle éventuellement substitué par des halogènes, des groupes halogénoalkyle contenant un ou deux atomes de carbone et 1 à 5 atomes d'halogènes, des groupes alkyle en $C_1$–$C_4$ et/ou alcoxy en $C_1$–$C_4$, ou le groupement –Z–$R^3$ dans lequel
Z représente l'oxygène, le soufre, SO ou $SO_2$, et
$R^3$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$,
$R^2$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe cycloalkyle en $C_3$–$C_8$ éventuellement substitué par des groupes alkyle en $C_1$–$C_4$ et/ou des halogènes, un groupe naphtyle éventuellement substitué

par des groupes alkyle en $C_1$–$C_4$ et/ou des halogènes, ou le groupe de formule

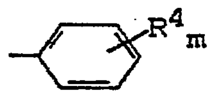

dans laquelle
$R^4$ représente le fluor, le chlore, le brome, un groupe méthyle, éthyle, isopropyle, tert-butyle, méthoxy, méthylthio, trifluorométhyle, trifluorométhoxy, trifluorométhylthio, un groupe phényle éventuellement substitué par le fluor, le chlore et/ou des groupes méthyle, un groupe phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans chaque partie alkyle, phénylamino ou alkylcarbonylamino conentant 1 à 4 atomes de carbone dans la partie alkyle, et
m est égal à 0, 1, 2 ou 3,
X représente l'azote ou un groupe CH, et
Y représente les groupements $-CH_2-CH_2-$, $-CH=CH-$, $-C{\equiv}C-$,

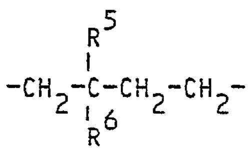 ou 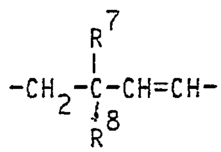

dans lesquels $R^5$, $R^6$, $R^7$ et $R^8$ représentent chacun, indépendamment les uns des autres, l'hydrogène, un groupe méthyle ou éthyle, et leurs sels formés par addition avec des acides et complexes de sels métalliques.

2. Procédé de préparation des dérivés hydroxyalkyl-azolyliques de formule

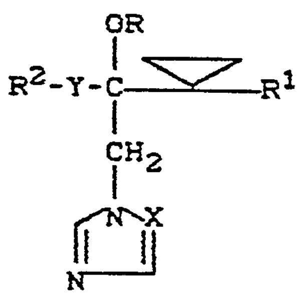

(I)

dans laquelle
R représente l'hydrogène, un groupe alkyle en $C_1$–$C_6$ ou alkylcarbonyle contenant 1 à 6 atomes de carbone dans la partie alkyle,
$R^1$ représente le fluor, le chlore, le brome, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou le groupement $-Z-R^3$ dans lequel
Z représente l'oxygène, le soufre, SO ou $SO_2$, et
$R^3$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$,
$R^2$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe cycloalkyle en $C_3$–$C_8$ éventuellement substitué par des groupes alkyle en $C_1$–$C_4$ et/ou des halogènes, un groupe naphtyle éventuellement substitué par des groupes alkyle en $C_1$–$C_4$ et/ou des halogènes, ou le groupe de formule

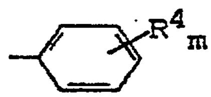

dans laquelle
$R^4$ représente le fluor, le chlore, le brome, un groupe méthyle, éthyle, isopropyle, tert-butyle, méthoxy, méthylthio, trifluorométhyle, trifluorométhoxy, trifluorométhylthio, un groupe phényle éventuellement substitué par le fluor, le chlore et/ou des groupes méthyle, un groupe phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans chaque partie alkyle, phénylamino ou alkylcarbonylamino contenant 1 à 4 atomes de carbone dans la partie alkyle, et
m est égal à 0, 1, 2 ou 3,
X représente l'azote ou un groupe CH, et
Y représente les groupements $-CH_2-CH_2-$, $-CH=CH-$, $-C\equiv C-$,

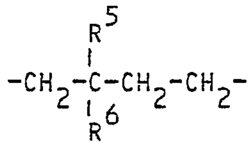 ou 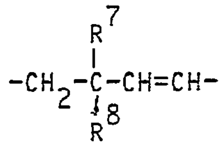

dans lesquels $R^5$, $R^6$, $R^7$ et $R^8$ représentent chacun, indépendamment les uns des autres, l'hydrogène, un groupe méthyle ou éthyle, de leurs sels formés par addition avec des acides et de leurs complexes de sels métalliques, caractérisé en ce que:
(a) on fait réagir des oxirannes de formule

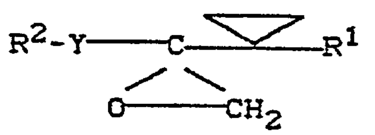 (II)

dans laquelle $R^1$, $R^2$ et Y ont les significations indiquées ci-dessus, avec des azoles de formule

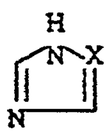 (III)

dans laquelle X a les significations indiquées ci-dessus, en présence d'un accepteur d'acide et en présence d'un diluant, ou bien
(b) on fait réagir des dérivés hydroxyalkyl-azolyliques de formule

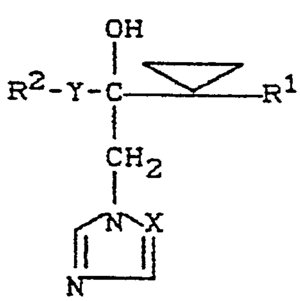 (Ia)

dans laquelle $R^1$, $R^2$, X et Y ont les significations indiquées ci-dessus,
avec des bases fortes en présence d'un diluant, ce qui donne les alcoolates de formule

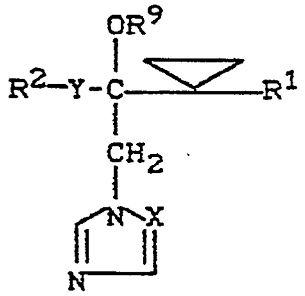

(Ib)

dans laquelle $R^1$, $R^2$, X et Y ont les significations indiquées ci-dessus, et
$R^9$ représente un cation de métal alcalin ou un cation d'ammonium ou de phosphonium quaternaire, qu'on fait réagir avec des dérivés halogénés de formule

$R^{10}$–Hal (IV)

dans laquelle
$R^{10}$ représente un groupe alkyle en $C_1$–$C_6$ ou alkylcarbonyle contenant 1 à 6 atomes de carbone dans la partie alkyle, et Hal représente le chlore ou le brome, en présence d'un diluant, et le cas échéant on fixe ensuite un acide ou un sel métallique sur les composés de formule I ainsi obtenus.

3. Produits fongicides et régulateurs de la croissance des végétaux, caractérisés en ce qu'ils contiennent au moins un dérivé hydroxyalkyl-azolylique de formule I selon revendication 1 ou un sel formé par addition avec un acide ou un complexe de sel métallique d'un dérivé hydroxyalkyl-azolylique de formule I.

4. Utilisation des dérivés hydroxyalkyl-azolylique de formule I selon la revendication 1 et de leurs sels formés par addition avec des acides et complexes de sels métalliques pour la lutte contre les mycètes et pour la régulation de la croissance des végétaux.

5. Oxirannes de formule

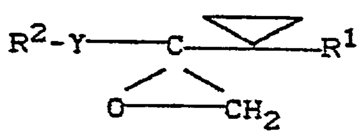

(II)

dans laquelle
$R^1$ représente le fluor, le chlore, le brome, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou le groupement –Z–$R^3$ dans lequel
Z représente l'oxygène, le soufre, SO ou $SO_2$, et
$R^3$ représente un groupe alkyle en $C_1$–$C_6$ éventuellement substitué apr des groupes alcoxy en $C_1$–$C_4$, alkylthio en $C_1$–$C_4$, phényle et/ou des halogènes, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$,
$R^2$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe cycloalkyle en $C_3$–$C_8$ éventuellement substitué par des groupes alkyle en $C_1$–$C_4$ et/ou des halogènes, un groupe naphtyle éventuellement substitué par des groupes alkyle en $C_1$–$C_4$ et/ou des halogènes, ou le groupe de formule

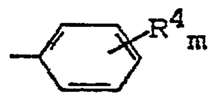

dans laquelle
$R^4$ représente le fluor, le chlore, le brome, des groupes méthyle, éthyle, isopropyle, tert-butyle, méthoxy, méthylthio, trifluorométhyle, trifluorométhoxy, trifluorométhylthio, un groupe phényle éventuellement substitué par le fluor, le chlore et/ou des groupes méthyle, un groupe phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans

chaque partie alkyle, phénylamino ou alkylcarbonylamino contenant 1 à 4 atomes de carbone dans la partie alkyle, et
m est égal à 0, 1, 2 ou 3, et
Y représente les groupements $-CH_2-CH_2-$, $-CH=CH-$, $-C\equiv C$,

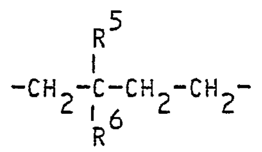 ou 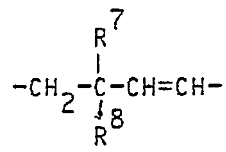

dans lesquels $R^5$, $R^6$, $R^7$ et $R^8$ représentent chacun, indépendamment les un des autres, l'hydrogène, un groupe méthyle ou éthyle.

6. Procédé de préparation des oxirannes de formule

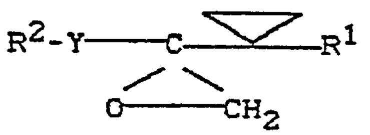 (II)

dans laquelle
$R^1$ représente le fluor, le chlore, le brome, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou le groupement $-Z-R^3$ dans lequel
Z représente l'oxygène, le soufre, SO ou $SO_2$, et
$R^3$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$,
$R^2$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe cycloalkyle en $C_3$–$C_8$ éventuellement substitué par des groupes alkyle en $C_1$–$C_4$ et/ou des halogènes, un groupe naphtyle éventuellement substitué par des groupes alkyle en $C_1$–$C_4$ et/ou des halogènes, ou le groupe de formule

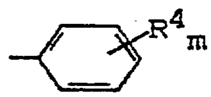

dans laquelle
$R^4$ représente le fluor, le chlore, le brome, un groupe méthyle, éthyle, isopropyle, tert-butyle, méthoxy, méthylthio, trifluorométhyle, trifluorométhoxy, trifluorométhylthio, phényle éventuellement substitué par le fluor, le chlore et/ou des groupes méthyle, phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans chaque partie alkyle, phénylamino ou alkylcarbonylamino contenant 1 à 4 atomes de carbone dans la partie alkyle, et
m est égal à 0, 1, 2 ou 3, et
Y représente les groupements $-CH_2-CH_2-$, $-CH=CH-$, $-C\equiv C-$,

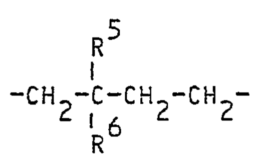 ou 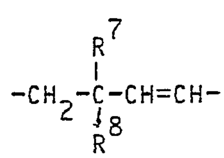

dans lesquels $R^5$, $R^6$, $R^7$ et $R^8$ représentent chacun, indépendamment les uns des autres, l'hydrogène, un groupe méthyle ou éthyle, caractérisé en ce que:
(c) on fait réagir des cyclopropylcétones de formule

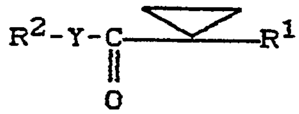

(V)

dans laquelle $R^1$, $R^2$ et Y ont les significations indiquées ci-dessus,

α) soit avec le méthylure de diméthyloxosulfonium de formule

$$\delta^{\oplus}\delta^{\ominus}$$

$$(CH_3)_2SOCH_2 \quad (VI)$$

β) soit avec le méthylure de diméthylsulfonium de formule

$$\delta^{\oplus}\delta^{\ominus}$$

$$(CH_3)_2S\ CH_2 \quad (VII)$$

en présence d'un diluant,

ou bien en ce que:

(d) on fait les carbinols de formule

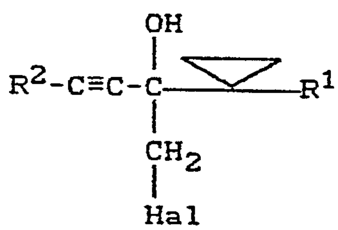

(VIII)

dans laquelle

$R^1$ et $R^2$ ont les significations indiquées ci-dessus, et

Hal' représente le chlore ou le brome,

avec des bases en présence d'un diluant.

7. Cyclopropylcétones de formule

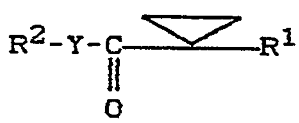

(V)

dans laquelle

$R^1$ représente le fluor, le chlore, le brome, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou le groupement $-Z-R^3$ dans lequel

Z représente l'oxygène, le soufre, SO ou $SO_2$, et

$R^3$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$,

$R^2$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe cycloalkyle en $C_3$–$C_8$ éventuellement substitué par des groupes alkyle en $C_1$–$C_4$ et/ou des halogènes, un groupe naphtyle éventuellement substitué par des groupes alkyle en $C_1$–$C_4$ et/ou des halogènes, ou le groupe de formule

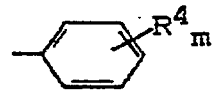

dans laquelle
$R^4$ représente le fluor, le chlore, le brome, un groupe méthyle, éthyle, isopropyle, tert-butyle, méthoxy, méthylthio, trifluorométhyle, trifluorométhoxy, trifluorométhylthio, un phényle éventuellement substitué par le fluor, le chlore et/ou des groupes méthyle, groupe phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans chaque partie alkyle, phénylamino ou alkylcarbonylamino contenant 1 à 4 atomes de carbone dans la partie alkyle, et
m est égal à 0, 1, 2 ou 3, et
Y représente les groupements $-CH_2-CH_2-$, $-CH=CH-$, $-C\equiv C-$,

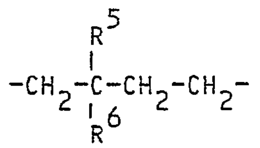 ou 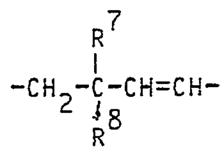

dans lesquels $R^5$, $R^6$, $R^7$ et $R^8$ représentent chacun, indépendamment les uns des autres, l'hydrogène, un groupe méthyle ou éthyle.

8. Procédé de préparation des cyclopropylcétones de formule

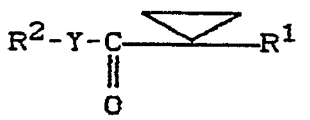 (V)

dans laquelle
$R^1$ représente le fluor, le chlore, le brome, un groupe phényle éventuellement substitué par des halogènes, des groupes halogénoalkyle contenant 1 ou 2 atomes de carbone et 1 à 5 atomes d'halogènes, des groupes alkyle en $C_1-C_4$ et/ou alcoxy en $C_1-C_4$, ou le groupement $-Z-R^3$ dans lequel
Z représente l'oxygène, le soufre, SO ou $SO_2$, et
$R^3$ représente un groupe alkyle en $C_1-C_6$ éventuellement substitué par des groupes alcoxy en $C_1-C_4$, alkylthio en $C_1-C_4$, phényle et/ou des halogènes, un groupe phényle éventuellement substitué par des halogènes, des groupes halogénoalkyle contenant 1 ou 2 atomes de carbone et 1 à 5 atomes d'halogènes, des groupes alkyle en $C_1-C_4$ et/ou alcoxy en $C_1-C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1-C_4$ et/ou alcoxy en $C_1-C_4$,
$R^2$ représente un groupe alkyle en $C_1-C_6$ éventuellement substitué par des groupes alcoxy en $C_1-C_4$, alkylthio en $C_1-C_4$, phényle et/ou des halogènes, un groupe cycloalkyle en $C_3-C_8$ éventuellement substitué par des groupes alkyle en $C_1-C_4$ et/ou des halogènes, un groupe naphtyle éventuellement substitué par des groupes alkyle en $C_1-C_4$ et/ou des halogènes, ou le groupe de formule

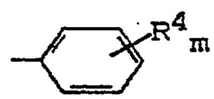

dans laquelle
$R^4$ représente le fluor, le chlore, le brome, un groupe méthyle, éthyle, isopropyle, tert-butyle, méthoxy, méthylthio, trifluorométhyle, trifluotométhoxy, trifluorométhylthio, phényle éventuellement substitué par le fluor, le chlore et/ou des groupes méthyle, phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans chaque partie alkyle, phénylamino ou alkylcarbonylamino contenant 1 à 4 atomes de carbone dans la partie alkyle, et
m est égal à 0, 1, 2 ou 3, et
Y représente les groupements $-CH_2-CH_2-$, $-CH=CH-$, $-C\equiv C-$,

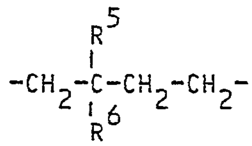 ou 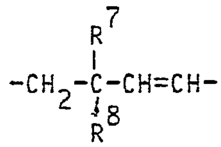

dans lesquels $R^5$, $R^6$, $R^7$ et $R^8$ représentent chacun, indépendamment les uns des autres, l'hydrogène, un groupe méthyle ou éthyle.
caractérisé en ce que:
(e) on fait réagir des aldéhydes de formules

$R^2$–CHO (IX-a)

ou

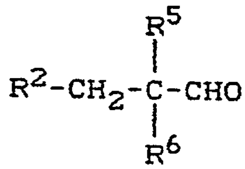 (IX-b)

dans lesquelles
$R^2$, $R^5$ et $R^6$ ont les significations indiquées ci-dessus, avec des méthyl-cyclopropyl-cétones de formule

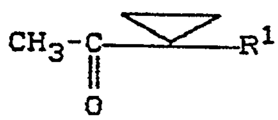 (X)

dans laquelle $R^1$ a les significations indiquées ci-dessus, en présence d'un catalyseur et en présence d'un diluant, ce qui donne les cyclopropylcétones de formules

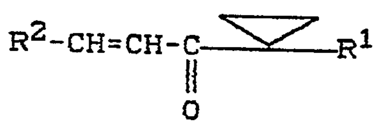 (V-a)

ou

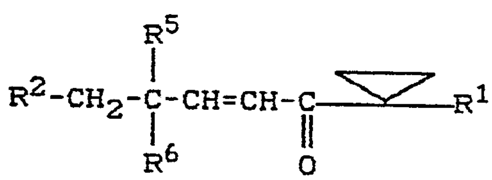 (V-b)

dans lesquelles $R^1$, $R^2$, $R^5$ et $R^6$ ont les significations indiquées ci-dessus, qu'on hydrogène le cas échéant en présence d'un catalyseur et en présence d'un diluant,
ou bien
(f) on fait réagir des composés acétyléniques de formule

$R^2$-C≡CH (XI)

dans laquelle $R^2$ a les significations indiquées ci-dessus, avec des halogénures d'acides de formule

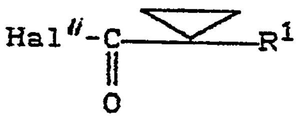

(XII)

dans laquelle
$R^1$ a les significations indiquées ci-dessus, et
Hal" représente le chlore ou le brome,
en présence d'un catalyseur et en présence d'un diluant.

9. Carbinols de formule

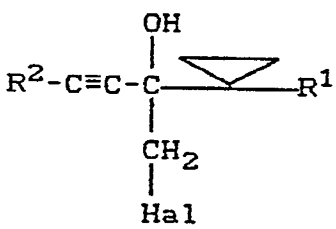

(VIII)

dans laquelle
$R^1$ représente le fluor, le chlore, le brome, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou le groupement –Z–$R^3$ dans lequel
Z représente l'oxygène, le soufre, SO ou $SO_2$, et
$R^3$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$,
$R^2$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe cycloalkyle en $C_3$–$C_8$ éventuellement substitué par des groupes alkyle en $C_1$–$C_4$ et/ou des halogènes, un groupe naphtyle éventuellement substitué par des groupes alkyle en $C_1$–$C_4$ et/ou des halogènes, ou le groupe de formule

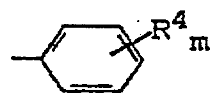

dans laquelle
$R^4$ représente le fluor, le chlore, le brome, un groupe méthyle, éthyle, isopropyle, tert-butyle, méthoxy, méthylthio, trifluorométhyle, trifluorométhoxy, trifluorométhylthio, phényle éventuellement substitué par le fluor, le chlore et/ou des groupes méthyle, phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans chaque partie alkyle, phénylamino ou alkylcarbonylamino contenant 1 à 4 atomes de carbone dans la partie alkyle, et
m est égal à 0, 1, 2 ou 3, et
Hal' représente le chlore ou le brome.

10. Procédé de préparation des carbinols de formule

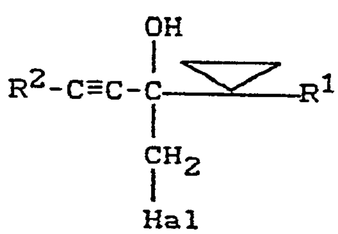

(VIII)

dans laquelle

$R^1$ représente le fluor, le chlore, le brome, un groupe phényle éventuellement substitué par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$, ou le groupement –Z–$R^3$ dans lequel
Z représente l'oxygène, le soufre, SO ou $SO_2$, et
$R^3$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe phényle éventuellement substitué par des halogènes, des groupes halogénoalkyle contenant 1 ou 2 atomes de carbone et 1 à 5 atomes d'halogènes, alkyle en $C_1$–$C_4$ et/ou alcoxy en $C_1$–$C_4$, ou un groupe benzyle éventuellement substitué dans la partie phényle par des halogènes, des groupes halogénoalkyle contenant 1 ou 2 atomes de carbone et 1 à 5 atomes d'halogènes, des groupes alkyle en $C_1$–$C_4$ et/ou alcoxy en $C_1$–$C_4$,

$R^2$ représente un groupe alkyle en $C_1$–$C_6$ éventuellement substitué par des groupes alcoxy en $C_1$–$C_4$, alkylthio en $C_1$–$C_4$, phényle et/ou des halogènes, un groupe cycloalkyle en $C_3$–$C_8$ éventuellement substitué par des groupes alkyle en $C_1$–$C_4$ et/ou des halogènes, un groupe naphtyle éventuellement substitué par des groupes alkyle en $C_1$–$C_4$ et/ou des halogènes, ou le groupe de formule

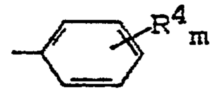

dans laquelle
$R^4$ représente le fluor, le chlore, le brome, un groupe méthyle, éthyle, isopropyle, tert-butyle, méthoxy, méthylthio, trifluorométhyle, trifluorométhoxy, trifluorométhylthio, phényle éventuellement substitué par le fluor, le chlore et/ou des groupes méthyle, phénoxy éventuellement substitué par le fluor, le chlore et/ou des groupes méthyle, ou un groupe nitro, amino, alkylamino contenant 1 à 4 atomes de carbone dans la partie alkyle, dialkylamino contenant 1 à 4 atomes de carbone dans chaque partie alkyle, phénylamino ou alkylcarbonylamino contenant 1 à 4 atomes de carbone dans la partie alkyle, et
m est égal à 0, 1, 2 ou 3, et
Hal' représente le chlore ou le brome,

caractérisé en ce que

(g) on fait réagir des halogénocétones de formule

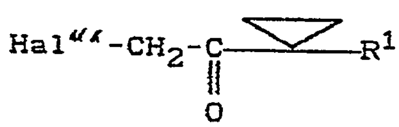

(XIII)

dans laquelle
$R^1$ a les significations indiquées ci-dessus, et
Hal''' représente le chlore ou le brome,

avec des sels de dérivés acétyléniques de formule

$R^2$–C=C Me (XIV)

dans laquelle
$R^2$ a les significations indiquées ci-dessus, et
Me représente un cation de lithium ou un équivalent d'un cation de cérium-III, éventuellement en présence d'un accepteur d'acide et en présence d'un diluant.

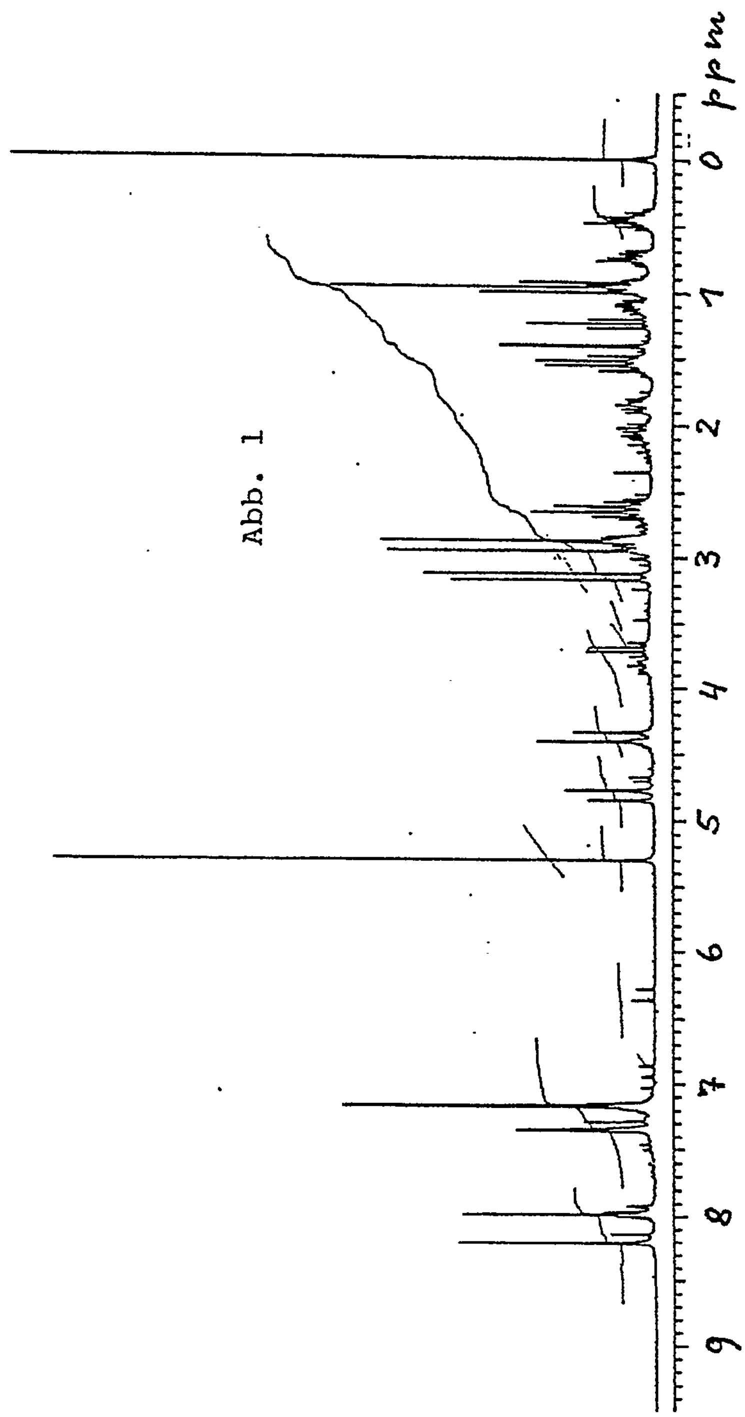


Abb. 1

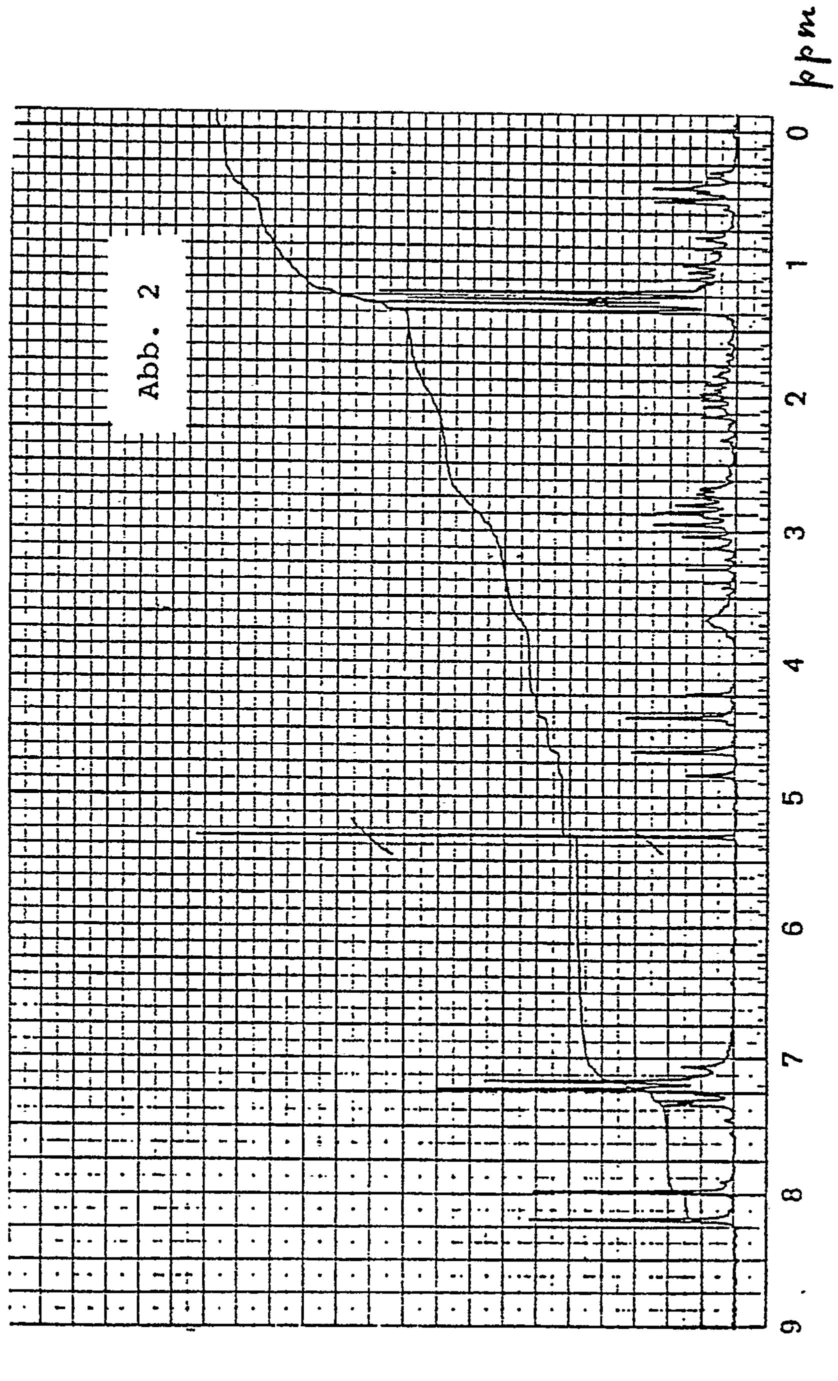
Abb. 2
ppm
0
1
2
3
4
5
6
7
8
9

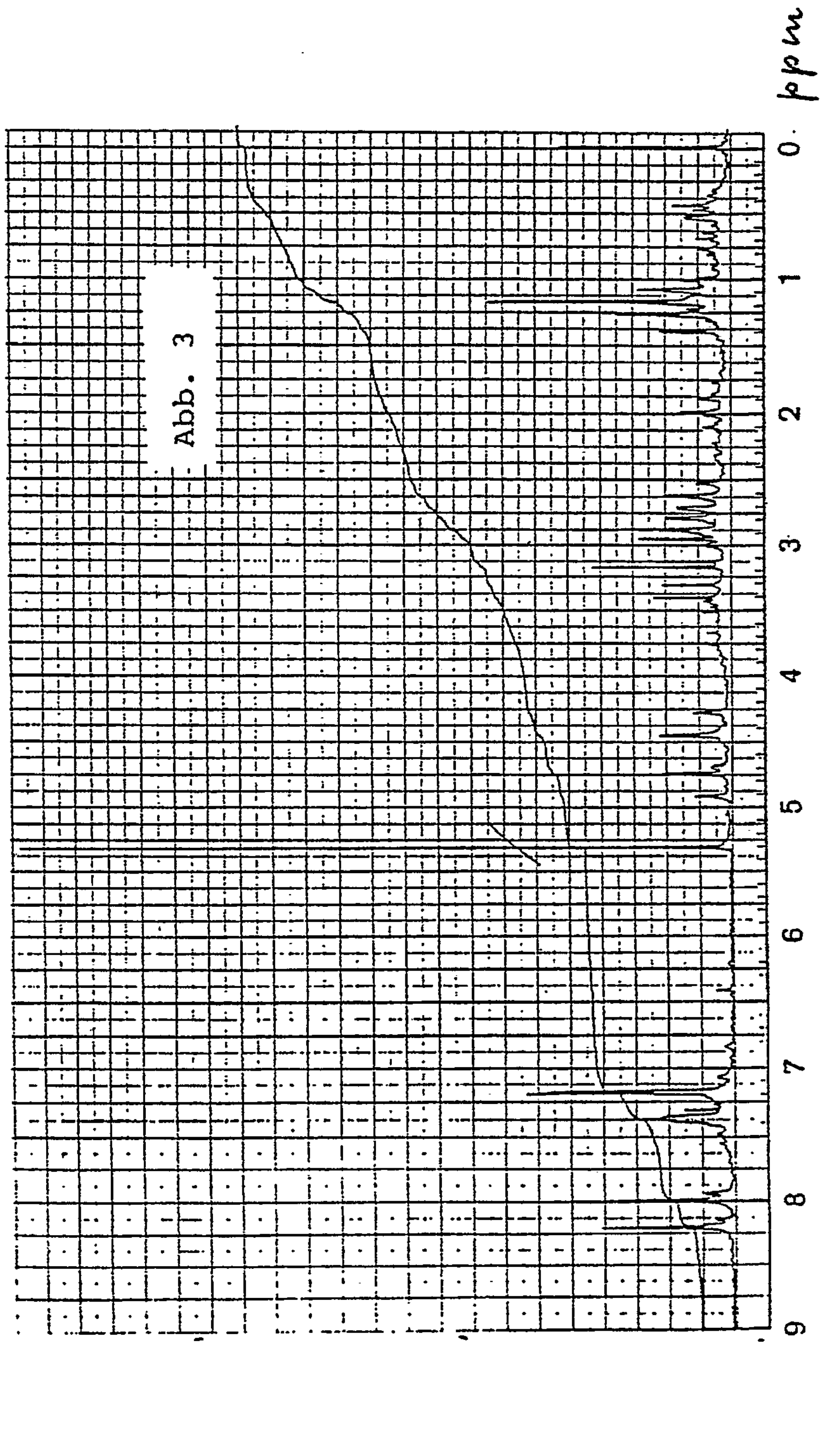

Abb. 3

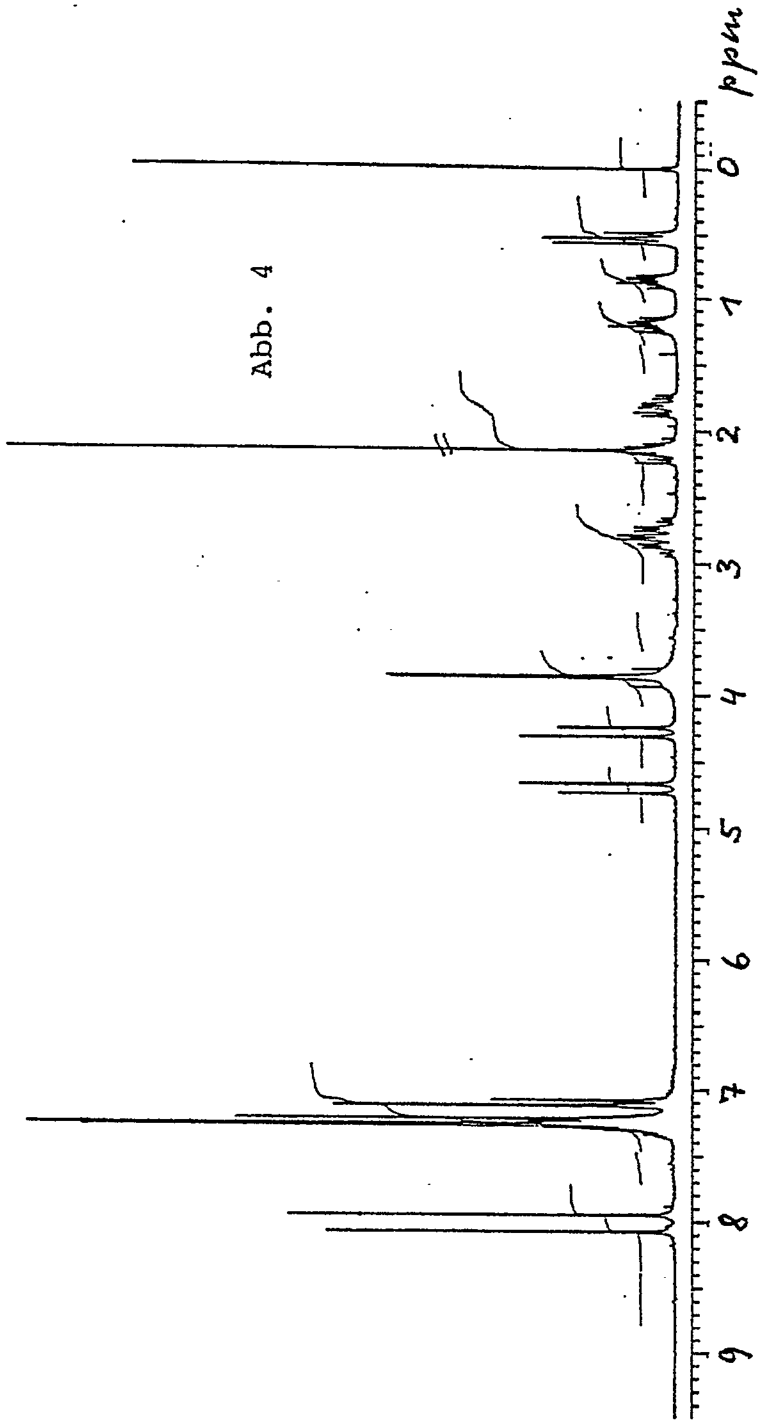


Abb. 4

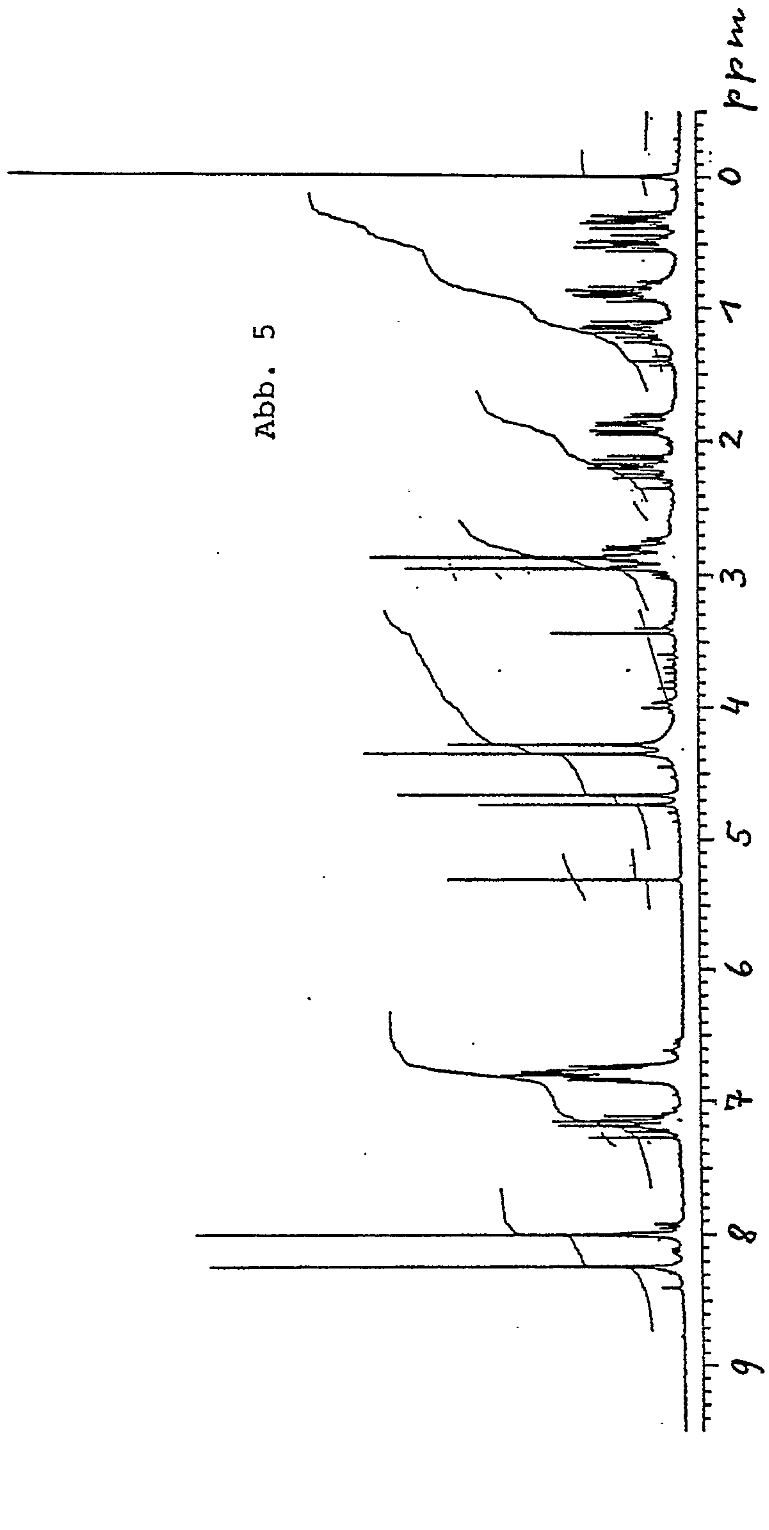


Abb. 5

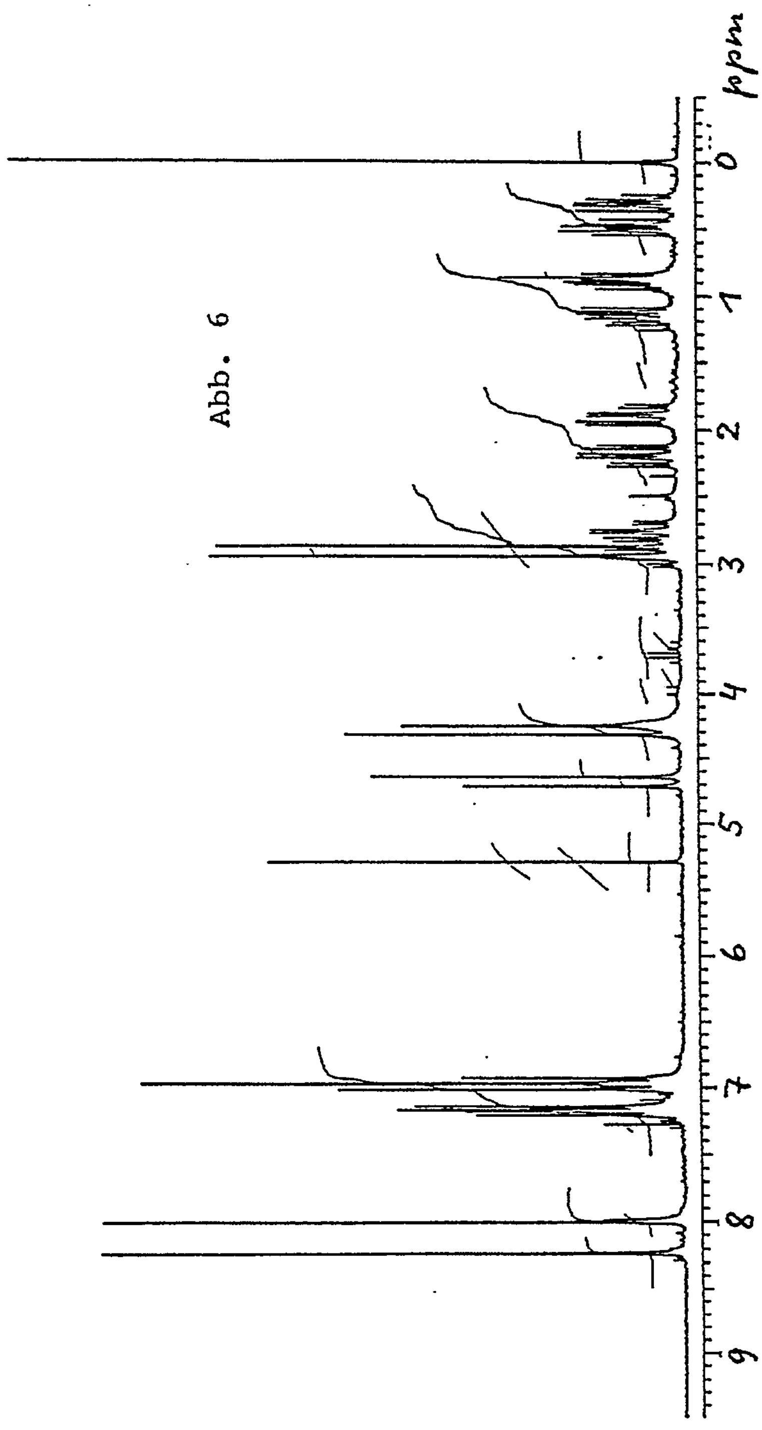


Abb. 6

Abb. 7

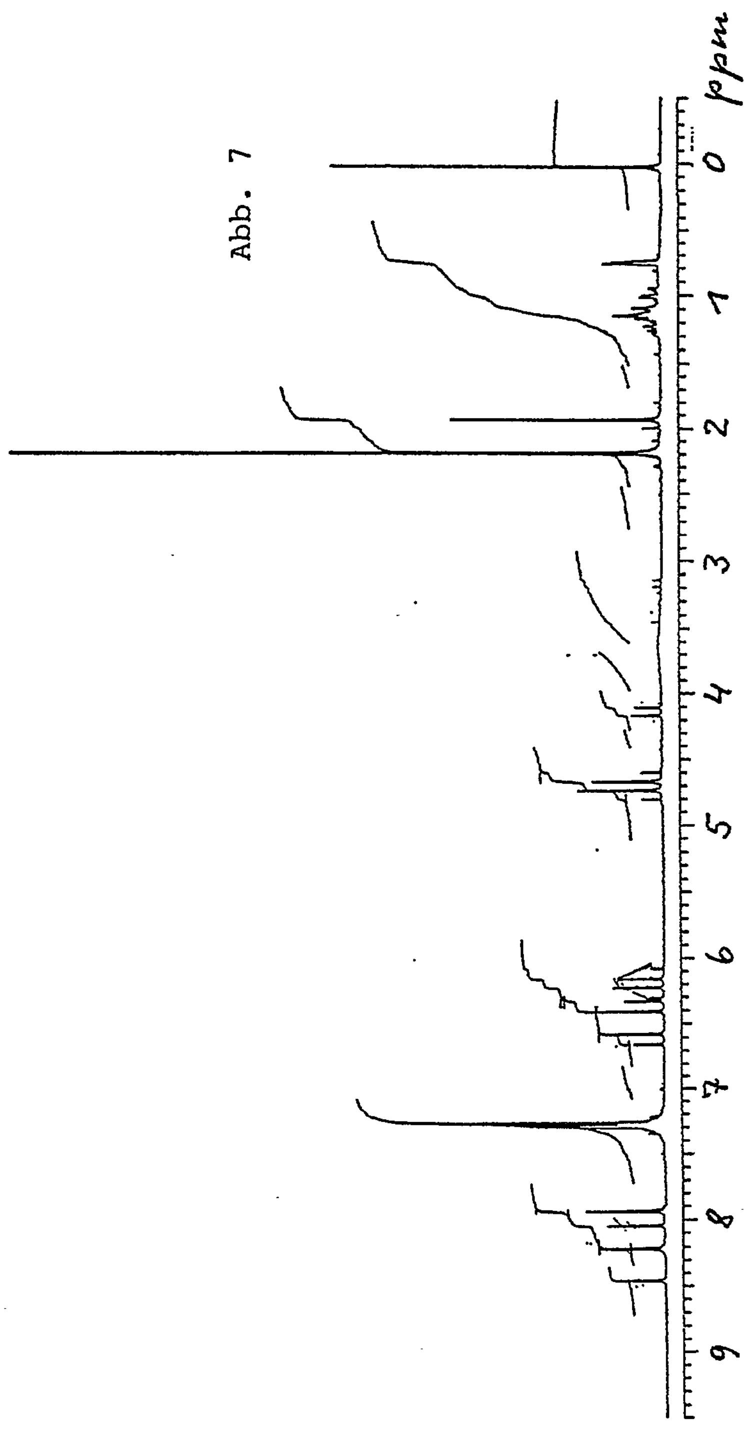

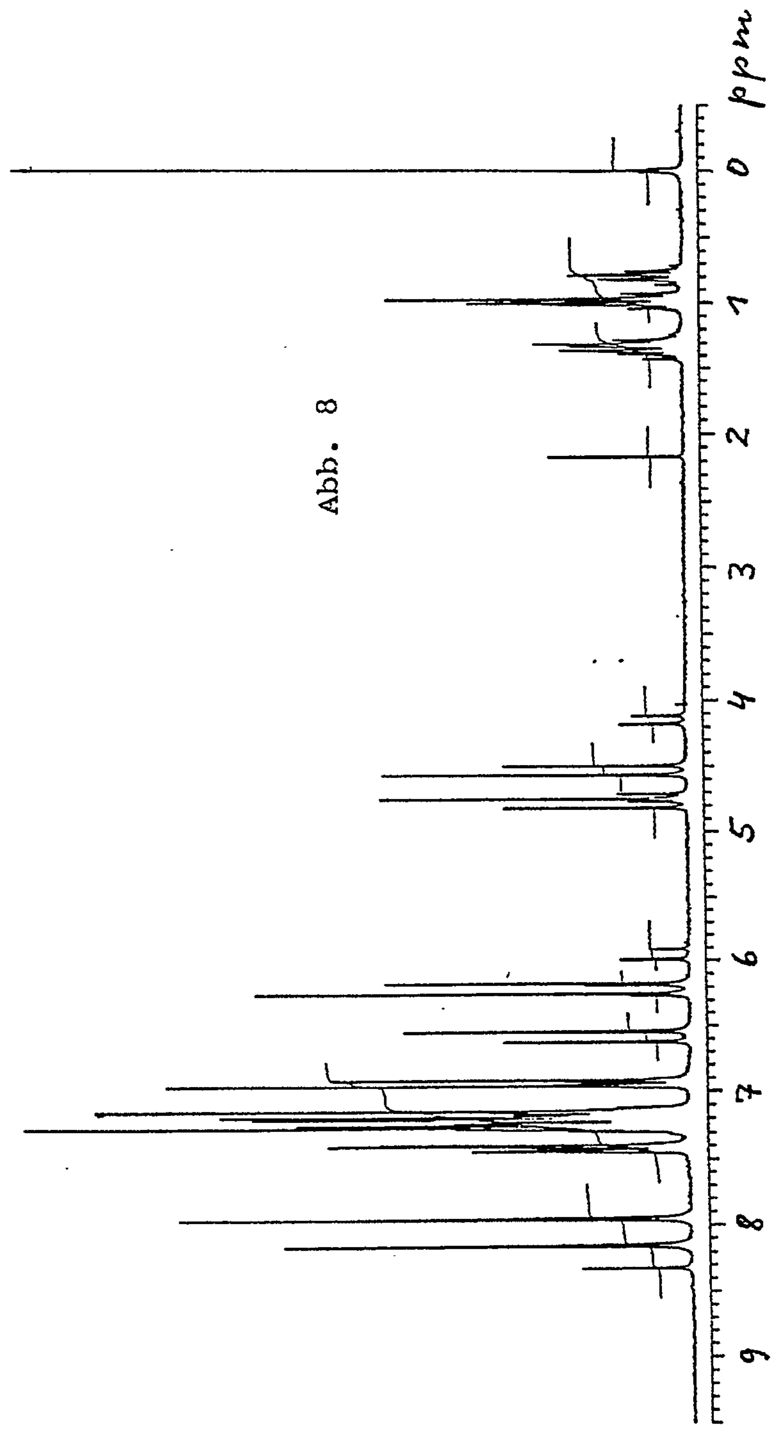


Abb. 8

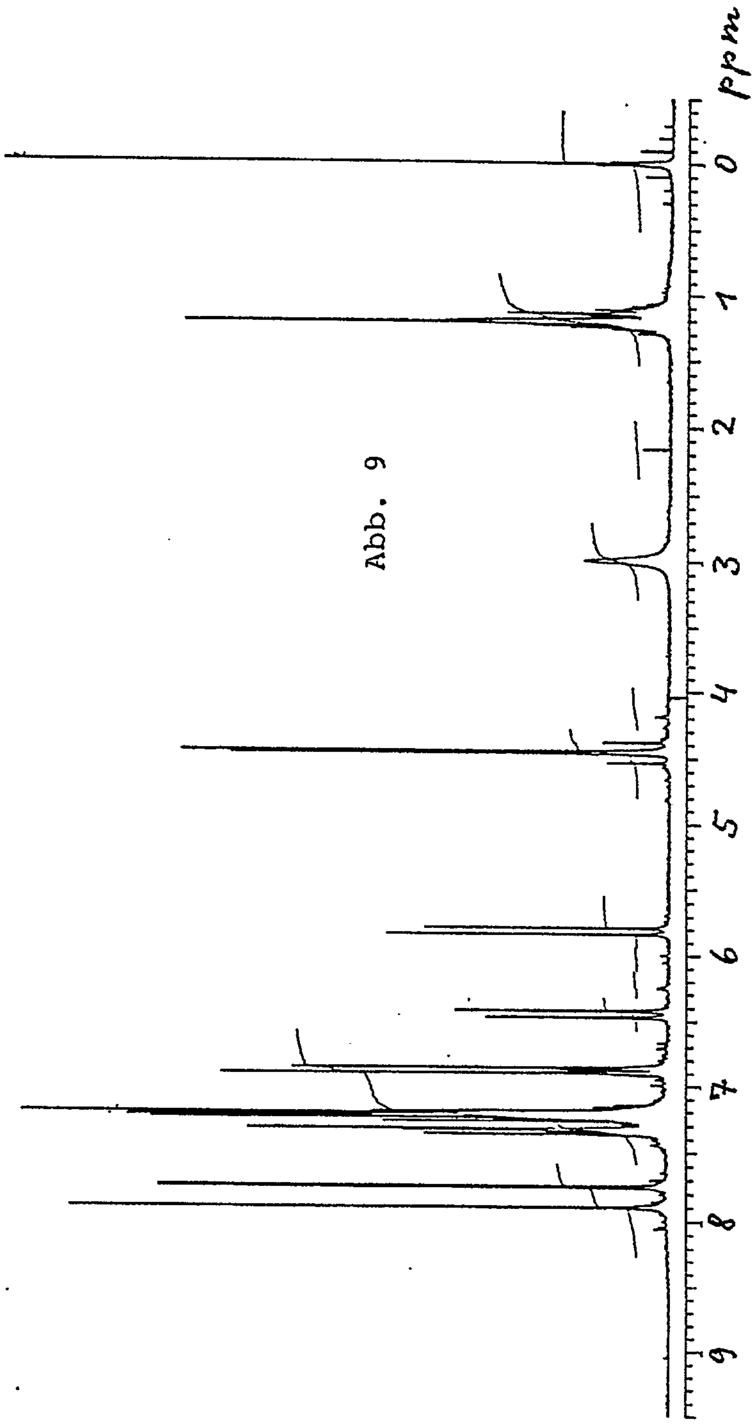


Abb. 9

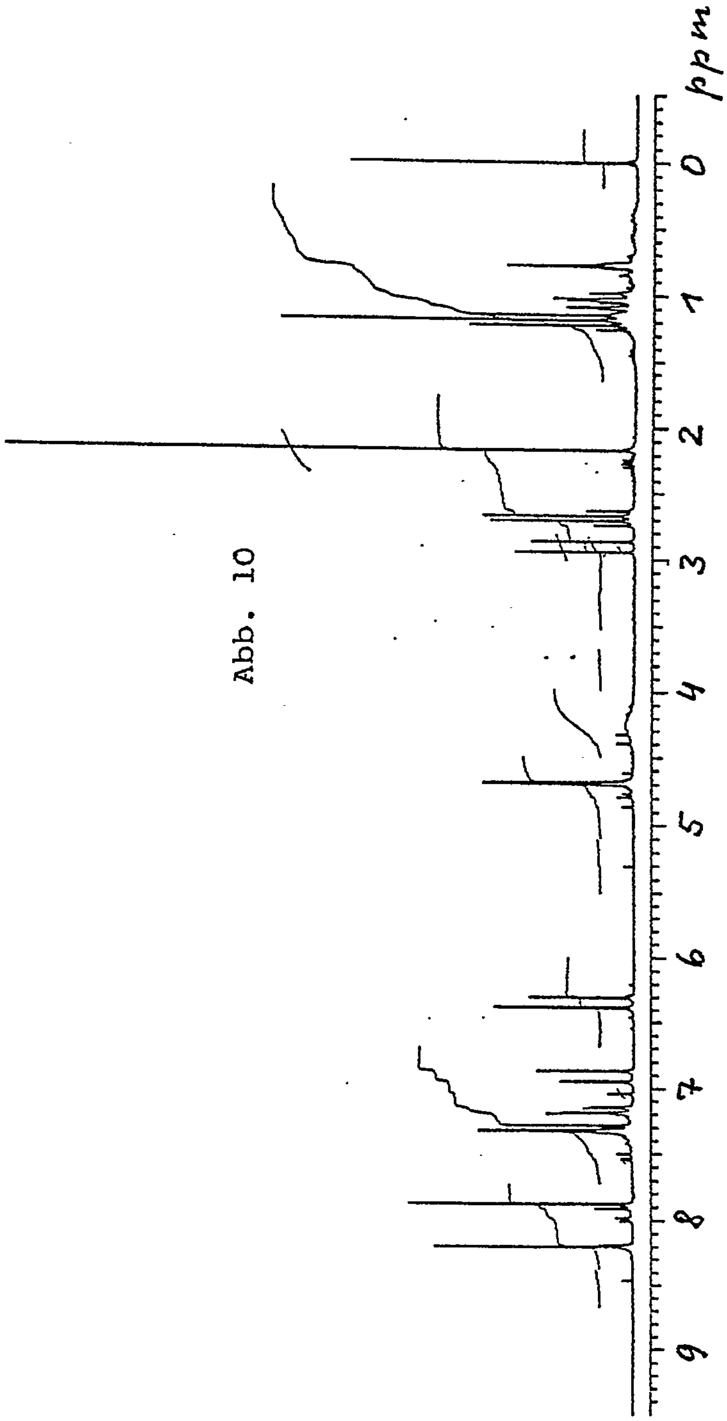


Abb. 10

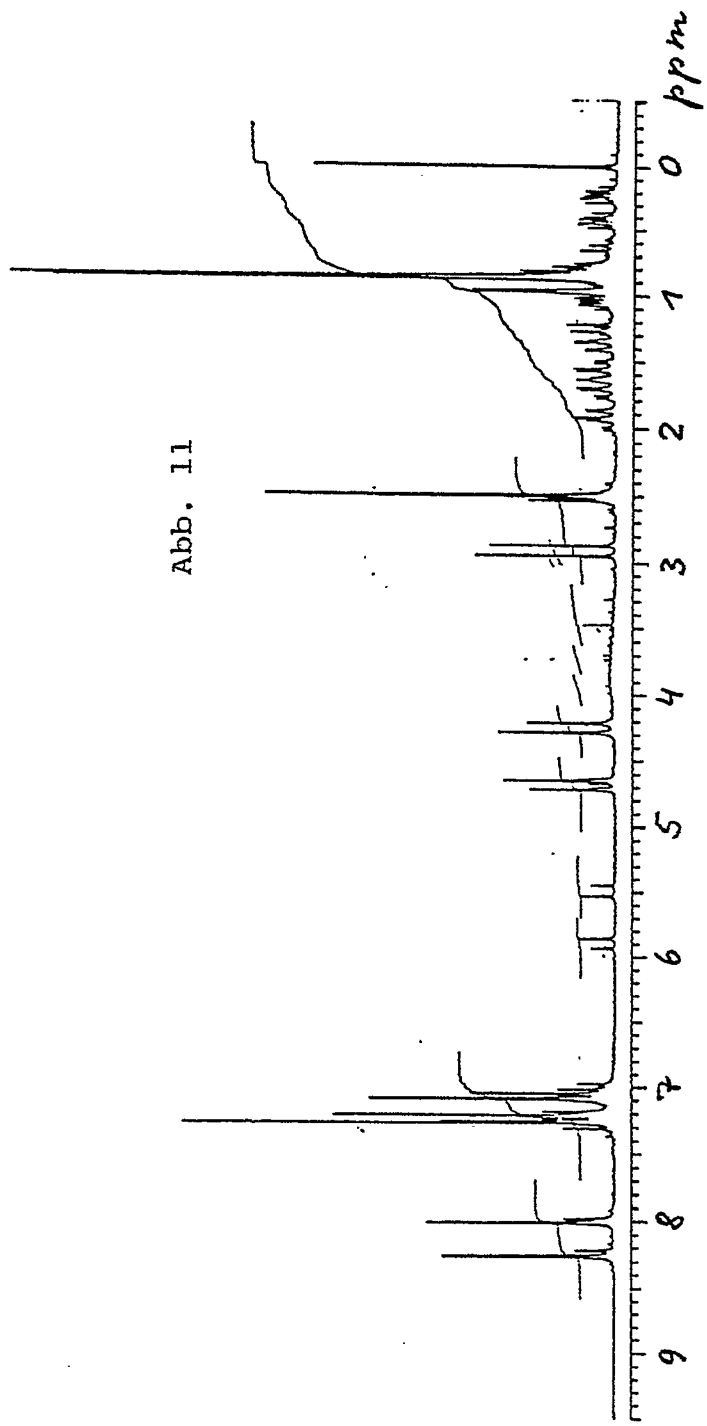


Abb. 11

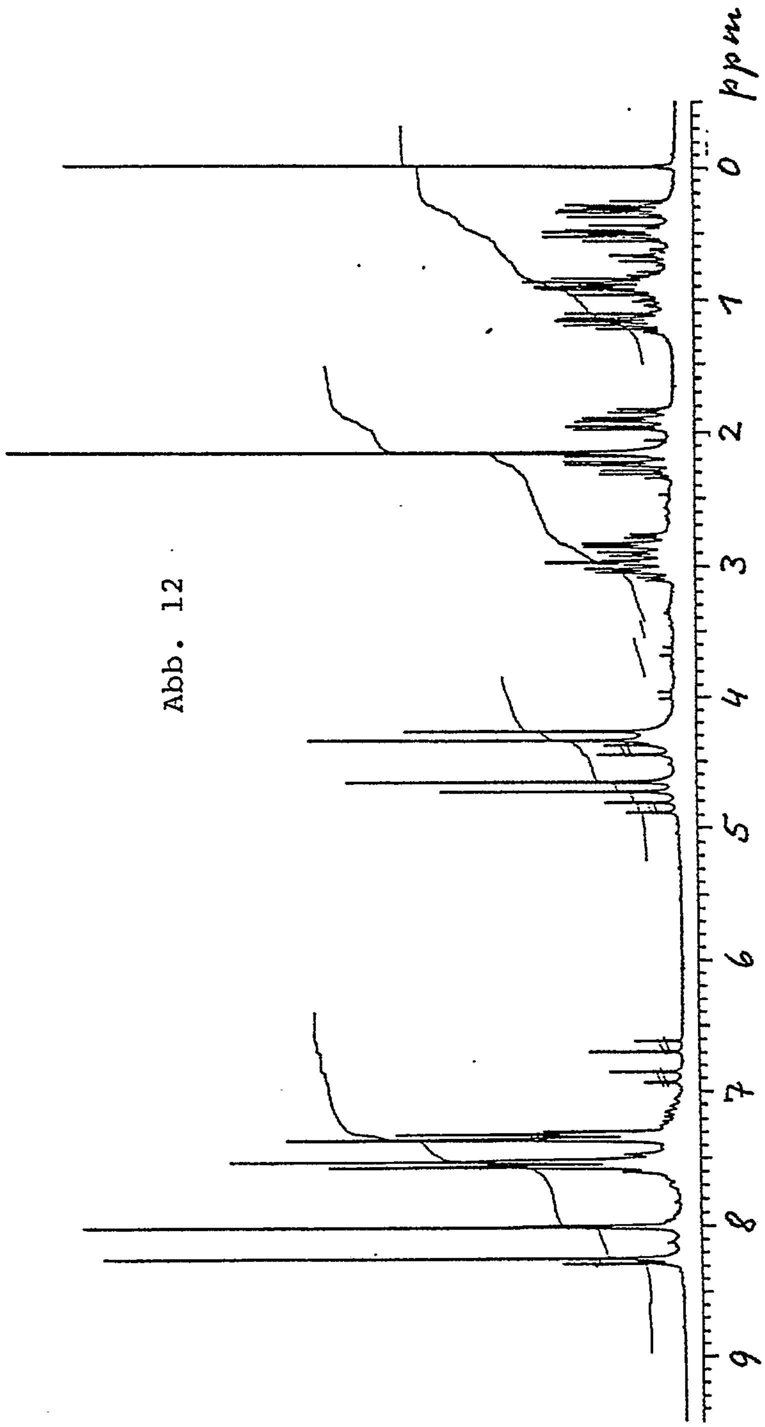


Abb. 12

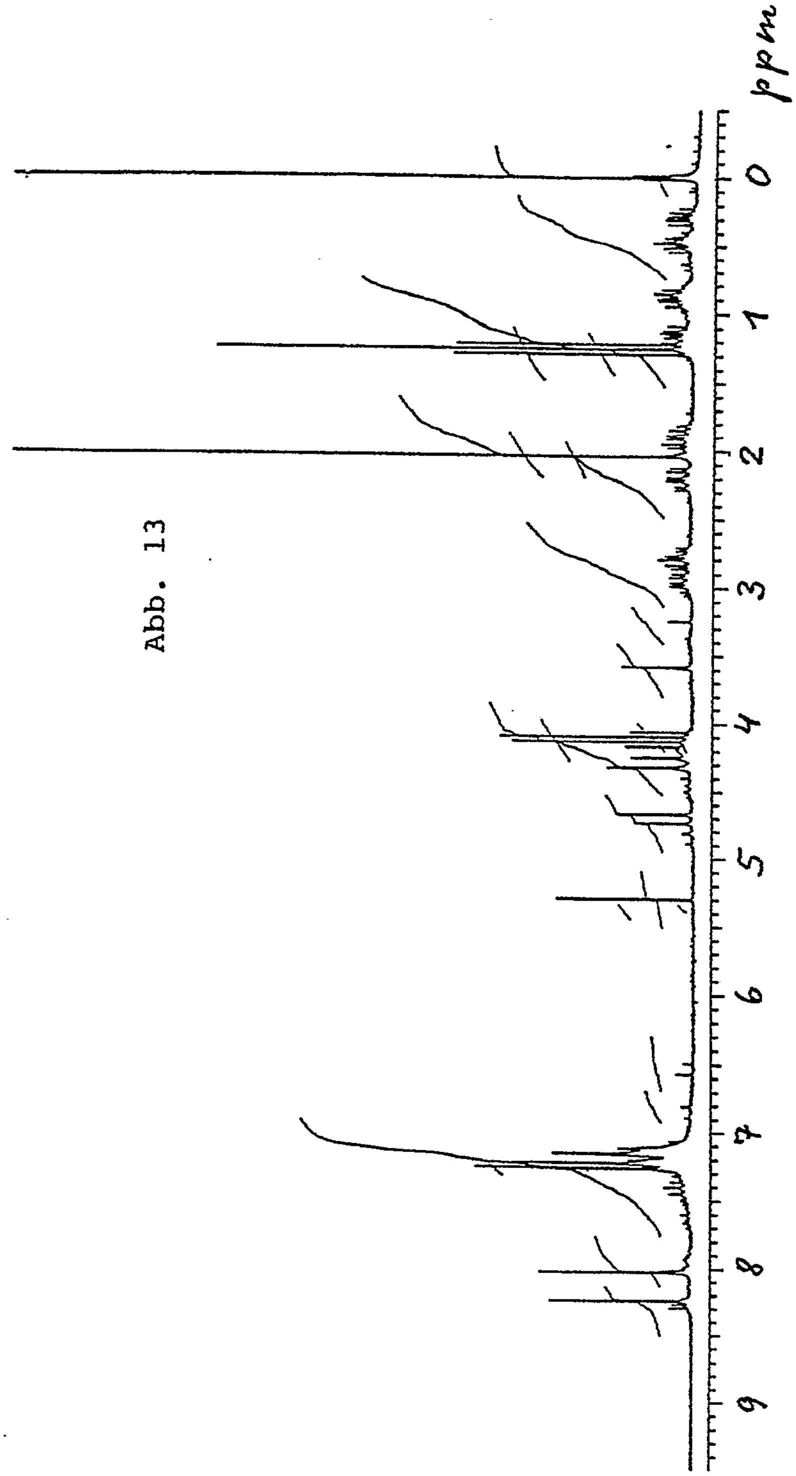


Abb. 13

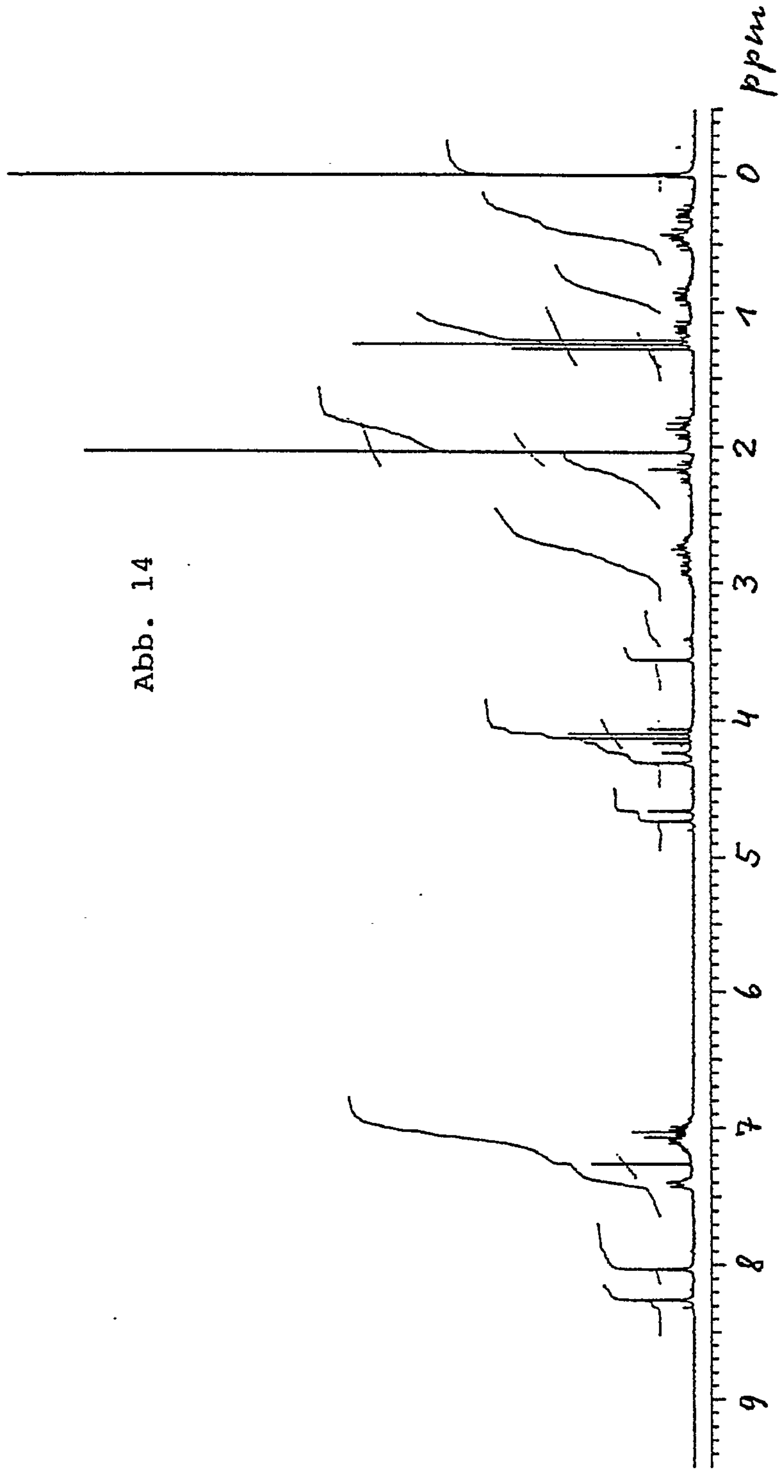


Abb. 14

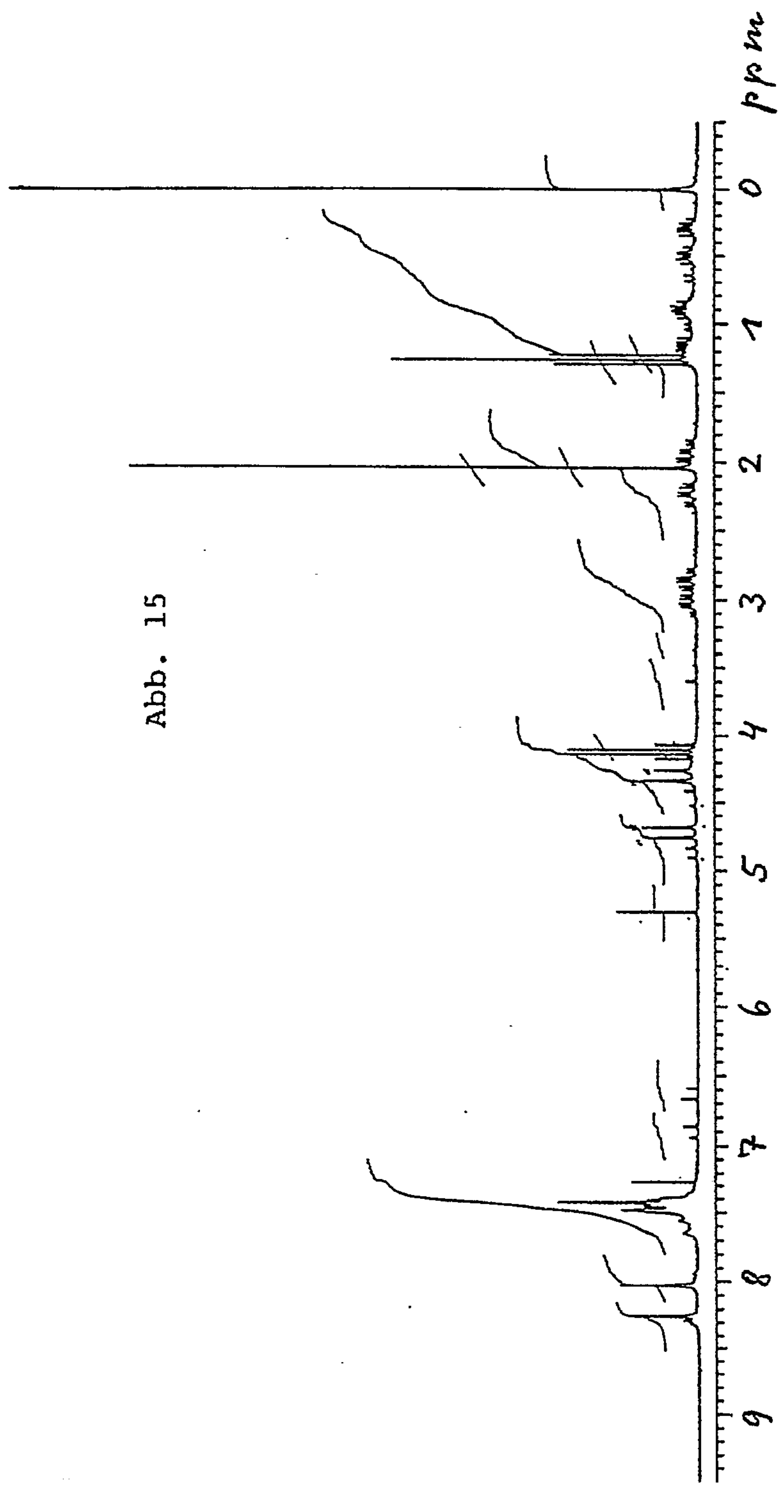


Abb. 15

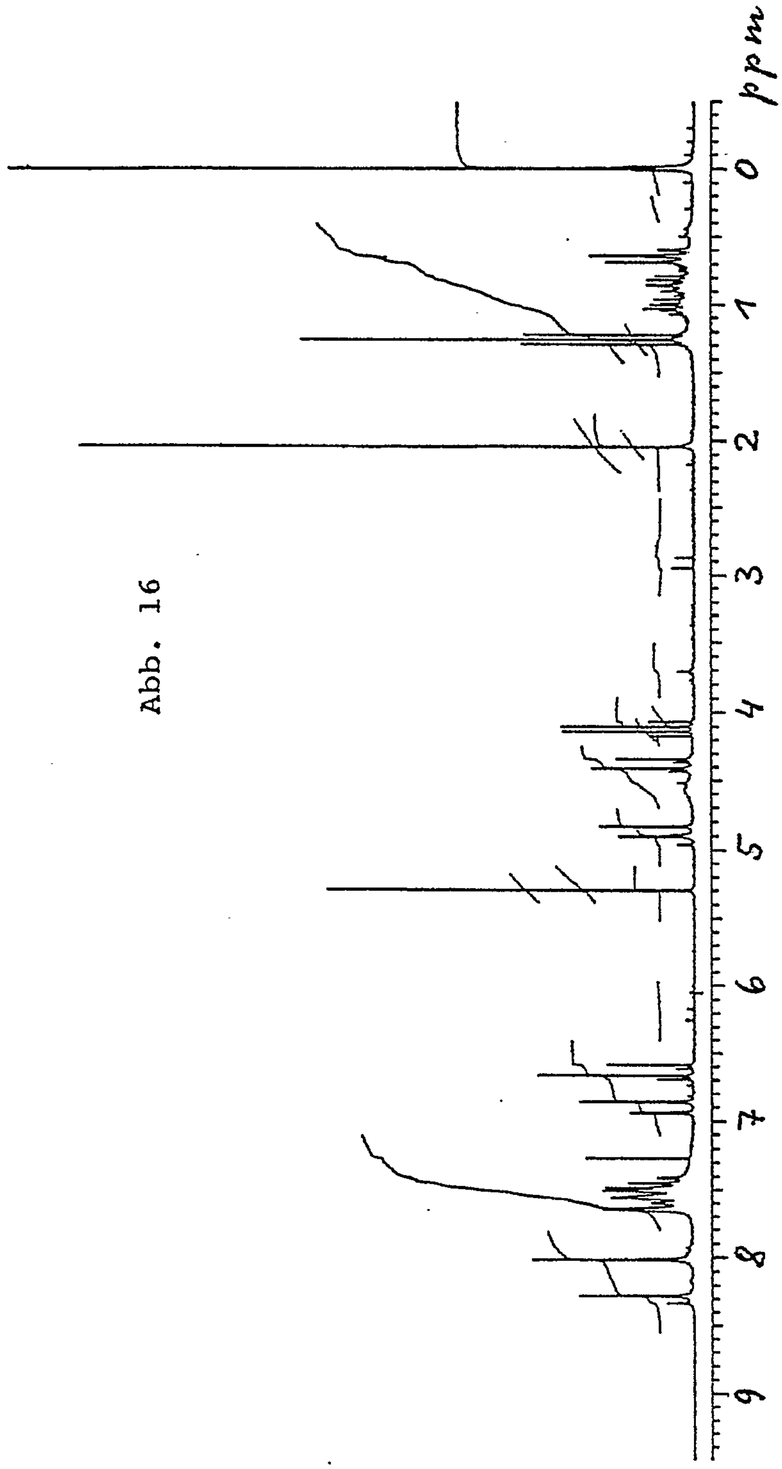


Abb. 16

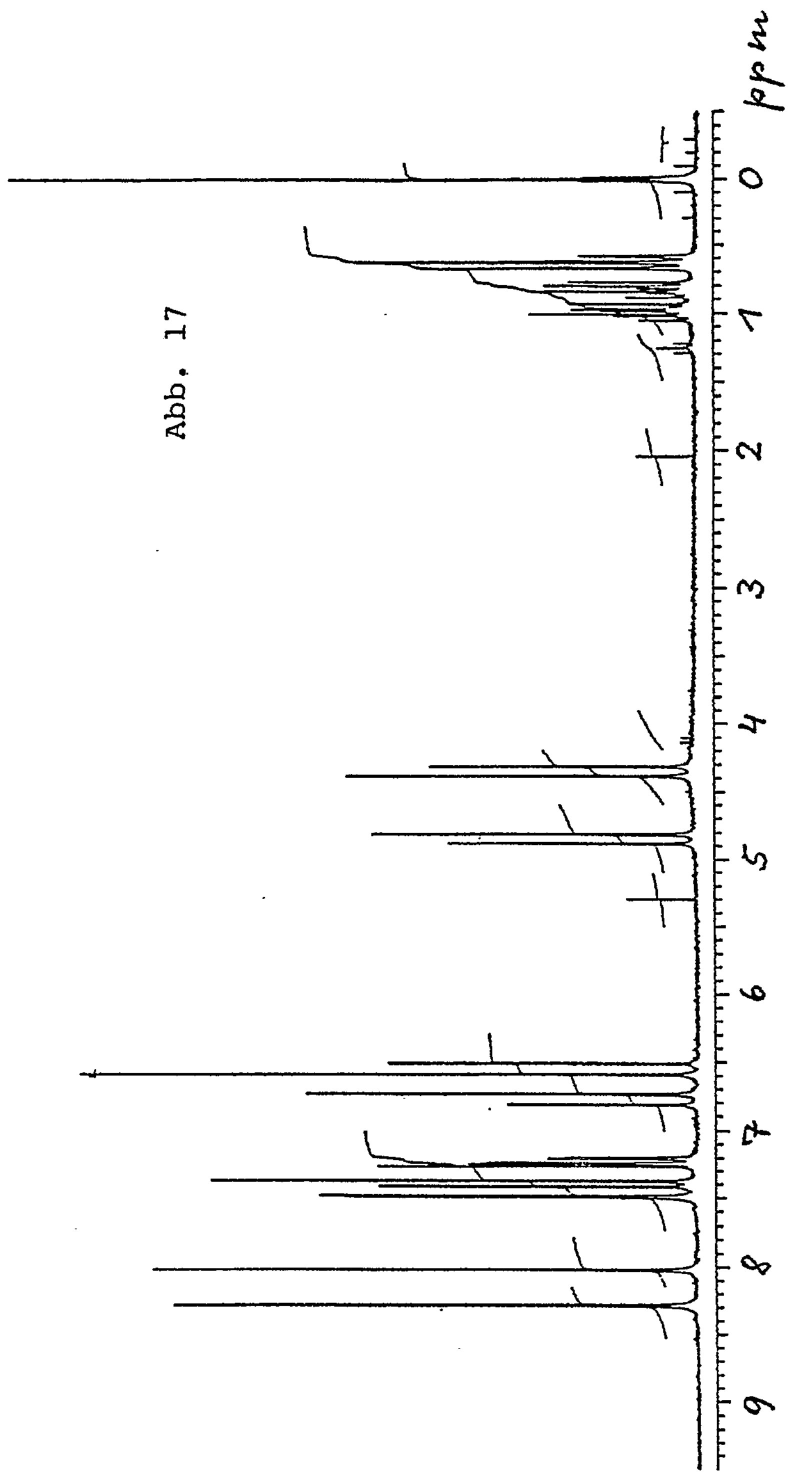


Abb. 17

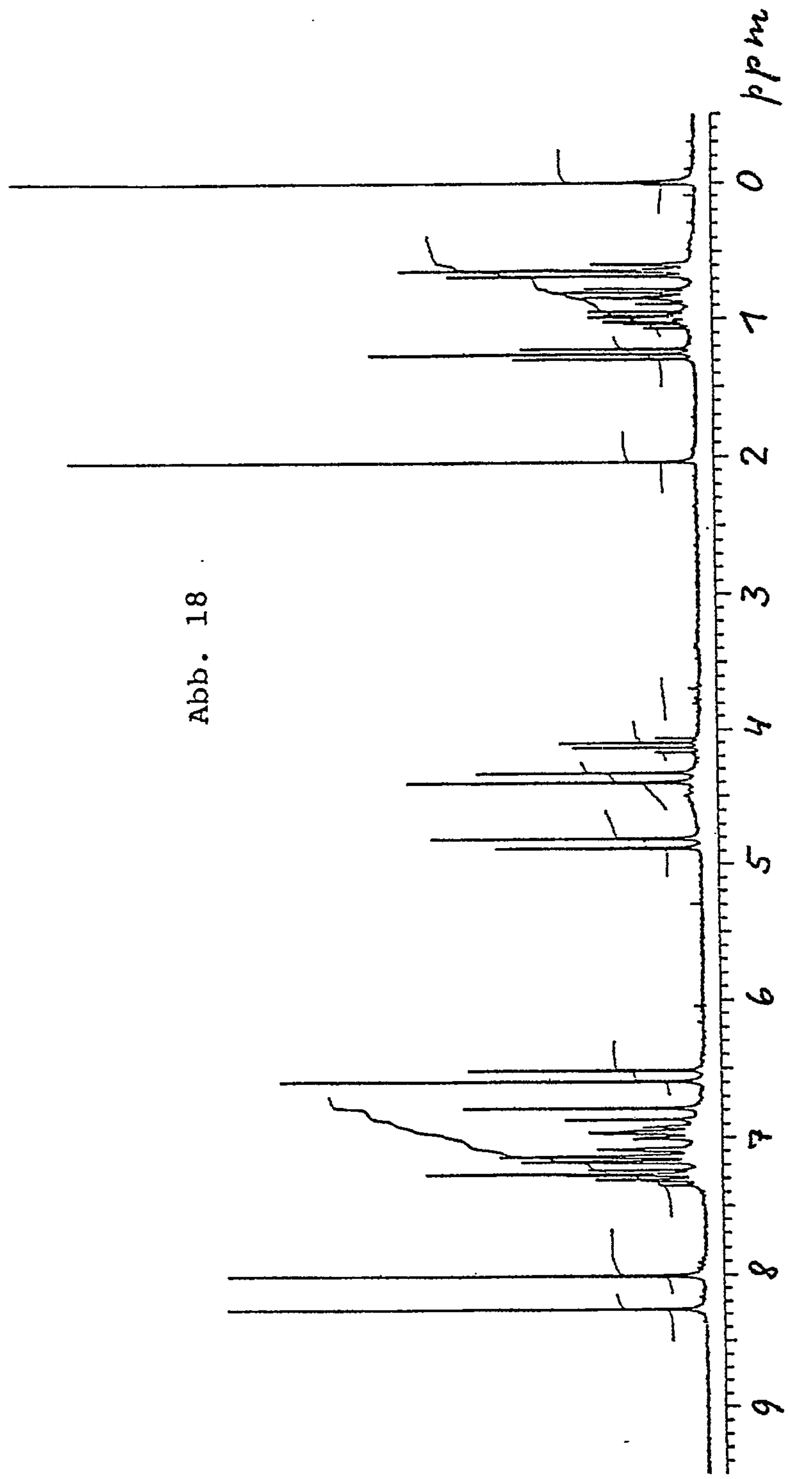


Abb. 18